(19) Europäisches Patentamt
European Patent Office
Office européen des brevets

(11) EP 0 392 384 B1

(12) EUROPEAN PATENT SPECIFICATION

(45) Date of publication and mention of the grant of the patent:
**18.02.1998 Bulletin 1998/08**

(51) Int. Cl.[6]: **C07H 15/203**, A61K 47/48

(21) Application number: **90106631.6**

(22) Date of filing: **06.04.1990**

# (54) Targeted forms of methyltrithio antitumor agents

Targetformen von Antitumor-Methyltrithioagenzien

Formes ciblés d'agents méthyltrithio antitumoraux

(84) Designated Contracting States:
**AT BE CH DE DK ES FR GB GR IT LI NL SE**

(30) Priority: **14.04.1989 US 339343**
**14.04.1989 US 339323**

(43) Date of publication of application:
**17.10.1990 Bulletin 1990/42**

(73) Proprietor:
**AMERICAN CYANAMID COMPANY**
**Wayne, NJ 07470-8426 (US)**

(72) Inventors:
- **Ellestad, George A.**
  **Pearl River, New York 10965 (US)**
- **McGahren, William James**
  **Demarest, New Jersey 07627 (US)**
- **Sassiver, Martin Leon**
  **Spring Valley, New York 10977 (US)**
- **Hamann, Philip R.**
  **Garnerville, New York 10923 (US)**
- **Hinman, Lois M.**
  **No. Tarrytown, New York 10594 (US)**
- **Upeslacis, Janis**
  **Pomona, New York 10970 (US)**

(74) Representative:
**Wächtershäuser, Günter, Prof. Dr.**
**Patentanwalt,**
**Tal 29**
**80331 München (DE)**

(56) References cited:
**EP-A- 0 276 485**
**EP-A- 0 313 873**
**EP-A- 0 313 874**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Xerox (UK) Business Services
2.15.9/3.4

## Description

SUMMARY OF THE INVENTION

The invention is disulfide compounds of the $\alpha_1$, $\alpha_2$, $\beta_1$, $\gamma$, and $\delta$ N-acyl derivatives of the LL-33288 complex and derivatives thereof as well as the disulfide compounds of N-acyl derivatives of BBM-1675, FR-900405, FR-900406, PD114759, PD115028, CL-1577A, CL-1577B, CL-1577D, CL-1577E, CL-1724 antibiotics and derivatives thereof prepared by reacting the antibiotic with an unsubstituted or substituted alkyl mercaptan. These disulfide compounds are effective antitumor agents.

The invention also describes carrier-drug conjugates of the disulfide analogs prepared from the materials listed above. The carrier (targeting) portion of the conjugate is a mono- or polyclonal antibody, their fragments, chemically or genetically manipulated counterparts, or growth factors or steroids. The invention includes the method of using the carrier-drug conjugates as well as their process of manufacture.

DESCRIPTION OF THE DRAWINGS

Figure 1 The ultraviolet spectrum of the antitumor antibiotic designated as N-acetyl LL-E33288$\gamma_1{}^I$.
Figure 2 The infrared spectrum of the antitumor antibiotic designated as N-acetyl LL-E33288$\gamma_1{}^I$.
Figure 3 The proton magnetic resonance spectrum of the antitumor antibiotic designated as N-acetyl LL-E33288$\gamma_1{}^I$.
Figure 4 The carbon-13 nuclear magnetic resonance spectrum of the antitumor antibiotic designated as N-acetyl LL-E33288$\gamma_1{}^I$.

DETAILED DESCRIPTION OF THE INVENTION

The family of antibacterial and antitumor agents, known collectively as the LL-E33288 complex are described and claimed in EPO publication number 0182152A3 published May 28; 1986 and are used to prepare the disulfur antitumor agents which are some of the starting materials for targeted forms of the antitumor agents of our invention.

The EPO publication number 0182152A3 describes the LL-E33288 complex, the components thereof, namely, LL-E33288$\alpha_1{}^{Br}$, LL-E33288$\alpha_1{}^I$, LL-E33288$\alpha_2{}^{Br}$, LL-E33288$\alpha_2{}^I$, LL-E33288$\beta_1{}^{Br}$, LL-E33288$\beta_1{}^I$, ll-E33288$\gamma_1{}^{Br}$, LL-E33288$\gamma_1{}^I$ and LL-E33288$\delta_1{}^I$, and methods for their production by aerobic fermentation utilizing a new strain of Micromonospora echinospora ssp calichensis or natural or derived mutants thereof. The EPO publication number 0182152A3 also discloses proposed structures for some of the above named components. These proposed structures are reproduced in Table 1

**<u>Table 1</u>: Proposed Structures for $CH_3$-SSS-W isolated from natural sources** (wherein W is the substituent attached to $CH_3$-SSS- below)

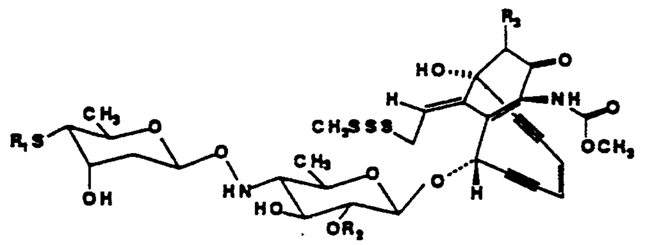

$R_{2'}$ =

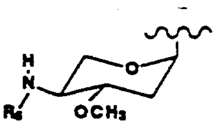

$R_{3'}$ =

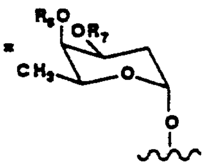

$Ar_1$ =

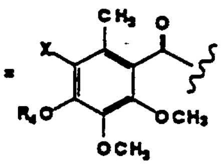

$Ar_2$ =

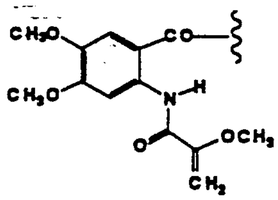

$R_{4'}$ =

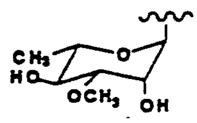

| Designation | $R_1$ | $R_2$ | $R_3$ | $R_4$ | $R_5$ | $R_6$ | $R_7$ | X |
|---|---|---|---|---|---|---|---|---|
| E33288$\alpha_2^I$ | $Ar_1$ | $R_{2'}$ | H | H | $C_2H_5$ | | | I |
| E33288$\beta_1^I$ | $Ar_1$ | $R_{2'}$ | H | $R_{4'}$ | $(CH_3)_2CH$ | | | I |
| E33288$\gamma_1^I$ | $Ar_1$ | $R_{2'}$ | H | $R_{4'}$ | $C_2H_5$ | | | I |
| E33288$\delta_1^I$ | $Ar_1$ | $R_{2'}$ | H | $R_{4'}$ | $CH_3$ | | | I |
| E33288$\beta_1^{Br}$ | $Ar_1$ | $R_{2'}$ | H | $R_{4'}$ | $(CH_3)_2CH$ | | | Br |
| E33288$\gamma_1^{Br}$ | $Ar_1$ | $R_{2'}$ | H | $R_{4'}$ | $C_2H_5$ | | | Br |
| E33288$\alpha_2^{Br}$ | $Ar_1$ | $R_{2'}$ | H | H | $C_2H_5$ | | | Br |
| Esperamicin $A_1$ | $CH_3$ | $R_{2'}$ | $R_{3'}$ | | $(CH_3)_2CH$ | H | $Ar_2$ | |
| Esperamicin $A_2$ | $CH_3$ | $R_{2'}$ | $R_{3'}$ | | $(CH_3)_2CH$ | $Ar_2$ | H | |
| Esperamicin $A_{1b}$ | $CH_3$ | $R_{2'}$ | $R_{3'}$ | | $CH_3CH_2$ | H | $Ar_2$ | |

Additional members of the LL-E33288 complex are described and claimed in EP-A-0 276 485 published August 3, 1988, and are likewise useful for preparing the disulfide derivatives and targeted forms of the antitumor agents of our invention.

These latter proposed structures are reproduced in Table 2.

Still other members of the LL-E33288 family of antitumor antibiotics are described and claimed in U. S. patent 5,079,233 and also are useful for preparing additional targeted forms of the antitumor agents of our invention. This application describes N-acyl derivatives of several members of the LL-E33288 complex which have been prepared by chemical means. These proposed structures are likewise reproduced in Table 2.

Table 2: **Proposed Structures for $CH_3$-SSS-W derived from chemical manipulation of the compound of Table 1** (wherein W is the substituent attached to $CH_3$-SSS- below)

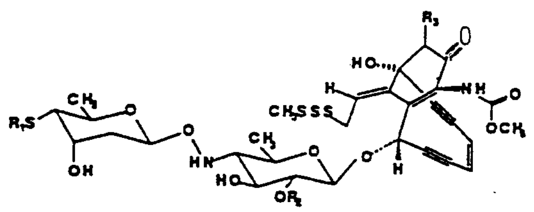

$R_2'$ = 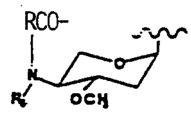

$R_4'$ = 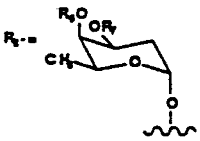

$Ar_1$ = 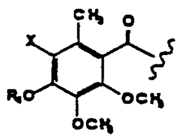

$Ar_2$ = 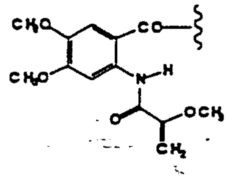

$R_3'$ = 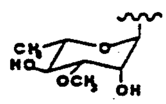

| Designation | $R_1$ | $R_2$ | $R_3$ | $R_4$ | $R_5$ | $R_6$ | $R_7$ | X |
|---|---|---|---|---|---|---|---|---|
| N-Acyl LL-E33288$\alpha_2^{I}$ | $Ar_1$ | $R_2'$ | H | H | $C_2H_5$ | | | I |
| N-Acyl LL-E33288$\beta_1^{I}$ | $Ar_1$ | $R_2'$ | H | $R_4'$ | $(CH_3)_2CH$ | | | I |
| N-Acyl LL-E33288$\gamma_1^{I}$ | $Ar_1$ | $R_2'$ | H | $R_4'$ | $C_2H_5$ | | | I |
| N-Acyl LL-E33288$\delta_1^{I}$ | $Ar_1$ | $R_2'$ | H | $R_4'$ | $CH_3$ | | | I |
| N-Acyl LL-E33288$\beta_1^{Br}$ | $Ar_1$ | $R_2'$ | H | $R_4'$ | $(CH_3)_2CH$ | | | Br |
| N-Acyl LL-E33288$\gamma_1^{Br}$ | $Ar_1$ | $R_2'$ | H | $R_4'$ | $C_2H_5$ | | | Br |
| N-Acyl LL-E33288$\alpha_2^{Br}$ | $Ar_1$ | $R_2'$ | H | H | $C_2H_5$ | | | Br |
| N-Acetyl Esperamicin $A_1$ | $CH_3$ | $R_2'$ | $R_3'$ | | $(CH_3)_2CH$ | H | $Ar_2$ | |
| N-Acetyl Esperamicin $A_2$ | $CH_3$ | $R_2'$ | $R_3'$ | | $(CH_3)_2CH$ | $Ar_2$ | H | |
| N-Acetyl Esperamicin $A_{1b}$ | $CH_3$ | $R_2'$ | $R_3'$ | | $CH_3CH_2$ | H | $Ar_2$ | |

R = hydrogen or a branched or unbranched alkyl ($C_1$ - $C_{10}$) or alkylene ($C_1$ - $C_{10}$) group, an aryl or heteroaryl group, or an a alkyl ($C_1$ - $C_5$) or heteroaryl-alkyl ($C_1$ - $C_5$) group, all optionally substituted by one or more hydroxy, amino, carboxy, halo, lower ($C_1$ - $C_3$) alkoxy, or lower ($C_1$ - $C_5$) thioalkoxy groups.

Certain other antitumor and antibacterial compounds are useful in our invention, namely:

1) Esperamicin BBM-1675, a novel class of potent antitumor antibiotics. I. Physico-chemical data and partial structure. M. Konishi, et. al., J. Antibiotics, 38, 1605 (1985). A new antitumor antibiotic complex, M. Konishi, et. al., U.K. Patent Application GB 2,141,425A, May 15, 1984.
2) New antitumor antibiotics, FR-900405 and FR-900406.I. Taxonomy of the producing strain. M. Iwami, et. al., J. Antibiotics 38, 835 (1985). New antitumor antibiotics FR-900405 and FR-900406.II. Production, isolation, characterization and antitumor activity. S. Kiyoto, et. al., J. Antibiotics, 38, 340 (1985).
3) PD 114759 and PD 115028, novel antitumor antibiotics with phenomenal potency. I. Isolation and characterization. R.H. Bunge, et. al., J. Antibiotics, 37, 1566 (1984). Biological and biochemical activities of the novel antitumor antibiotic PD 114759 and related derivatives. D.W. Fry et. al., Investigational New Drugs, 4, 3 (1986).

4) New antibiotic complex CL-1577A, CL-1577B produced by Streptomyces sp. ATCC 39363. European Patent Application 0,132,082, A2.

5) CL-1577D and CL-1577E Antibiotic antitumor compounds, their production and use. U.S. Patent 4,539,203.

6) CL-1724 Antibiotic compounds, their production and use. U.S. Patent 4,554,162.

7) New antitumor antibiotics BBM-1675-A3 and BBM-1675-A4, obtained by fermentation of actinomadura verrucosospora strains H964-92 (ATCC 39334) or AB27Y (ATCC 39638). U.S. Patent 4,675,187.

8) New N-acetyl-esperamicin $A_1$, $A_2$ and $A_{1b}$ derivatives with antimicrobial and antitumor activities. European Patent 289,030.

Information regarding BBM-1675, FR-900405, FR-900406, PD 114759, PD 115028, CL-1577A, CL-1577B, CL-1577D, CL-1577E and CL-1724 is contained in the above citations. The complete structures of esperamicins $A_1$, $A_2$, and $A_{1b}$ (the BBM-1675 complex) and their respective N-acetyl derivatives have been reported, and these are included in Tables 1 and 2. The physical characteristics of the other above-named antitumor antibiotics indicate that they all are identical or very similar in structure to the esperamicins, and all contain a methyltrithio functional group.

As can be seen from the structures disclosed above, the N-acyl counterparts $\alpha_1$, $\alpha_2$, $\beta_1$, $\gamma_1$, $\delta$ of the LL-E33288 complex, as well as the N-acyl counterparts BBM-1675, FR-900405, FR-900406, PD 114759, PD 115028, CL-1577A, CL-1577B, CL-1577D, CL-1577E and CL-1724 antibiotics each contain a methyltrithio group in their structure.

It has now been discovered that the reaction of any of the above cited antibiotics with an unsubstituted or substituted alkyl or aryl mercaptan results in displacement of the methyl perthiolate anion from the trisulfide moiety resulting in the formation of a stable disulfide (Scheme I), below. Other compounds on which this transformation works are disclosed in EPO publication number 0313874A2 published May 3, 1989.

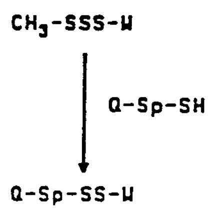


Scheme I

The compounds of Tables 1 and 2 are transformed with thiol-containing organic molecules according to Scheme I to produce new compositions useful as antibacterial and anticancer agents in their own right, wherein W, R, $R_1$, $R_2$, $R_3$, $R_4$, $R_5$, $R_6$, $R_7$, $R_8$, $R_9$, $R_2$', $R_3$', $R_4$', $R_5$', $Ar_1$, $Ar_2$ and X are as hereinbefore defined in Tables 1 and 2, Sp is straight or branched-chain divalent or trivalent ($C_1$-$C_{18}$) radicals, divalent or trivalent aryl or heteroaryl radicals, divalent or trivalent ($C_3$-$C_{18}$) cycloalkyl or heterocycloalkyl radicals, divalent or trivalent aryl- or heteroarylalkyl ($C_1$-$C_{18}$) radicals, divalent or trivalent cycloalkyl- or heterocycloalkyl-alkyl ($C_1$-$C_{18}$) radicals, or divalent or trivalent ($C_2$-$C_{18}$) unsaturated alkyl radicals, wherein if Sp is a trivalent radical, it can be additionally substituted by amino, alkylamino, arylamino, heteroarylamino, carboxyl, lower alkoxy, hydroxy, thiol, or lower alkylthio groups; Q is hydrogen, halogen, amino, alkylamino, dialkylamino, arylamino, heteroarylamino, piperidino, pyrrolidino, piperazino, carboxyl, nitrophenyloxycarbonyl, carboxaldehyde, lower alkoxy, hydroxy, thiol, lower alkyldicarboxyl, or a group chosen from the following: -$CONHNH_2$, -$NHCONHNH_2$, -$NHCSNHNH_2$, or -$ONH_2$; with the provisos that when Sp is ethylidene, Q cannot be hydrogen; and when $CH_3$-SSS-W is LL-E33288$\alpha_1$-Br, $\alpha_1$-I, $\alpha_2$-Br, $a_2$-I, $\alpha_3$-Br, $\alpha_3$-I, $\alpha_4$-Br, $\beta_1$-Br, $\beta_1$-I, $\beta_2$-Br, $\beta_2$-I, $\gamma_1$-Br, $\gamma_1$-I, $\delta_1$-I, BBM-1675, FR-900405, FR-900406, PD114759, PR115028, CL-1577A, CL-1577B, CL-1577D, CL-1577E, or CL-1724 and Sp is divalent, Q cannot be hydrogen, halogen, amino, alkylamino, dialkylamino, arylamino, heteroarylamino, piperidino, pyrrolidino, piperazino, or carboxyl.

As a preferred embodiment of this invention, disulfides of the formula Q-Sp-SS-W are prepared from the antitumor antibiotics designated LL-E33288 ($CH_3$SSS-W) wherein:

W is 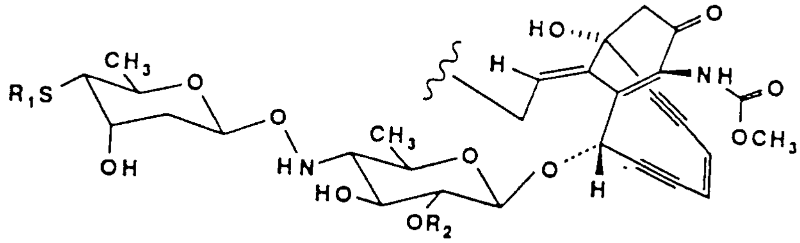

$R_1$ is 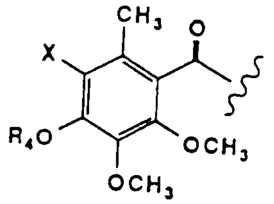 ;

$R_2$ is 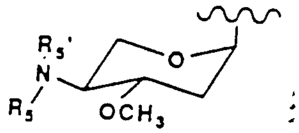 , or H;

$R_4$ is 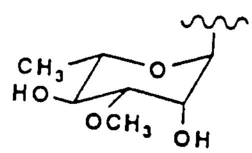 or H;

$R_5$ is $CH_3$, $C_2H_5$, or $(CH_3)_2$; X is an iodine or bromine atom; $R_5$ is a hydrogen or the group RCO, wherein R is hydrogen, $CH_3$, or a mono-substituted aryl group; and Sp and Q are as hereinbefore defined.

The transformation of the compounds listed in Tables 1 and 2 to a variety of disulfides can be accomplished with a large variety of thiol-containing organic molecules to yield novel antitumor and antibacterial agents. Moreover, the products from the reaction themselves are amenable to further transformations within the newly-introduced side-chain to produce still more novel compounds with biological activities.

It has also been discovered that the displacement of the methyltrithio unit of the compounds listed in Tables 1 and 2 and as depicted in Scheme I can be used to introduce a spacer (Sp), the judicious choice of which enables the introduction of targeting units into the disulfide derivatives compounds of the application. Introduction of targeting units to other disulfide derivatives is disclosed in EPO publication number 0313873A1 published May 3, 1989. Thus, with reference to Scheme I, the compounds of formula $CH_3SSS$-W wherein $CH_3SSS$-W is an N-acyl derivative of an antitumor antibiotic designated as LL-E33288, $\alpha_1^{I}$, $\alpha_2^{Br}$, $\alpha_2^{I}$, $\beta_1^{Br}$, $\beta_1^{I}$, $\gamma_1^{Br}$, $\gamma_1^{I}$, $\delta_1^{I}$, BBM-1675, FR-900405, FR-900406, PD 114759, PD 115028, CL-1577A, CL-1577B, CL-1577D, CL-1577E, CL-1724 are first reacted with a compound of formula Q-Sp-SH to produce an intermediate

Q-Sp-SS-W

wherein Sp is a straight or branched-chain divalent or trivalent ($C_1$-$C_{18}$) radical, divalent or trivalent aryl or heteroaryl

radical, divalent or trivalent ($C_3$-$C_{18}$) cycloalkyl or heterocycloalkyl radical, divalent or trivalent aryl- or heteroaryl-alkyl ($C_1$-$C_{18}$) radical, divalent or trivalent cycloalkyl- or heterocycloalkyl-alkyl ($C_1$-$C_{18}$) radical, or divalent or trivalent ($C_2$-$C_{18}$) unsaturated alkyl radical, wherein if Sp is a trivalent radical, it can be additionally substituted by amino, alkylamino, arylamino, heteroarylamino, carboxyl, lower alkoxy, hydroxy, thiol or lower alkylthio groups, with the proviso that when $CH_3$-SSS-W is the parent antitumor antibiotic LL-E33288$\alpha_1$-Br, $\alpha_1$-I, $\alpha_2$-Br, $\alpha_2$-I, $\beta_1$-Br, $\beta_1$-I, $\beta_2$-Br, $\gamma_1$-Br, $\gamma_1$-I, $\delta_1$-I, BBM-1675, FR-900405, FR-900406, PD114759, PR115028, CL-1577A, CL-1577B, CL-1577D, CL-1577E, or CL-1724 itself, Sp can not be divalent; Q is, or can be subsequently converted to, halogen, amino, alkylamino, carboxyl, carboxaldehyde, hydroxy, thiol, $\alpha$-haloacetyloxy, lower alkyldicarboxyl, $-CONHNH_2$, $-NHCONHNH_2$, $-NHCSNHNH_2$, $-ONH_2$, $-CON_3$,

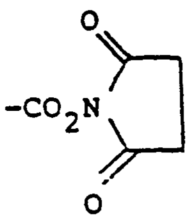 , 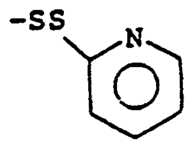 , 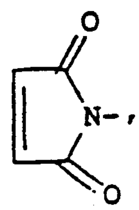 , 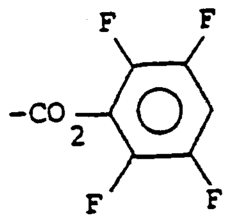 ,

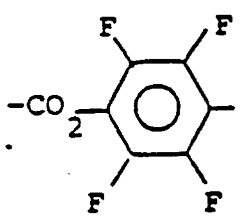 , 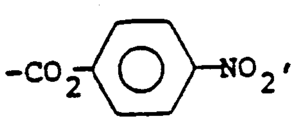 , 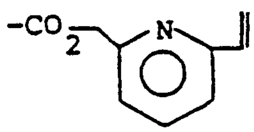 , 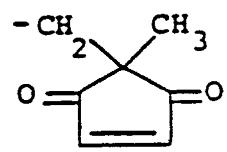

As long as the product from Scheme contains at least one functional group which can be converted to, or is directly reactive with a targeting unit (Tu), targeted forms of the antitumor antibiotics of the above-named patents and applications can be generated, as shown in Scheme II below:

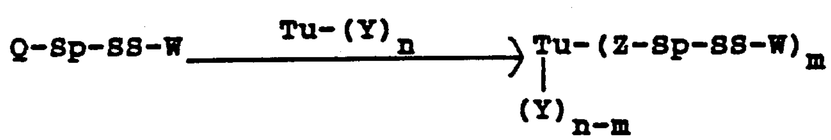


**Scheme II**

wherein Q, Sp, and W are as hereinbefore defined, Tu is a mono- or polyclonal antibody, its fragments, its chemically or genetically manipulated counterparts, or growth factors or steroids; Y is a side-chain amino, carboxy, or thiol group of a protein, an aldehyde derived from carbohydrate residues, or an amidoalkylthio group; n is an integer of from 1 to 100; Z is formed from covalent reaction of the groups Q and Y directly or after subsequent reduction and Z is -CONH-, -CONHN=CH-, $-CONHNHCH_2-$, -NHCONHN=CH-, $-NHCONHNHCH_2-$, -NHCSNHN=CH-, $-NHCSNHNHCH_2-$, -ON=CH-, -NH-, $-NHCH_2-$, -N=CH-, $-CO_2-$, $-NHCH_2CO_2-$, -SS-,

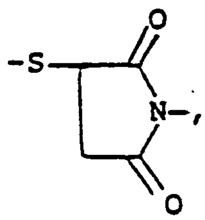, 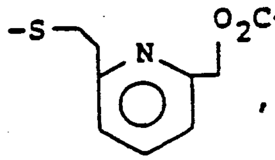, 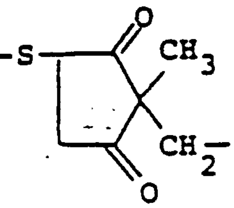 or 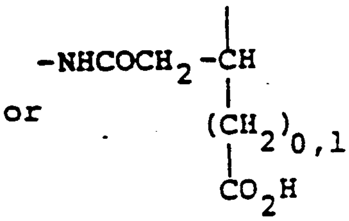

and m is 0.1 to 15.

As an example, and with reference to Scheme II, above, the 3-mercaptopropionic acid derivative of N-acetyl E-33288$\gamma_1^I$ (Q=$CO_2H$, Sp=-$CH_2CO_2$-), when converted to its activated hydroxysuccinimide form (Q=$CO_2Su$, Sp=-$CH_2$-$CH_2$-) can be used to react with some of the ε-amino groups of lysine residues (e.g., Tu=monoclonal antibody, Y=-$NH_2$ wherein n=50-100 from available lysine residues), at a pH between 7.0 and 9.5 in aqueous buffered solutions at temperatures between 4°C to 40°C to produce targeted forms of the antibiotics attached at random sites along the protein backbone (Tu=monoclonal antibody, Z=-NHCO-, Sp=-$CH_2CH_2$-, m=1-10). Only a fraction of the available lysine residues are substituted in this manner, since high loading is generally not considered compatible with preserving the antibody immunoreactivity. The same randomly-substituted immunoconjugates can also be prepared from the 3-mercaptopropionic acid derivative using other carboxyl group activating agents such as a variety of carbodiimides, or the corresponding acyl azide. Alternatively, a 3-mercaptopropionyl hydrazide derivative of N-acetyl LL-E33288$\delta_1^I$ (Q=$H_2NNHCO$-, Sp=-$CH_2CH_2$-), when reacted with a periodate-oxidized antibody (Tu=monoclonal antibody, Y=-CHO, n=1-15) as described in U.S. Patent No. 4,671,958 at a pH between 4 and 7, in a buffered aqueous solution at a temperature of between 4°C and 40°C, reacts only at the aldehyde functionality (derived from cleavage of vic-diols of carbohydrate residues situated on the Fc portion of the antibodies) to generate monoclonal antibody conjugates containing the drug substituted at specific sites along the backbone of the protein (Tu=monoclonal antibody, Z=-CH=NNHCO-, Sp=-$CH_2CH_2$-, m=0.5-10). In order to block unreacted aldehyde groups on the antibody and thus avoid crosslinking, as well as stabilize the hydrolytically labile Schiff's base linkages, it is preferable (though not essential) to react the latter conjugate first with a compound such as acetyl hydrazide or tyrosine hydrazide, then reduce with sodium cyanoborohydride or sodium borohydride to produce the stabilized constructs of this invention (Tu=monoclonal antibody, Z=-$CH_2NHNHCO$-, Sp=-$CH_2CH_2$-, m=0.5-10). Other aldehyde-reactive groups as part of the drug construct are within our invention to generate the products of Scheme II. Such functional groups are preferably, though not limited to, those which react with aldehydes under acidic aqueous conditions. The reactivity of protein lysines under basic conditions is sufficiently great such that their amines compete with the products of Scheme II for available aldehydes of the monoclonal antibody. Alternative aldehyde-reactive groups are, for example, the semicarbazide, the thiosemicarbazide, and the O-substituted hydroxylamine functionalities.

Assembly of targeted forms of the compounds listed in Tables 1 and 2 is not restricted to the sequence outlined in Scheme II. The targeting unit (Tu) can be first modified to contain a thiol group, which is then reacted with the compounds of Tables 1 and 2 in accordance with Scheme III below:

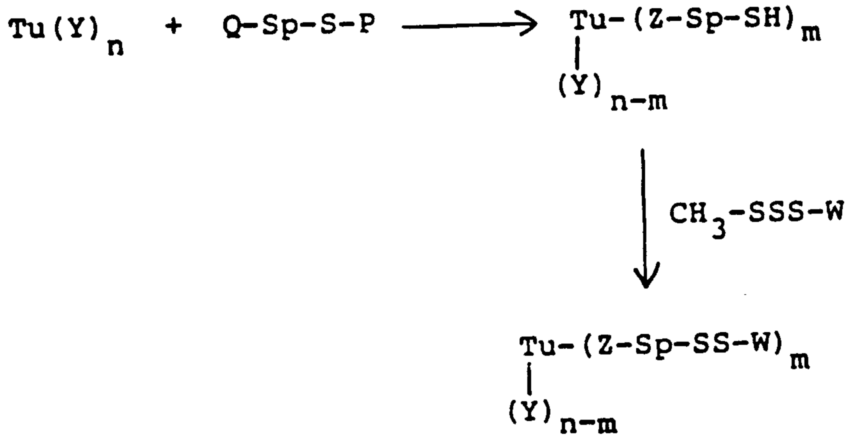


Scheme III

wherein Tu, Y, Q, Sp, W, n, and m are as hereinbefore defined, and P is hydrogen or 2-(pyridylthio), with the proviso that when Y is a thiol derived from a backbone amino acid residue of Tu, Z-Sp taken together is a covalent bond.

As an example, and with references to Scheme III, above, a monoclonal antibody can be reacted with 3-(2-dithiopyridyl)propionic acid hydroxysuccinimide ester to modify the protein through lysine residues (Tu=monoclonal antibody, Y=$NH_2$, n=50-100, Q=-$CO_2$Su, Sp=-$CH_2$-$CH_2$-, P=2-pyridylthio). Following reduction with, for example, dithiothreitol, an intermediate is generated (Tu=monoclonal antibody, Z=-NHCO-, Sp=-$CH_2CH_2$-, m=1 to 15) which can be reacted with the compounds of Tables 1 and 2 to generate the subject immunoconjugates. Similarly, 2-iminothiolane can be reacted with a monoclonal antibody to introduce thiol groups onto the surface of the protein directly, without requiring a reduction step (Tu=monoclonal antibody, Z=-NHCO-, Sp=-$(CH_2)_3$-, m=1 to 15), and this intermediate can be reacted with the compounds of Tables 1 and 2 as before. Alternatively, sulfhydryl groups inherent within the structure of monoclonal antibodies in dimeric form as cystine residues can be used to participate in the reaction of Scheme III directly. Such sulfhydryls are traditionally exposed by a combination of enzymatic digestion and reduction of native monoclonal antibodies (Tu=Fab′ fragment, Z-Sp=bond, Y=SH), but the use of genetically-altered constructs of monoclonal antibodies containing unpaired cystine residues is likewise contemplated.

A preferred embodiment of targeted derivatives of this invention is a protein-drug conjugate of the formula:

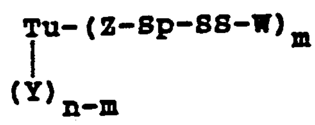

prepared from the class of antitumor antibiotics designated N-acyl LL-E33288 ($CH_3$SSS-W) comprising:

displacing the dithiomethyl moiety with a compound of formula Q-Sp-SH, wherein Sp is straight or branched-chain divalent or trivalent ($C_2$-$C_{10}$) radicals or divalent or trivalent ($C_2$-$C_5$) arylalkyl or heteroarylalkyl radicals, wherein if Sp is a trivalent radical, it can be additionally substituted by amino, heteroarylamino, hydroxy, or thiol groups; and Q is carboxyl, lower alkyldicarboxyl anhydride, -$CO_2$Su, -$CONHNH_2$, or

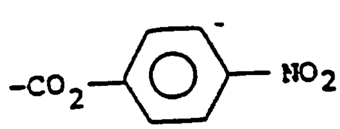

to produce an intermediate of general formula Q-Sp-SS-W, wherein Q, Sp, and W are as hereinbefore defined, reacting Q-Sp-SS-W with a molecule of the formula TU-$(Y)_n$ wherein Tu is a monoclonal antibody which exhibits

preferential reactivity with a human tumor-associated antigen, Y is a side-chain amino group on the antibody, or an aldehyde generated by oxidation of the carbohydrate groups of the antibody, and n is an integer of from 1 to 100, to produce a compound of the formula:

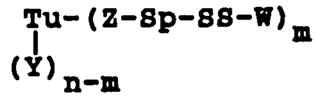

wherein Tu, Y, Sp, W, and n are as hereinbefore defined, and Z is formed from covalent reaction of the groups Q and Y directly or after subsequent reduction, and Z is -CONH-, -CONHN=CH-, $-CONHNHCH_2-$, or

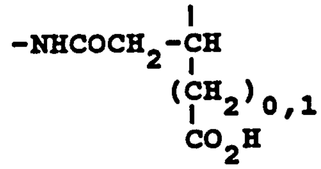

and m is 0.1 to 15.

A number of different monoclonal antibodies (MoAbs) are used to exemplify targeting of the methyltrithio anticancer compounds. MoAbs Lym 1 and Lym 2 recognize different antigens on mature B-lymphocytes and their product lymphomas. The production and characteristics of these MoAbs are described by A.L. Epstein, et. al., "Cancer Research" 47, 830 (1987). MoAb B72.3 targets primarily to carcinomas of the breast and colon, though reactivity with pancreatic, ovarian, and lung carcinomas has also been noted. The antibody has been described by T.L. Klug, et. al., "Int. J. Cancer" 38, 661 (1986). MoAb CT-M-01, which recognizes primarily breast tumors is described in EPO application 86 401482.4 filed July 3, 1986 and MAC-68 is produced by a sub-clone of the hybridoma which produces CT-M-01, and recognizes both breast and colon carcinomas. Intermediates of the subject compounds useful for, and conjugates with these antibodies, are described in the experimental section. It should not, however, be construed that this patent is limited to or restricted by the aforementioned antibodies. Instead, the methodology is sufficiently general that it can be applied to all antibodies regardless of their class or isotype, their enzymatically-derived fragments, their chemically manipulated and stabilized fragments, as well as their respective chimeric and humanized counterparts. Nor are the targeting units restricted only to monoclonal antibodies. Other proteins, as well as small molecules for which receptors exist on target issues, are within the purview of our discovery as targeting entities.

The compounds of this invention designated as Q-Sp-SS-W are active as antibacterial agents. Their in vitro antibacterial activity can be determined against gram-positive and gram-negative bacteria by a standard agar dilution method. Mueller-Hinton agar containing two-fold decreasing concentrations of the antibiotics is poured into petri dishes. The agar surfaces are inoculated with 1 to 5 x $10^4$ colony forming units of bacteria by means of a Steers replicating device. The lowest concentration of compound that inhibits growth of a bacterial strain after about 18 hours of incubation at approximately 36°C is recorded as the minimal inhibitory concentration (MIC) for that strain.

The disulfide compounds described above are active antitumor agents.

Certain in vivo testing systems and protocols have been developed by the National Cancer Institute for testing compounds to determine their suitability as antineoplastic agents. These have been reported in "Cancer Chemotherapy Reports", Part III, Vol. 3, No. 2 (1972), Geran, et. al. These protocols have established standardized screening tests which are generally followed in the field of testing for antitumor agents. Of these systems, lymphocytic leukemia P388, melanotic melanoma B16, L1210 leukemia and colon 26 adenocarcinoma are particularly significant to the present invention. These neoplasms are utilized for testing as transplantable tumors in mice. Generally, significant anti tumor activity, shown in these protocols by a percentage increase of mean survival times of the treated animals (T) over the control animals (C) is indicative of similar results in human leukemias and solid tumors.

Lymphocytic Leukemia P388 Test

The animals used were $BDF_1$ female mice. There were 5 or 6 mice per test group. The tumor transplant was by intraperitoneal injection of 0.5 ml of dilute ascitic fluid containing 1 x $10^6$ cells of lymphocytic leukemia P388 on day

zero. The test compounds were administered intraperitoneally at a volume of 0.5 ml in sterile, pyrogen free saline on days 1, 5 and 9 (relative to tumor inoculation) at the indicated doses. The mice were weighed and survivors recorded on a regular basis for 30 days. The median survival time and the ratio of survival time for treated (T)/control (C) animals were calculated. Values of T/C greater than or equal to 125 are considered to reflect significant antitumor activity. The results appear in Table 3.

Table 3

| Lymphocytic Leukemia P388 Test | | | |
|---|---|---|---|
| Compound | Dose (mg/kg) | Median Survival (Days) | T/Cx100 % |
| Propionic acid disulfide of LL-E33288$\gamma_1$-I (from Example 3) | 0.005 | | 145 |
| | 0.0025 | | 125 |
| 3-Mercaptopropionyl hydrazide disulfide of N-acetyl LL-E33288$\gamma_1$-I (from Example 4) | 0.080 | 21.0 | 175 |
| | 0.040 | 18.0 | 150 |
| | 0.020 | 15.0 | 125 |
| Control | - | 12.0 | - |
| 3-Mercaptopropionyl hydrazide disulfide of LL-E33288$\alpha_2$-I (from Example 5) | 0.010 | 26.5 | 230 |
| | 0.005 | 21.0 | 183 |
| | 0.0025 | 18.5 | 161 |
| | 0.00125 | 18.5 | 161 |
| Control | - | 11.5 | - |
| 3-Mercaptobutyryl hydrazide disulfide of LL-E33288$\gamma_1$-I (from Example 7) | 0.005 | 28.5 | 190 |
| | 0.0025 | 23.0 | 153 |
| | 0.00125 | 21.0 | 140 |
| | 0.0006 | 19.5 | 130 |
| Control | - | 15.0 | - |
| 3-Mercaptoisovaleryl hydrazide disulfide of LL-E33288$q_1$-I (from Example 8) | 0.010 | 26.5 | 252 |
| | 0.005 | 18.0 | 171 |
| | 0.0025 | 17.0 | 162 |
| | 0.00125 | 16.0 | 152 |
| | 0.0006 | 13.0 | 124 |
| | 0.0003 | 14.0 | 133 |
| Control | - | 10.5 | - |

<u>Melanotic Melanoma B16 Test</u>

Animals used can be BDF1 female mice. There are 5 or 6 mice per test group. A one gram portion of melanotic melanoma B16 tumor is homogenized in 10 ml of cold balanced salt solution and a 0.5 ml aliquot of the homogenate is implanted intraperitoneally into each of the test mice. The test compounds are administered intraperitoneally on days 1 through 9 (relative to tumor inoculation) at the indicated doses. The mice are weighed and survivors recorded on a regular basis for 60 days. The median survival time and the ratio of survival time for treated (T)/control (C) animals are calculated. A value of T/C of greater or equal to 125 is considered indicative of significant antitumor activity.

The methods of this invention used to produce monoclonal antibody conjugates from the compounds of Tables 1 and 2 and designated as

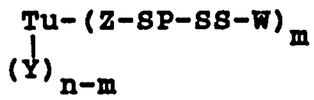

yield constructs which retain good immunoreactivity with target cell lines, as determined by the following <u>in vitro</u> assays:

Target Cells

All target cells were maintained in RPMI 1640 media supplemented with 5% Fetal Calf Serum (FCS), ITS (Collaborative Research, Cat# 40351), streptomycin (50 µg/ml), penicillin (50 units/ml), gentamycin sulfate (50 µg/ml) and glutamine (.03%). The cells were maintained in a humidified 5% $CO_2$ incubator at 37°C.

1. Immunoreactivity Assays

Procedure I - Elisa

Appropriate target cells were harvested, counted and suspended in Dulbecco's Phosphate Buffered Saline (DPBS) at an optimal concentration for monoclonal antibody (MoAb) being tested. 0.1 ml of cells was aliquoted in each well of a sterile tissue culture polystyrene 96-well plate. The plates were centrifuged for 5 minutes at 1,000 RPM's and the supernatant was flicked off. Plates were air-dried overnight and may be stored at 4°C for up to 3 months.

Non-specific binding sites were blocked by adding 200 µl of 1% gelatin in DPBS per well and incubating the plate for 1 hour at 37°C in a humid incubator. (All subsequent incubations are done under similar conditions). The plates were washed once with 250 µl of 0.05% TWEEN-20 in DPBS (washing solution) using the automated ELISA washing system from Dynatech (Ultrawash II). Samples to be tested were diluted to make a final concentration of 3 µg/ml MoAb equivalents in 0.1% gelatin-DPBS. Six additional threefold serial dilutions were prepared from each 3 µg/ml sample and 100 µl was added to appropriate wells in triplicate. The bottom row of wells only received 100 µl of 0.1% gelatin as background. Plates were incubated for 45 minutes and then washed three times. Alkaline phosphatase conjugated affinity purified goat anti-mouse immunoglobulins (Cappel Cat# 8611-0231) was diluted 1:125 in 0.1% gelatin and 100 µl was added to each well. Plates were incubated for 45 minutes and then washed three times. 200 µl of p-nitrophenyl phosphate substrate solution (see below) was added to each well. After 45 minutes at room temperature the reaction was stopped by the addition of 50 µl of 3M NaOH. The absorbance of the contents of each well was read at 405 nm in the automated spectrophotometer from Dynatech (EIA Autoreader # EL-310).

Substrate Diethanolamine Buffer (10%)

97 ml diethanolamine
800 ml water
0.2 grams $NaN_3$
100 mg $MgCl_2\ 6H_2O$

The reagents were dissolved by continuous stirring and 1M HCl was added until the pH was 9.8. The total volume was made up to 1 liter with water and filter sterilized with a 0.2 µ filter. The buffer was stored in the dark at 4°C. Immediately before use, p-nitrophenyl phosphate (Sigma, Cat# 104-40) was dissolved in the 10% diethanolamine buffer (must be at room temperature) to give a final concentration of 1 mg/ml.

Calculation of O. D. Values

The percentage binding of each sample was calculated by the following equation:

$$\frac{A-B}{C-B} \times 100 = \%\ \text{Binding}$$

A = Average O.D. of test sample
B = Average O.D. of background
C = Average O.D. of 3 µg/ml unmanipulated MoAb control

The % binding was plotted on the non-log scale of a semi-log graph and the MoAb concentration was plotted on the log scale. The $BD_{50}$ (i.e. dose of antibody needed to give 50% binding) of each test sample was derived from the graph and the amount of retention of immunoreactivity was calculated by the following equation:

$$\frac{BD_{50}\text{ of MoAb control}}{BD_{50}\text{ of test sample}} \times 100 = \%\text{ Immunoreactivity retained}$$

Procedure 2 - Indirect RIA

Appropriate amounts of target cells in 0.2 ml of 10% FCS media were aliquoted into 4 ml polystyrene tubes. Samples to be tested were diluted to a concentration of 2 µg/ml MoAb equivalents in 10% FCS media. Five three-fold serial dilutions were prepared from each 2 µg/ml sample and 0.2 ml was added to each tube in duplicate. Background samples received only cells and media. Cells were incubated at 4°C for 1 hour, then washed 2 times (all RIA washes were done with a 3 ml volume) with 2% FCS media. 0.05 ml of sheep $F(ab')_2$ anti-mouse IgG [$^{125}$I] (DuPont, Cat# NEX 162-0142) containing approximately 500,000 CPM's was added to each tube; cells were incubated an additional hour at 4°C, washed once with 2% FCS and twice with PBS. 0.5 ml of PBS was added to each tube, cells were vortexed, transferred to clean tubes and counted for 1 minute in a Packard Gamma 500.

The % binding of each value was determined and graphed like the preceding ELISA equation, except CPM's were substituted for O.D. units and C = Average CPM's of 1 µg/ml unmanipulated MoAb control. The % immunoreactivity retained of each sample was calculated as previously discussed.

Procedure 3 - Direct RIA

Appropriate amounts of target cells in 1 ml of 10% FCS media were aliquoted into 4 ml polystyrene test tubes, centrifuged and supernatant was discarded. Samples to be tested were diluted to make a concentration of 200 µg/ml MoAb equivalents in 10% FCS media. Five additional five-fold serial dilutions were prepared from each 200 µg/ml sample and 0.05 ml was added to each tube in duplicate. 0.05 ml of $^{125}$I-MoAb was added to each tube (optimal amount is individually determined for each MoAb and batch). Positive control samples contained cells, media and $^{125}$I-MoAb. Background samples contained non-specific cells, media and $^{125}$I-MoAb. Cells were incubated for 1 hour at 4°C, washed once with 2% FCS media, twice with PBS, transferred and counted as previously mentioned.

The % $^{125}$I-MoAb binding inhibition of each sample was calculated by the following formula:

$$\frac{A-B}{C-B} \times 100 = \%\ ^{125}\text{I-MoAb Binding inhibition}$$

A = Average CPM's of sample
B = Average CPM's of background
C = Average CPM's of positive control

The plot and % immunoreactivity retained by each sample was calculated as previously discussed except the $BD_{50}$ is actually $BID_{50}$ (Dose of MoAb needed to give 50% inhibition of the binding of $^{125}$I-MoAb).

Notes:

1) Tubes were always vigorously vortexed immediately after the addition of every reagent in the RIA's.
2) An internal control sample equalling 50% of the unmanipulated MoAb control was included in each set of assays to confirm whether each procedure was quantitative in predicting the conjugates' retention of immunoreactivity.

The results from these assays are tabulated below in Table 4.

Table 4

| Immunoreactivity of MoAb Conjugates | | |
|---|---|---|
| Hydrazide of 3-mercaptopropionic acid disulfide of N-acetyl LL-E33288$\delta_1^I$ (Example 7) conjugated to: | Preparation | Immunoreactivity % of unmodified MoAb control |
| Lym 1 | | 82 |
| CT-M-O1 | | 100 |
| B72.3 | | 100 |

The monoclonal antibody conjugates of this invention are active as anticancer agents. The assay described below for assessing in vitro cytotoxicity shows the dramatic preference of the constructs for target cell lines as opposed to non-target cells, and provides a measure of utility of targeted forms of the compounds compared to their non-targeted counterparts.

Cytotoxicity Assays

In Vitro

Samples to be tested were diluted to a concentration of 0.2 or 0.02 µg/ml of parent drug equivalents (starting concentration is dependent on cell line to be tested and potency of the parent drug). Three or four additional five-fold dilutions were prepared from each original sample dilution and 0.2 ml was added to sterile 15 ml polystyrene tubes. At least one similar conjugate consisting of parent drug and an irrelevant MoAb was included in each assay to determine specificity of the relevant conjugate. $10^5$ appropriate target cells in 0.2 ml of 10% FCS media were aliquoted into the tubes and vortexed. In addition, an identical test was performed utilizing irrelevant cells as targets to further confirm specificity of relevant conjugate. MoAb controls received only equivalent amounts of MoAb and positive control samples received only 10% FCS media.

Cells were incubated at 37°C for 7 minutes then washed 4 times with 8 ml of 2% FCS media. 0.1 ml of 10% FCS was added to each tube, cells were vortexed and 0.2 ml was aliquoted to each well of a sterile 96-well polystyrene tissue culture plate.

Plates were incubated for 2 days in a humidified 37°C incubator with 5% $CO_2$. One half of the media was removed and replaced with fresh media containing 2 µCi/ml $^3$H thymidine (DuPont, NEN, Cat# NET-027). Incubation was continued for 24 hours, cells were frozen, thawed and harvested by a PHD cell harvester (Cambridge Technology, Inc.). Each sample was counted for 1 minute in a Beckman LS 5800 scintillation counter on Channel 1. The % growth inhibition was calculated as follows:

$$\frac{\text{Average CPM of test value}}{\text{Average CPM of media control}} \times 100 = \%\ \text{Growth}$$

$$100 - \%\ \text{Growth} = \%\ \text{Inhibition}$$

The % inhibition was plotted on the non-log scale of a semi-log graph and the parent drug concentration was plotted on the log scale. The $IC_{50}$ (concentration of parent drug needed to give 50% inhibition) of each test sample was derived from the graph and the amount of retention of cytotoxicity was calculated by the following equation:

$$\frac{IC_{50}\ \text{of parent drug}}{IC_{50}\ \text{of test sample}} \times 100 = \%\ \text{Cytotoxicity Retained}$$

The results from the in vitro cytotoxicity assay are tabulated below in Table 5

Table 5

| In Vitro Cytotoxicity of MoAb Conjugates | |
|---|---|
| MoAb | Cytotoxicity |
| Hydrazide conjugate prepared using product of Example 3 with: | % N-acetyl LL-E33288$\delta_1^I$ |
| Lym 1 | 400 |
| CT-M-01 | 700 |

The following assay system was used to measure the in vivo activity of the conjugates of this invention.

In vivo tests for antitumor activity on drug-monoclonal antibody conjugates were done using human tumor xenographs in athymic (nude) mice.

Burkitt lymphoma (Raji) and myeloma (HS Sultan) cells were harvested from culture flasks and inoculated subcutaneously ($\geq 80 \times 10^6$ Raji cells or $40 \times 10^6$ HS Sultan cells) into test mice. Solid tumors, ovarian carcinomas (CA73, Ovcar-3), breast carcinoma (MX-1) and colon carcinoma (LS174T) were propagated in athymic mice, removed, cut into 2 $mm^3$ fragments and implanted subcutaneously into test mice (5-8 fragments per mouse).

Drugs, monoclonal antibodies and drug-monoclonal antibody conjugates were administered intraperitoneally once each 3 days for 3 or 5 total injections starting on day 2, 3, 4, 6, 7 or 8 days after tumor implantation. Tumor measurements (the length and width of the tumor) were made by means of a Fowler ultra CAL II electronic caliper each 7 days for 4 to 6 weeks post tumor implantation. Tumor mass in mg was estimated from the formula:

$$\frac{\text{Length(mm)} \times \text{Width(mm)}}{2}$$

Tumor growth inhibition was calculated for each test group on a percent of control [mean mg of treated (T) divided by mean mg of control (C) x 100]. A T/C value $\leq$ 42% in groups with $\geq$ 65% surviving animals is considered necessary to demonstrate activity.

The results from this assay appear in Table 6.

Table 6

| In Vivo Antitumor Testing Results | | | | |
|---|---|---|---|---|
| | Dosage(mcg) | | Tumor Size (T/C)%control | S/T |
| | MoAb | Drug | | |
| Hydrazide of 3-mercaptopropionic acid disulfide of N-Acetyl LL-E33288$\delta_1^I$ conjugated to CT-M-O1 | 125 | 1.0 | 0 | 6/6 |
| Hydrazide alone | - | 2.0 | 71 | 3/6 |
| Mixture, N-acetyl LL-E33288$\delta_1^I$ plus MoAb CT-M-O1 | 125 | 1.0 | 5 | 1/6 |
| N-acetyl LL-E33288$\delta_1^I$ | - | 1.0 | 46 | 2/6 |
| iv treatment against human breast cancer cell line MX-1, three injections starting on day 2 following tumor implantation, measurements given made on day 42 post-implantation | | | | |
| Hydrazide of 3-mercaptopropionic acid disulfide of N-acetyl LL-E33288$\delta_1^I$ conjugated to Lym 1 | 37 | 1.0 | 11 | 6/6 |
| Hydrazide alone | - | 1.0 | 517 | 6/6 |
| N-acetyl LL-E33288$\delta_1^I$ alone | - | 0.5 | 226 | 6/6 |
| MoAb Lym 1 alone | 37 | - | 178 | 6/6 |
| iv treatment against human Burkitt lymphoma cell line Raji TC, three injections starting on day 7 after tumor implantation, measurements given made on day 35 post-implantation | | | | |
| Hydrazide of 3-mercaptopropionic acid disulfide of N-acetyl LL-E33288$\delta_1^I$ conjugated to B72.3 | 127 | 2.7 | 61 | 6/6 |
| N-acetyl LL-E33288$\delta_1^I$ alone | - | 2.0 | 32 | 1/6 |
| MoAb B72.3 alone | 127 | - | 125 | 6/6 |
| Vincristine (positive control) | - | 20 | 23 | 6/6 |
| iv treatment against human colon cancer cell line LS174T, three injections started on day 8 following tumor implantation, measurements given made on day 21 post-implantation | | | | |
| Hydrazide of 3-mercaptopropionic acid disulfide of N-acetyl LL-E33288$\delta_1^I$ conjugated to CT-M-O1 | 112 | 2.7 | 40 | 5/6 |
| N-acetyl LL-E33288$\delta_1^I$ alone | - | 2.0 | 32 | 1/6 |
| Vincristine (positive control) | - | 20 | 23 | 6/6 |
| iv treatment against human colon carcinoma line LS174T, three injections started 8 days after tumor implantation, measurements given made on day 21 post-implantation | | | | |

The invention will be further described in conjunction with the following non-limiting examples.

Reference Example 1

3-Mercaptopropionic Acid Disulfide of LL-E33288$\gamma_1^I$

To a solution of 90 mg of LL-E33288$\gamma_1^I$ in 90 ml of acetonitrile was added 10.6 mg of 3-mercaptopropionic acid in 1 ml of acetonitrile. The solution was vortexed and then stored at -20°C for 6 days. The solvent was removed *in vacuo*

and the residue chromatographed over 10 ml of silica gel in methylene chloride. The column was developed with 50 ml of methylene chloride, 50 ml of 4% methanol in methylene chloride and finally 100 ml of 8% methanol in methylene chloride. Evaporation of this last fraction gave a residue which was taken up in ethyl acetate with the aid of a little acetone and added dropwise to an excess of hexane. The precipitate was collected and dried, giving 39 mg of the desired product (FABMS, M+H 1394).

Reference Example 2

N-Hydroxysuccinimidyl 3-mercaptopropionate disulfide of LL-E33288$\gamma_1^I$

To a solution of 5 mg of the 3-mercaptopropionic acid disulfide analog of LL-E33288$\gamma_1^I$ from EPO publication in 0.5 ml of tetrahydrofuran was added 0.45 mg of N-hydroxysuccinimide in 0.1 ml of tetrahydrofuran and then 1.8 mg of dicyclohexylcarbodiimide in 0.2 ml of tetrahydrofuran. The reaction was allowed to stir at room temperature for 4 hours and was then quenched with a large excess of hexanes. The solid was isolated by filtration and dissolved in ethyl acetate. The resulting solution was washed three times with brine, dried with magnesium sulfate, and evaporated to 5 mg of the desired product as a tan powder which was used without further purification. Retention time on reverse phase $C_{18}$ HPLC: 15 minutes with 40% acetonitrile/0.1 M aqueous ammonium acetate (starting material: 6.0 minutes).

Example 3

3-Mercaptopropionyl hydrazide disulfide of N-acetyl LL-E33288$\gamma_1^I$

To 10 mg of N-acetyl LL-E33288$\gamma_1^I$ in 10 ml of acetonitrile at -15°C was added 1.5 eq of 3-mercaptopropionyl hydrazide in 85 µl acetonitrile. One equivalent of triethylamine was added. The reaction was allowed to stir at 0°C for two hours and the solvent was then evaporated. The residue was chromatographed on silica gel with a 10-15% methanol-in-chloroform gradient to yield the desired product. FABMS, m/z=1450(M+H); retention time on $C_{18}$ reverse phase HPLC:2.5 min. with 50% acetonitrile/0.05 M aqueous ammonium dihydrogen phosphate (N-acetyl LL-E33288$\gamma_1^I$:6.6 min. in the same system).

Example 4

3-Mercaptoisovaleryl hydrazide disulfide of N-acetyl LL-E33288$\gamma_1^I$

To 20 mg of N-acetyl LL-E33288$\gamma_1^I$ in 15 ml of acetonitrile at -15°C was added 3 eq of 3-mercaptoisovaleryl hydrazide in 6.2 ml acetonitrile. One equivalent of triethylamine was added. The reaction was allowed to stir at ambient temperature for 2 hours and the solvent was then exaporated. The residue was chromatographed on silica gel with a 10-15% methanol-in-chloroform gradient to yield the desired product. Retention time on $C_{18}$ reverse phase HPLC:2.5 min. with 50% acetonitrile/0.05 M aqueous ammonium dihydrogen phosphate (N-acetyl LL-E33288$\gamma_1^I$: 6.6 min. in the same system).

Example 5

3-Mercaptobutyryl hydrazide disulfide of N-acetyl LL-E33288$\gamma_1^I$

To 10 mg of N-acetyl LL-E33288$\gamma_1^I$ in 7.5 ml off acetonitrile at -15°C was added 3 eq of 3-mercaptobutyryl hydrazide in 5 ml acetonitrile. One equivalent of triethylamine was added. The reaction was allowed to stir at ambient temperature for 10 hours and the solvent was then evaporated. The residue was chromatographed on silica gel with a 10-15% methanol-in-chloroform gradient to yield the desired product. Retention time on $C_{18}$ reverse phase HPLC:7.3 min. with 43% acetonitrile/0.05 M aqueous ammonium dihydrogen phosphate (N-acetyl LL-E33288$\gamma_1^I$:5.6 min. in the same system).

Example 15

p-Mercaptodihydrocinnamoyl hydrazide disulfide of N-acetyl LL-E33288$\gamma_1^I$

To 10 mg of N-acetyl LL-E33288$\gamma_1^I$ in 7.5 ml of acetonitrile at -15°C was added 3.0 eq of p-mercaptodihydrocinnamoyl hydrazide in 2.0 ml acetonitrile. One equivalent of triethylamine was added. The reaction was allowed to stir at ambient temperature for 2 hours and the solvent was then evaporated. The residue was chromatographed on silica gel

with a 10-15% methanol-in-chloroform gradient to yield the desired product. Retention time on $C_{18}$ reverse phase HPLC:7.3 min. with 43% acetonitrile/0.05 M aqueous ammonium dihydrogen phosphate (N-acetyl LL-E33288$\gamma_1{}^I$:5.6 min. in the same system).

Example 16

Non-specific conjugation to proteins

The hydroxysuccinimide enter described in Example 3 was covalently attached to antibodies under slightly alkaline conditions. The following is a general procedure used to make the antibody conjugates listed in Table 7. Antibody at a concentration of 3-5 mg/ml in phosphate buffer containing 0.1M sodium chloride, pH 7.5 was reacted with a 5-20-fold molar excess of the product from Example 3 with stirring, at room temperature for from 1-4 hours. The conjugated protein was desalted chromatographically and aggregated protein was separated from monomeric material by gel filtration HPLC. Monomeric fractions were pooled and concentrated.

Non-specific conjugates prepared using the product of Example 2

| MoAb | Drug Loading M/M |
|---|---|
| Lym 1 | 5.2 |
| B72.3 | 6.0 |
| B72.3 | 2.9 |

Example 8

Site-specific conjugate preparation

The general method for attaching hydrazide derivatives of drugs to oxidized antibodies is described in T. J. McKearn, et al., in U.S. Patent No. 4,671,958. The procedure has been applied to preparing antibody conjugates from the products of Examples 4 to 15 with specific modifications as described below. The products from these reactions and their characteristics are summarized in Table 7.

(A) Antibody Oxidation Antibody at a concentration of 5 to 10 mg/ml was dialyzed overnight against a 200 fold volume of 50mM sodium acetate buffer, pH 5.5 containing 0.1M sodium chloride (Buffer A). After dialysis, the MoAb was oxidized with 15mM to 200mM periodic acid in 0.2M sodium acetate. The oxidation was allowed to proceed in the dark, with stirring, at 4°C for 45 minutes after which time the oxidized MoAb was desalted on a ≥5 bed volume Sephadex G-25 column. The degree of oxidation of the antibody was assessed by reaction with p-nitrophenylhydrazine and comparing absorbance of the protein at 280mm vs. p-nitrophenylhydrazine at 395mm.

(B) Drug Hydrazide Conjugation The oxidized MoAb was reacted with 10 to 200-fold molar excess of drug hydrazide. The hydrazides were dissolved into dimethylformamide and added to the aqueous solution of MoAb. To avoid precipitation of MoAb, the final volume of dimethylformamide added did not exceed 10% of the total reaction volume. Reaction was allowed to proceed for 3 hours at room temperature, with stirring. To prevent crosslinking of unreacted aldehydes and subsequent aggregation, a blocking agent, acetyl hydrazide was added in 100-fold molar excess three hours after addition of the drug hydrazide. To stabilize the Schiff's base linkage between aldehyde and drug hydrazide (a hydrazone), the product generally was reduced to an alkyl hydrazine by the addition of 10mM sodium cyanoborohydride, allowing the reaction to proceed for one more hour (total conjugation time - 4 hours). The conjugate was chromatographically desalted and exhaustively dialyzed (minimum time 48 hours) into pH 6.5 phosphate buffer for storage and testing.

Conjugates were analyzed for the presence of aggregates by gel filtration HPLC and for free drug by reverse phase HPLC. Drug loading was determined spectroscopically using the extinction coefficients of both the antibody and the drug to estimate molar concentrations of drug in conjugates.

Hydrazide conjugates prepared from the product of Example 3

| | | |
|---|---|---|
| Lym 1 | | 2.8 |
| Lym 2 | | 1.4 |
| B72.3 | #1 | 2.1 |
| | #2 | 2.4 |
| CT-M-01 | #1 | 1.6 |
| | #2 | 3.6 |
| | #3 | 2.5 |
| | #4 | 2.4 |

## Claims

**Claims for the following Contracting States : AT, BE, CH, DE, DK, FR, GB, IT, LI, NL, SE**

1. A substituted disulfide of formula Q-Sp-SS-W, prepared from a compound of formula $CH_3SSS$-W which is an N-acyl derivative of an antitumor antibiotic designated as LL-E33288, $\alpha_1$-I, $\alpha_2$-Br, $\alpha_2$-I, $\beta_1$-Br, $\beta_1$-I, $\gamma_1$-Br, $\gamma_1$-I, $\delta_1$-I, BBM-1675, FR-900405, FR-900406, PD114759, PD115028, CL-1577A, CL-1577B, CL-1577D, CL-1577E, CL-1724, wherein

W is 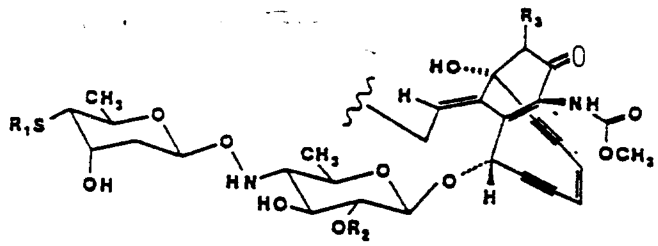

$R_1$ is 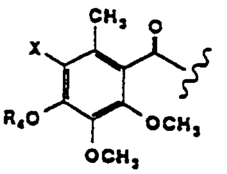 or $CH_3$;

$R_2$ is 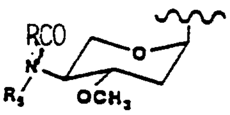 ;

$R_3$ is 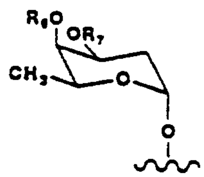 or H;

$R_4$ is 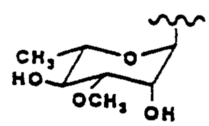 or H;

$R_6$ or $R_7$ is H or 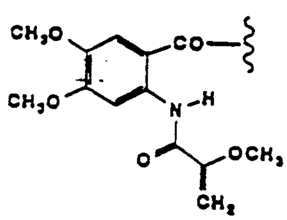

$R_5$ is $CH_3$, $C_2H_5$, or $(CH_3)_2CH$;
X is an iodine or bromine atom;
R is hydrogen or a branched or unbranched alkyl ($C_1$-$C_{10}$) or alkylene ($C_1$-$C_{10}$) group, an aryl or heteroaryl group, or an aryl-alkyl ($C_1$-$C_5$) or heteroaryl-alkyl ($C_1$-$C_5$) group, all optionally substituted by one or more hydroxy, amino, carboxy,' halo, nitro, lower ($C_1$-$C_3$) alkoxy, or lower ($C_1$-$C_5$) thioalkoxy groups; Sp is straight or branched-chain divalent or trivalent ($C_1$-$C_{18}$) radicals, divalent or trivalent aryl or heteroaryl radicals, divalent or trivalent ($C_3$-$C_{18}$) cycloalkyl or heterocyclo-alkyl radicals, divalent or trivalent aryl- or heteroaryl-alkyl ($C_1$-$C_{18}$) radicals, divalent or trivalent cycloalkyl- or heterocycloalkyl-alkyl ($C_1$-$C_{18}$) radicals, or divalent or trivalent ($C_2$-$C_{18}$) unsaturated alkyl radicals, wherein if Sp is a trivalent radical, it can be additionally substituted by amino, alkylamino, arylamino, heteroarylamino, carboxyl, lower alkoxy, hydroxy, thiol, or lower alkylthio groups; Q is hydrogen, halogen, amino, alkylamino, dialkylamino, arylamino, heteroarylamino, piperidino, pyrrolidino, piperazino, carboxyl, nitrophenyloxycarbonyl, carboxaldehyde, lower alkoxy, hydroxy, thiol, lower alkyldicarboxyl, $-CONHNH_2$, $-NHCONHNH_2$, $-CSNHNH_2$, $-NHCSNHNH_2$, or $-ONH_2$; with the provisos that when Sp is ethylidene, Q cannot be hydrogen, and when $CH_3$-SSS-W is LL-E33288 , $\alpha_1$-I, $\alpha_2$-Br, $\alpha_2$-I, , $\beta_1$-Br, $\beta_1$-I, $\gamma_1$-Br, $\gamma_1$-I, $\delta_1$-I, BBM-1675, FR-900405, FR-900406, PD114759, PR115028, CL-1577A, CL-1577B, CL-1577D, CL-1577E, or CL-1724 and Sp is divalent, Q can not be hydrogen, halogen, amino, alkylamino, dialkylamino, arylamino, heteroarylamino, piperidino, pyrrolidino, piperazino, or carboxyl.

2. A substituted disulfide of formula Q-Sp-SS-W, according to Claim 1 prepared from a compound of formula $CH_3SSS$-W wherein:

W is 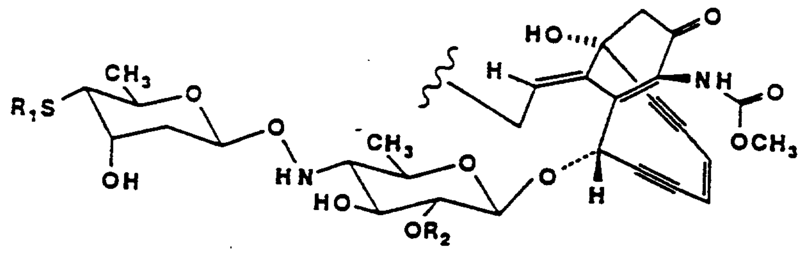

$R_1$ is 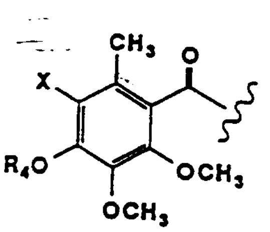 ;

$R_2$ is 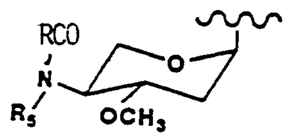 ;

$R_4$ is 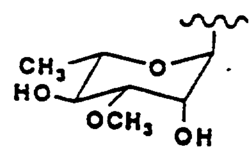 or H;

$R_5$ is $CH_3$, $C_2H_5$, or $(CH_3)_2CH$; X is an iodine or bromine atom;
wherein R is hydrogen, $CH_3$, or a mono-substituted aryl group; and Sp and Q are as defined.

3. A carrier-drug conjugate of the formula

$$\underset{(Y)_{n-m}}{\overset{|}{\text{Tu}}}-(\text{Z-Sp-SS-W})_m$$

prepared from a compound or formula ($CH_3SSS$-W) wherein $CH_3SSS$-W is an N-acyl derivative of an antitumor antibiotic designated as LL-E33288, $\alpha_1{}^I$, $\alpha_2{}^{Br}$, $\alpha_2{}^I$, $\beta_1{}^{Br}$, $\beta_1{}^I$, $\gamma_1{}^{Br}$, $\gamma_1{}^I$, $\delta_1{}^I$, BBM-1675, FR-900405, FR-900406, PD114759, PD115028, CL-1577A, CL-1577B, CL-1577D, CL-1577E, CL-1724, wherein W is as hereinbefore described in claim 1 comprising:

reacting $CH_3SSS$-W with a compound of general formula Q-Sp-SH, wherein Sp is a straight or branched-chain divalent or trivalent ($C_1$-$C_{18}$) radical, divalent or trivalent aryl or heteroaryl radical, divalent or trivalent ($C_3$-$C_{18}$)

cycloalkyl or heterocycloalkyl radical, divalent or trivalent aryl- or heteroaryl-alkyl ($C_1$-$C_{18}$) radical, divalent or trivalent cycloalkyl- or heterocycloalkyl-alkyl ($C_1$-$C_{18}$) radical or divalent or trivalent ($C_2$-$C_{18}$) unsaturated alkyl radical, wherein if Sp is a trivalent radical, it can be additionally substituted by amino, alkylamino , arylamino, heteroarylamino, carboxyl, lower alkoxy, hydroxy, thiol, or lower alkylthio groups, with the proviso that when $CH_3$-SSS-W is the parent antitumor antibiotic LL-E33288$\alpha_1$-Br, $\alpha_1$-I, $\alpha_2$-Br, $\alpha_2$-I, $\beta_1$-Br, $\beta_1$-I, $\gamma_1$-Br, $\gamma_1$-I, $\delta_1$-I, BBM-1675, FR-900405, FR-900406, PD114759, PR115028, CL-1577A, CL-1577B, CL-1577D, CL-1577E, or CL-1724 itself, Sp can not be divalent; and Q is, or can be subsequently converted to, halogen, amino, alkylamino, carboxyl, carboxaldehyde, hydroxy, thiol, $\alpha$-haloacetyloxy, lower alkyldicarboxyl, -$CONHNH_2$, -$NHCONHNH_2$, -$NHCSNHNH_2$, -$ONH_2$, -$CON_3$,

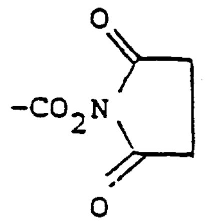 , 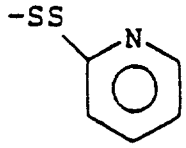 , 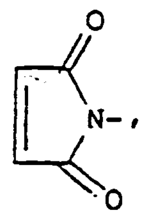 , 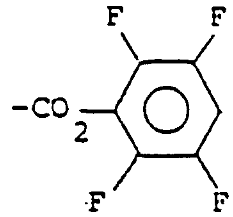 ,

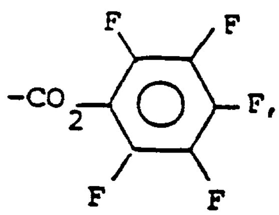 , 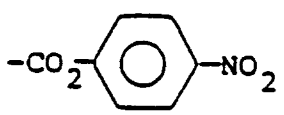 , 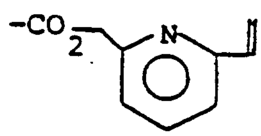 , 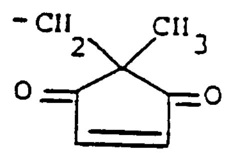

to produce an intermediate of formula Q-Sp-SS-W, wherein Q, Sp, and W are as hereinbefore defined, reacting Q-Sp-SS-W with a molecule of the formula Tu-$(Y)_n$ wherein the carrier Tu is defined as a mono- or polyclonal antibody, its fragments, its chemically or genetically manipulated counterparts, or growth factors or steroids; Y is a side-chain amino, carboxy, or thiol group of a protein, an aldehyde derived from glycoprotein carbohydrate residues, or an amidoalkylthio group; and n is an integer of from 1 to 100, to produce a compound of the formula:

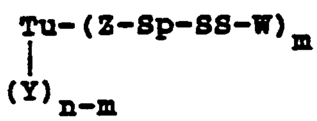

wherein Tu, Y, Sp, W, and n are as hereinbefore defined, and Z is formed from covalent reaction of the groups Q and Y directly or after subsequent reduction, and Z is -CONH-, -CONHN=CH-, -$CONHNHCH_2$-, -NHCONHN=CH-, -$NHCONHNHCH_2$-, -NHCSNHN=CH-, -$NHCSNHNHCH_2$-, -ON=CH-, -NH-, -$NHCH_2$-, -N=CH-, -$CO_2$-, -$NHCH_2CO_2$-, -SS-,

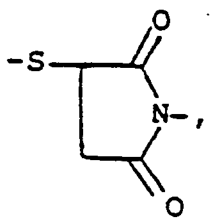,
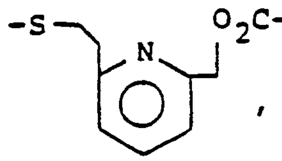,
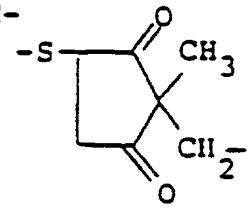 or
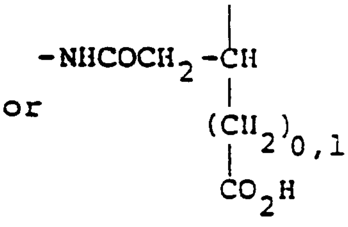

and m is 0.1 to 15.

**4.** A protein-drug conjugate according to Claim 3 of the formula

$$\underset{(Y)_{n-m}}{\overset{|}{Tu}}-(Z-Sp-SS-W)_m$$

prepared from the class of N-acyl derivatives of antitumor antibiotics designated LL-E33288 ($CH_3SSS$-W) comprising:

displacing the dithiomethyl moiety with a compound of formula Q-Sp-SH, wherein Sp is straight or branched-chain divalent or trivalent ($C_2$-$C_{10}$) radicals or divalent or trivalent aryl- or heteroaryl-alkyl ($C_2$-$C_5$) radicals, wherein if Sp is a trivalent radical, it can be additionally substituted by amino, heteroarylamino, hydroxy, or thiol groups; and Q is, or can be subsequently converted to, carboxyl, lower alkyldicarboxylanhydride, -$CO_2$Su, -$CONHNH_2$, or

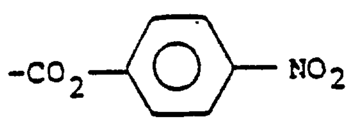

to produce an intermediate of general formula Q-Sp-SS-W, wherein Q, Sp, and W are as hereinbefore defined, reacting Q-Sp-SS-W with a molecule of the formula Tu-$(Y)_n$ wherein Tu is a monoclonal antibody which exhibits preferential reactivity with a human tumor-associated antigen, Y is a side-chain amino group on the antibody, or an aldehyde generated by oxidation of the carbohydrate groups of the antibody, and n is an integer of from 1 to 100, to produce a compound of the formula:

$$\underset{(Y)_{n-m}}{\overset{|}{Tu}}-(Z-Sp-SS-W)_m$$

wherein Tu, Y, Sp, W, and n are as hereinbefore defined, and Z is formed from covalent reaction of the groups Q and Y directly or after subsequent reduction, and Z is -CONH-, -CONHN=CH-, -CONHNHCH$_2$-, or

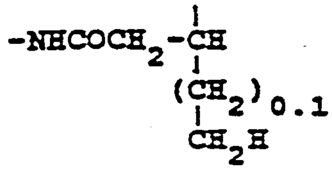

and m is 0.1 to 15.

5. A process for producing a substituted disulfide of formula Q-Sp-SS-W from a compound of formula $CH_3SSS$-W which is an N-acyl derivative of an antitumor antibiotic designated as LL-E33288, $\alpha_1$-I, $\alpha_2$-Br, $\alpha_2$-I, $\beta_1$-Br, $\beta_1$-I, $\gamma_1$-Br, $\gamma_1$-I, $\delta_1$-I, BBM-1675, FR-900405, FR-900406, PD114759, PD115028, CL-1577A, CL-1577B, CL-1577D, CL-1577E, CL-1724, wherein

W is 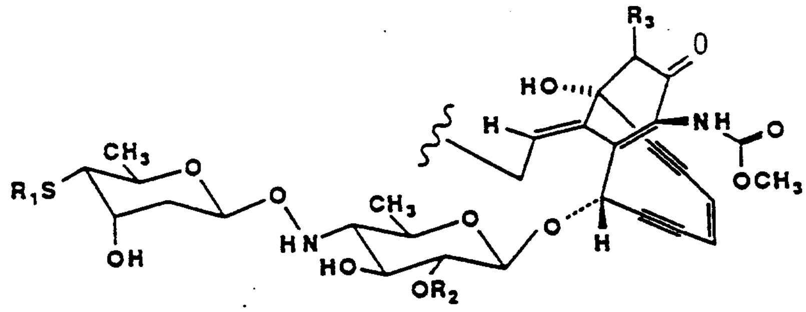

R₁ is 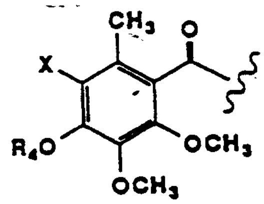 or $CH_3$;

R₂ is 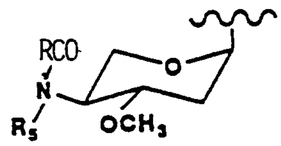 ;

R₃ is 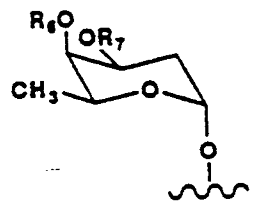 or H;

R₄ is 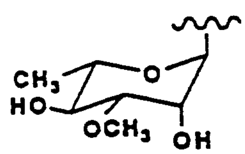 or H;

R₆ or R₇ is H or 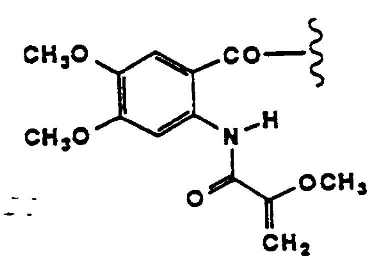

$R_5$ is $CH_3$, $C_2H_5$, or $(CH_3)_2CH$;
X is an iodine or bromine atom;
R is hydrogen or a branched or branched alkyl ($C_1$-$C_{10}$) or alkylene ($C_1$-$C_{10}$) group, an aryl or heteroaryl group, or an aryl-alkyl ($C_1$-$C_5$) or heteroaryl-alkyl ($C_1$-$C_5$) group, all optionally substituted by one or more hydroxy, amino, carboxy, halo, nitro, lower ($C_1$-$C_3$) alkoxy, or lover ($C_1$-$C_5$) thioalkoxy groups; Sp is straight or branched-chain divalent or trivalent ($C_1$-$C_{18}$) radicals, divalent or trivalent aryl or heteroaryl radicals, divalent or trivalent ($C_3$-$C_{18}$) cycloalkyl or heterocyclo-alkyl radicals, divalent or trivalent aryl- or hetoroaryl-alkyl ($C_1$-$C_{18}$) radicals,

divalent or trivalent cycloalkyl- or heterocycloalkyl-alkyl ($C_1$-$C_{18}$) radicals, or divalent or trivalent ($C_2$-$C_{18}$) unsaturated alkyl radicals, wherein it Sp is a trivalent radical, it can be additionally substituted by amino, alkylamino, arylamino, heteroarylamino, carboxyl, lower alkoxy, hydroxy, thiol, or lower alkylthio groups; Q is hydrogen, halogen, amino, alkylamino, dialkylamino, arylamino, heteroarylamino, piperidino, pyrrolidino, piperazino, carboxyl, nitrophenyloxylcarbonyl, carboxaldehyde, lower alkoxy, hydroxy, thiol, lower alkyldicarboxyl, -$CONHNH_2$, -$NHCONHNH_2$, -$CSNHNH_2$, -$NHCSNHNH_2$, or -$ONH_2$; with the provisos that when Sp is ethylidene, Q cannot be hydrogen; and when $CH_3$-SSS-W is LL-E33288 , $\alpha_1$-I, $\alpha_2$-Br, $\alpha_2$-I, $\beta_1$-Br, $\beta_1$-I, $\gamma_1$-Br, $\gamma_1$-I, $\delta_1$-I, BBM-1675, FR-900405, FR-900406, PD114759, PR115028, CL-1577A, CL-1577B, CL-1577D, CL-1577E, or CL-1724 and Sp is divalent, Q can not be hydrogen, halogen, amino, alkylamino, dialkylamino, arylamino, heteroarylamino, piperidino, pyrrolidino, piperazino, or carboxyl;

which comprises reacting a methyl trisulfide antitumor antibiotic from those of the above with an appropriately substituted mercaptan in acetonitrile or a combination of acetonitrile and tetrahydrofuran or acetonitrile and dimethylformamide, at a temperature of between -20° to about +40°C, for a period of from one hour to six days, preferably in the presence of one equivalent of a tertiary amine base or a mixture of one equivalent of a tertiary amine base and one equivalent of an organic carboxylic acid.

**6.** A process for preparing the targeted derivatives

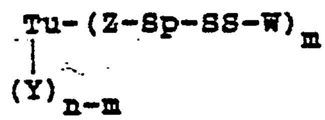

of compounds of formula $CH_3SSS$-W wherein $CH_3SSS$-W is an N-acyl derivative of an antitumor antibiotic LL-E33288, $\alpha_1^{I}$, $\alpha_2^{Br}$, $\alpha_2^{I}$, $\beta_1^{Br}$, $\beta_1^{I}$, $\gamma_1^{Br}$, $\gamma_1^{I}$, $\delta_1^{I}$, BBM-1675, FR-900405, FR-900406, PD 114759, PD 115028, CL-1577A, CL-1577B, CL-1577D, CL-1577E, CL-1724, comprising

reacting $CH_3SSS$-W with a compound of formula Q-Sp-SH, wherein Sp is a straight or branched-chain divalent or trivalent ($C_1$-$C_{18}$) radical, divalent or trivalent aryl or heteroaryl radical, divalent or trivalent ($C_3$-$C_{18}$) cycloalkyl or heterocycloalkyl radical, divalent or trivalent aryl- or heteroaryl-alkyl ($C_1$-$C_{18}$) radical, divalent or trivalent cycloalkyl- or heterocycloalkyl-alkyl ($C_1$-$C_{18}$) radical or divalent or trivalent ($C_2$-$C_{18}$) unsaturated alkyl radical, wherein if Sp is a trivalent radical, it can be additionally substituted by amino, alkylamino, arylamino, heteroarylamino, carboxyl, lower alkoxy, hydroxy, thiol, or lower alkylthio groups, with the proviso that when $CH_3$-SSS-W is the parent antitumor antibiotic LL-E33288, $\alpha_1$-I, $\alpha_2$-Br, $\alpha_2$-I, $\beta_1$-Br, $\beta_1$-I, $\gamma_1$-Br, $\gamma_1$-I, $\delta_1$-I, BBM-1675, FR-900405, FR-900406, PD114759, PR115028, CL-1577A, CL-1577B, CL-1577D, CL-1577E, or CL-1724 itself, Sp can not be divalent; and Q is halogen, amino, alkylamino, carboxyl, carboxaldehyde, hydroxy, lower alkyldicarboxyl anhydride, -$CONHNH_2$, -$NHCONHNH_2$, -$NHCSNHNH_2$, -$ONH_2$, or

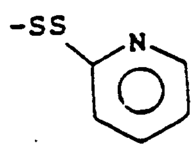

in acetonitrile in the presence of one equivalent of triethylamine or one equivalent of triethylamine and one equivalent of acetic acid at -10° to -30°C for 1-48 hours,
isolating the intermediate of formula Q-Sp-SS-W, wherein Q, Sp, and W are as hereinbefore defined, then reacting the compound of formula Q-Sp-SS-W, wherein Sp and W are as hereinbefore defined and Q is halogen, amino, alkylamino, carboxyl, carboxaldehyde, hydroxy, or lower alkyldicarboxylic anhydride with a molecule of the formula Tu-$(Y)_n$ wherein Tu is a mono- or polyclonal antibody, its fragments, its chemically or genetically manipulated counterparts, or growth factors or steroids; Y is a side-chain amino or carboxy functionality; n is 1-100, in aqueous buffer at a pH of between 6.5 and 9, at 4° to 40°C either directly or in the presence of a water-soluble carbodiimide, to generate the compound

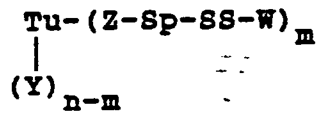

wherein Tu, Sp, W, n, and Y are as hereinbefore defined, m is 1-15 and Z is formed from covalent reaction of the groups Q and Y and is -CONH-, -NH-,

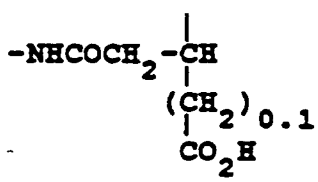

-N=CH-, or $-CO_2-$

or

reacting the compound of formula Q-Sp-SS-W, wherein Sp and W are as hereinbefore defined and Q is a carboxylic acid, with N-hydroxysuccinimide, 2,3,5,6-tetrafluorophenol, pentafluorophenol, or 4-nitrophenol in the presence of a carboxyl activating agent such as a carbodiimide to generate a compound of formula Q-Sp-SS-W wherein Sp and W are as hereinbefore defined and Q is

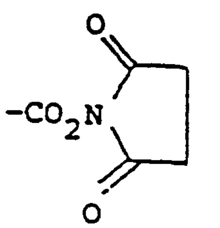
,
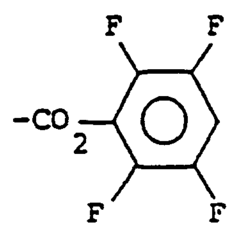
,
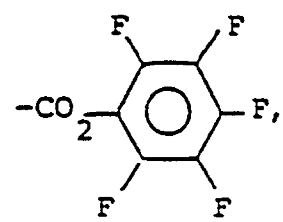

or

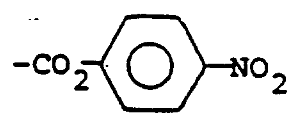

with a molecule of formula $Tu-(Y)_n$,

where Tu and n are as hereinbefore defined, and Y is a side-chain amino group, in an aqueous buffered solution at a pH between 6.5 and 9, at a temperature of between 4° and 40°C, inclusive, to generate compounds of the formula:

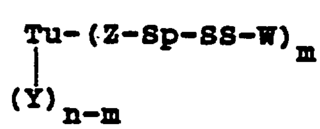

wherein Tu, Sp, Y, and n are as hereinbefore defined, m is 1-15, and Z is formed from covalent reaction between Q and Y and is defined as -CONH-

or

reacting a compound of formula Q-Sp-SS-W, wherein Sp and W are as hereinbefore defined and Q is -$CONHNH_2$ with nitrous acid in agueous acetonitrile to generate a compound of formula Q-Sp-SS-W, wherein Sp and W are as hereinbefore defined and Q is -$CON_3$ with a compound of formula Tu-$(Y)_n$, wherein Tu, Y, and n are as hereinabove defined to produce a compound of the formula

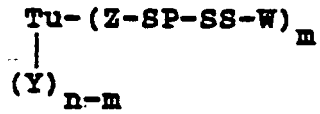

wherein Tu, Z, Sp, W, m, Y, and n are as hereinabove defined;

or

reacting a compound of formula Q-Sp-SS-W wherein Sp and W are as hereinbefore defined and Q is hydroxy, with an alpha-haloacetic anhydride to produce a compound wherein Q is $\alpha$-haloacetyloxy, and reacting the $\alpha$-haloacetyloxy-Sp-SS-W, or a compound of formula Q-Sp-SS-W, wherein Sp and W are as hereinbefore defined and Q is

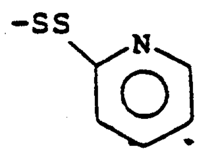 , 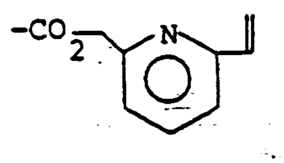 , or 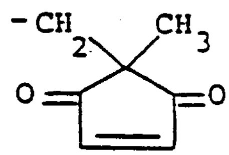

with a molecule of the formula Tu-$(Y)_n$ wherein Tu is as hereinbefore defined, Y is a side-chain thiol of a protein, or an amidoalkylthio group introduced on an amine of Tu using reagents such as 3-(2-dithiopyridyl)propionic acid hydroxysuccinimide ester followed by reduction with an agent such as dithiothreitol, or an amidoalkylthio group introduced on an amine of Tu using 2-iminothiolane, and n is 1-10, under aqueous buffered conditions at a pH between 4.5 and 7, at a temperature between 4° and 40°C, inclusive, to produce a compound of formula:

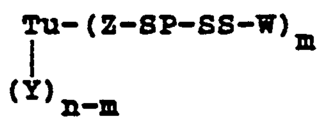

wherein Tu, Sp, W, and n are as hereinbefore defined, and Z is formed from covalent reaction of the groups Q and Y and Z is

-SS-,

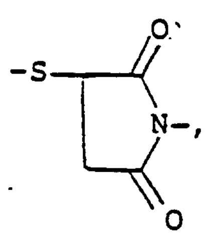 , 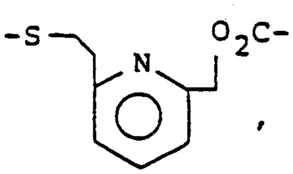 , or 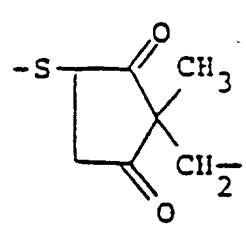

and n is 0.1 to 10;

or

reacting a compound of the formula Q-Sp-SS-W wherein Sp and W are as hereinbefore defined and Q is $-NH_2$, $-CONHNH_2$, $-NHCONHNH_2$, $-NHCSNHNH_2$, or $-ONH_2$ with a molecule of formula Tu-$(Y)_n$ wherein Tu is as hereinbefore defined, Y is an aldehyde generated from carbohydrate residues on Tu by oxidation in the presence of an alkaline earth periodate, in an aqueous buffer at a pH between 4.0 and 6.5, at 4° to 40°C, inclusive, and n is 1 to 20 to generate a compound of formula:

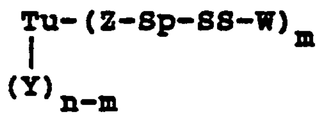

wherein Tu, Sp, W, Y, and n are as hereinbefore defined and Z is formed from the covalent reaction of Q and Y and is -ON=CH-, -N=CH-, -CONHN=CH-, -NHCONHN=CH-, or -NHCSNHN=CH-, and m is 0.1 to 15; or treating the compound immediately hereinabove of formula:

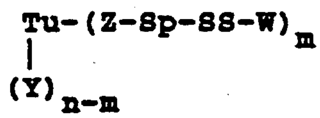

wherein Tu, Z, Sp, W, Y, n, and m are as immediately hereinabove defined with acetylhydrazine or tyrosine hydrazine in an aqueous buffer at a pH between 4.0 and 6.5, at 4° to 40°C, inclusive, to generate a compound of formula:

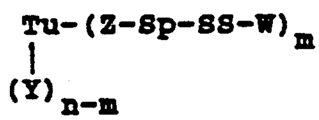

wherein Tu, Z, Sp, W, n, and m are as immediately hereinabove defined and Y is $-CH=NNHCOCH_3$ or

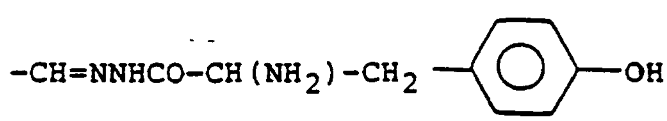

and

reacting this compound with sodium cyanoborohydride or sodium borohydride, in an aqueous buffer at a pH of 4.0 to 6.5, at a temperature of 4° to 40°C, inclusive, to generate a compound of formula:

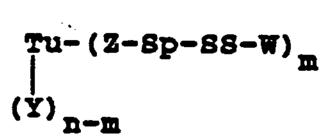

wherein Tu, Sp, W, m, and n are as hereinabove defined, Z is $-NH-CH_2-$, $-CONHNHCH_2-$, $-NHCONHNHCH_2-$, or $-NHCSNHNHCH_2-$, and Y is $-CH_2NHNHCOCH_3$ or

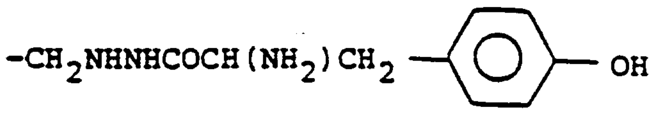

7. A product containing a substituted disulfide analog of formula Q-Sp-SS-W, prepared from a compound of formula $CH_3SSS$-W which is an N-acyl derivative of LL-E33288, $\alpha_1^{I}$, $\alpha_2^{Br}$, $\alpha_2^{I}$, $\beta_1^{Br}$, $\beta_1^{I}$, $\gamma_1^{Br}$, $\gamma_1^{I}$, $\delta_1^{I}$, BBM-1675, FR-900405, FR-900406, PD 114759, PD 115028, CL-1577A, CL-1577B, CL-1577D, CL-1577E, CL-1724, for use in bacterial infections therapy wherein

W is 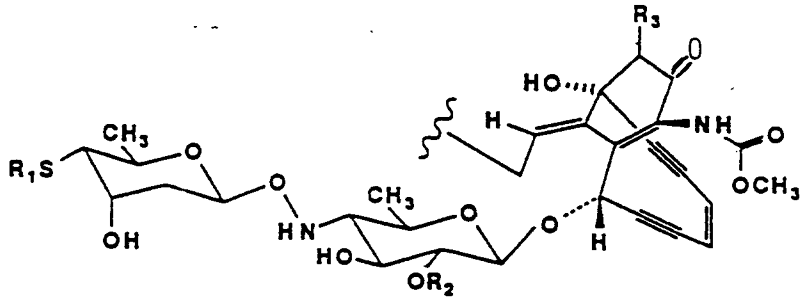

$R_1$ is 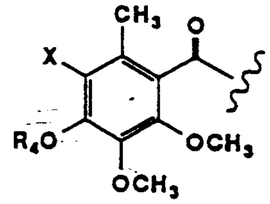 or $CH_3$;

$R_2$ is 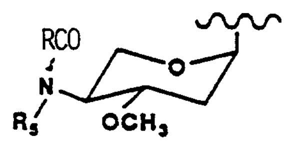 ;

$R_3$ is 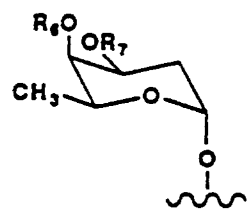 or H;

$R_4$ is 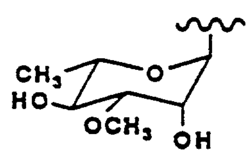 or H;

$R_6$ or $R_7$ is H or 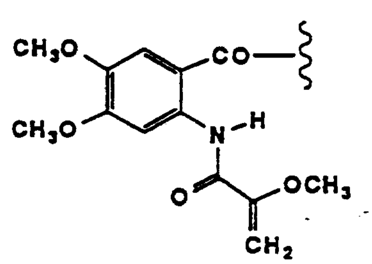

$R_5$ is $CH_3$, $C_2H_5$, or $(CH_3)_2CH$;
X is an iodine or bromine atom;
R is hydrogen or a branched or unbranched alkyl ($C_1$-$C_{10}$) or alkylene ($C_1$-$C_{10}$) group, an aryl or heteroaryl group, or an aryl-alkyl ($C_1$-$C_5$) or heteroaryl-alkyl ($C_1$-$C_5$) group, all optionally substituted by one or more hydroxy, amino, carboxy, halo, nitro, lower ($C_1$-$C_3$) alkoxy, or lower ($C_1$-$C_5$) thioalkoxy groups; Sp is straight or branched-chain divalent or trivalent ($C_1$-$C_{18}$) radicals, divalent or trivalent aryl or heteroaryl radicals, divalent or trivalent ($C_3$-$C_{18}$) cycloalkyl or heterocyclo-alkyl radicals, divalent or trivalent aryl- or heteroaryl-alkyl ($C_1$-

$C_{18}$) radicals, divalent or trivalent cycloalkyl- or heterocycloalkyl-alkyl ($C_1$-$C_{18}$) radicals, or divalent or trivalent ($C_2$-$C_{18}$) unsaturated alkyl radicals, wherein, if Sp is a trivalent radical, it can be additionally substituted by amino, alkylamino, arylamino, heteroarylamino, carboxyl, lower alkoxy, hydroxy, thiol, or lower alkylthio groups; Q is hydrogen, halogen, amino, alkylamino, dialkylamino, arylamino, heteroarylamino, piperidino, pyrrolidino, piperazino, carboxyl, nitrophenyloxycarbonyl, carboxaldehyde, lower alkoxy, hydroxy, thiol, lower alkyldicarboxyl, -$CONHNH_2$, -$NHCONHNH_2$, -$CSNHNH_2$, -$NHCSNHNH_2$, or -$ONH_2$; with the provisos that when Sp is ethylidene, Q cannot be hydrogen and when $CH_3$-SSS-W is LL-E33288 , $\alpha_1$-I, $\alpha_2$-Br, $\alpha_2$-I, $\beta_1$-Br, $\beta_1$-I, , $\gamma_1$-Br, $\gamma_1$-I, $\delta_1$-I, BBM-1675, FR-900405, FR-900406, PD114759, PR115028, CL-1577A, CL-1577B, CL-1577D, CL-1577E, or CL-1724 and Sp is divalent, Q can not be hydrogen, halogen, amino, alkylamino, dialkylamino, arylamino, heteroarylamino, piperidino, pyrrolidino, piperazino, or carboxyl.

**8.** A product containing a substituted disulfide of formula Q-Sp-SS-W, prepared from a compound of formula $CH_3$SSS-W which is an N-acyl derivative of LL-E33288, $\alpha_1{}^I$, $\alpha_2{}^{Br}$, $\alpha_2{}^I$, $\beta_1{}^{Br}$, $\beta_1{}^I$, $\gamma_1{}^{Br}$, $\gamma_1{}^I$, $\delta_1{}^I$, BBM-1675, FR-900405, FR-900406, PD 114759, PD 115028, CL-1577A, CL-1577B, CL-1577D, CL-1577E, CL-1724, for use in tumor therapy, wherein

W is 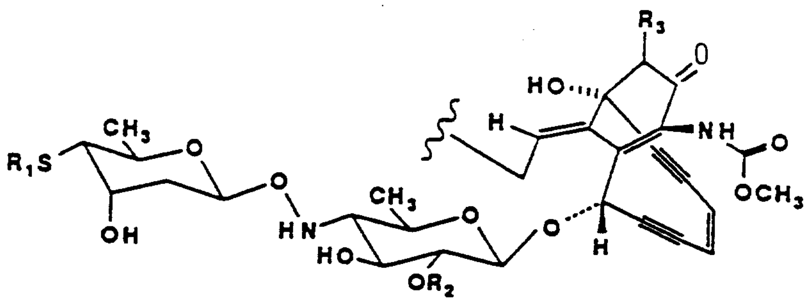

$R_1$ is 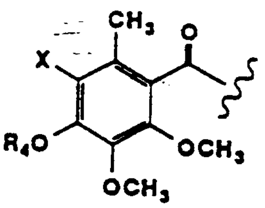 or $CH_3$;

$R_2$ is 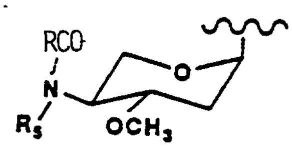 ;

$R_3$ is 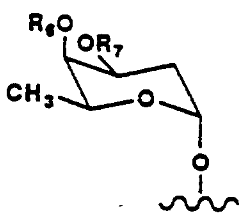 or H;

$R_4$ is 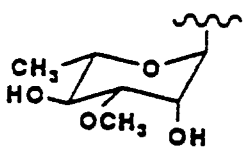 or H;

$R_6$ or $R_7$ is H or 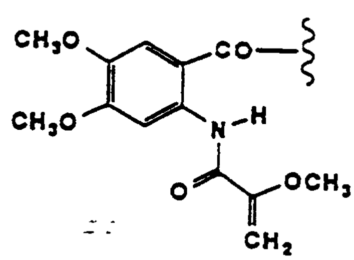

$R_5$ is $CH_3$, $C_2H_5$, or $(CH_3)_2CH$;
X is an iodine or bromine atom;
wherein R is hydrogen or a branched or unbranched alkyl ($C_1$-$C_{10}$) or alkylene ($C_1$-$C_{10}$) group, an aryl or heteroaryl group, or an aryl-alkyl ($C_1$-$C_5$) or heteroaryl-alkyl ($C_1$-$C_5$) group, all optionally substituted by one or more hydroxy, amino, carboxy, halo, nitro, lower ($C_1$-$C_3$) alkoxy, or lower ($C_1$-$C_5$) thioalkoxy groups; Sp is straight or branched-chain divalent or trivalent ($C_1$-$C_{18}$) radicals, divalent or trivalent aryl or heteroaryl radicals, divalent or trivalent ($C_3$-$C_{18}$) cycloalkyl or heterocyclo-alkyl radicals, divalent or trivalent aryl- or heteroaryl-

alkyl ($C_1$-$C_{18}$) radicals, divalent or trivalent cycloalkyl- or heterocycloalkyl-alkyl ($C_1$-$C_{18}$) radicals, or divalent or trivalent ($C_2$-$C_{18}$) unsaturated alkyl radicals, wherein, if Sp is a trivalent radical, it can be additionally substituted by amino, alkylamino, arylamino, heteroarylamino, carboxyl, lower alkoxy, hydroxy, thiol, or lover alkylthio groups; Q is hydrogen, halogen, amino, alkylamino, dialkylamino, arylamino, heteroarylamino, piperidino, pyrrolidino, piperazino, carboxyl, nitrophenyloxycarbonyl, carboxaldehyde, lower alkoxy, hydroxy, thiol, lower alkyldicarboxyl, $-CONHNH_2$, $-NHCONHNH_2$, $-CSNHNH_2$, $-NHCSNHNH_2$, or $-ONH_2$; with the provisos that when Sp is ethylidene, Q cannot be hydrogen; and when $CH_3$-SSS-W is LL-E33288 , $\alpha_1$-I, $\alpha_2$-Br, $\alpha_2$-I, $\beta_1$-Br, $\beta_1$-I, $\gamma_1$-Br, $\gamma_1$-I, $\delta_1$-I, BBM-1675, FR-900405, FR-900406, PD114759, PR115028, CL-1577A, CL-1577B, CL-1577D, CL-1577E, or CL-1724 and Sp is divalent, Q can not be hydrogen, halogen, amino, alkylamino, dialkylamino, arylamino, heteroarylamino, piperidino, pyrrolidino, piperazino, or carboxyl.

**9.** A product containing a compound

$$\begin{array}{l} Tu\text{-}(Z\text{-}Sp\text{-}SS\text{-}W)_m \\ | \\ (Y)_{n-m} \end{array}$$

prepared from a compound of general formula $CH_3SSS$-W, wherein $CH_3SSS$-W is an N-acyl derivative of an antitumor antibiotic designated as LL-E33288, $\alpha_1^I$, $\alpha_2^{BR}$, $\alpha_2^I$, $\beta_1^{BR}$, $\beta_1^I$, $\gamma_1^{Br}$, $\gamma_1^I$, $\delta_1^I$, BBM-1675, FR-900405, FR-900406, PD 114759, PD 115028, CL-1577A, CL-1577B, CL-1577D, CL-1577E, CL-1724, by a process comprising:

reacting $CH_3SSS$-W with a compound of general formula Q-Sp-SH, wherein Sp is a straight or branched-chain divalent or trivalent ($C_1$-$C_{18}$) radical, divalent or trivalent aryl or heteroaryl radical, divalent or trivalent ($C_3$-$C_{18}$) cycloalkyl or heterocycloalkyl radical, divalent or trivalent aryl- or heteroaryl-alkyl ($C_1$-$C_{18}$) radical divalent or trivalent cycloalkyl- or heterocycloalkyl-alkyl ($C_1$-$C_{18}$) radical or divalent or trivalent ($C_2$-$C_{18}$) unsaturated alkyl radical, wherein if Sp is a trivalent radical, it can be additionally substituted by amino, alkylamino, arylamino, heteroarylamino, carboxyl, lower alkoxy, hydroxy, thiol, a lower alkylthio groups, with the proviso that when $CH_3$-SSS-W is the parent antitumor antibiotic LL-E33288 $\alpha_1$-I, $\alpha_2$-Br, $\alpha_2$-I, $\beta_1$-Br, $\beta_1$-I, $\gamma_1$-Br, $\gamma_1$-I, $\delta_1$-I, BBM-1675, FR-900405, FR-900406, PD114759, PR115028, CL-1577A, CL-1577B, CL-1577D, CL-1577E, or CL-1724 itself, Sp can not be divalent; and Q is, or can be subsequently converted to, halogen, amino, alkylamino, carboxyl, carboxaldehyde, hydroxy, thiol, $\alpha$-haloacetyloxy, lower alkyldicarboxyl, $-CONHNH_2$, $-NHCONHNH_2$, $-NHCSNHNH_2$, $-ONH_2$, $-CON_3$,

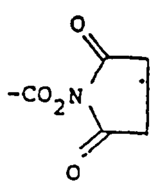 ; 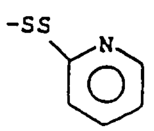 , 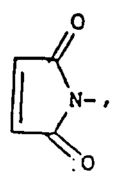 , 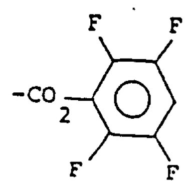 ,

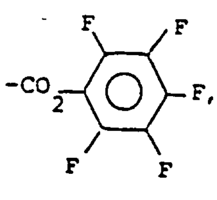 , 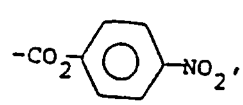 , 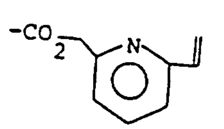 , 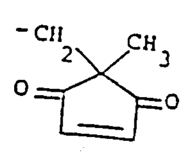

to produce an intermediate of formula Q-Sp-SS-W, wherein Q, Sp, and W are as hereinbefore defined, reacting Q-Sp-SS-W with a molecule of the formula Tu-$(Y)_n$ wherein Tu is defined as a mono- or polyclonal antibody, its fragments, its chemically or genetically manipulated counterparts, growth factors, or steroids; Y is a

side-chain amino, carboxy, or thiol group of a protein, an aldehyde derived from glycoprotein carbohydrate residues, or an amidoalkylthio group; and n is an integer of from 1 to 100, to produce a compound of the formula:

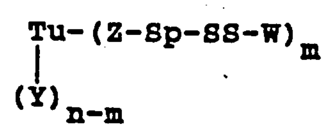

wherein Tu, Y, Sp, W, and n are as hereinbefore defined, and Z is formed from covalent reaction of the groups Q and Y directly or after subsequent reduction, and Z is -CONH-, -CONHN=CH-, -CONHNHCH$_2$-, -NHCONHN=CH-, -NHCONHNHCH$_2$-, -NHCSNHN=CH-, -NHCSNHNHCH$_2$-, -ON=CH-, -NH-, -NHCH$_2$-, -N=CH-, -CO$_2$-, -NHCH$_2$CO$_2$-, -SS-,

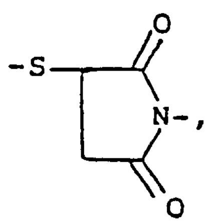

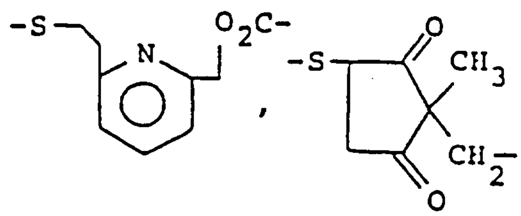

,

or

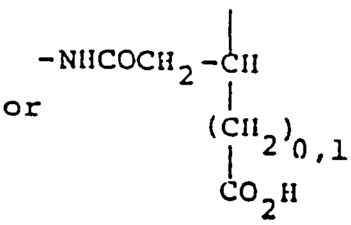

and m is 0.1 to 15, for use in tumor therapy.

**10.** A product of a protein-drug conjugate of the formula

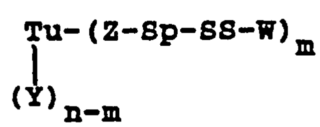

prepared from the antitumor antibiotic designated N-acetyl LL-E33288$\gamma_1^I$ ($CH_3SSS$-W) having

a) ultraviolet spectrum as shown in Figure 1;
b) an infrared spectrum as shown in Figure 2;
c) a proton magnetic resonance spectrum as shown in Figure 3; and
d) a carbon-13 nuclear magnetic resonance spectrum as shown in Figure 4;

by a process comprising:

displacing the dithiomethyl moiety with a compound of formula Q-Sp-SH, wherein Sp is straight or branched-chain divalent or trivalent ($C_2$-$C_5$) radicals or divalent or trivalent aryl- or heteroaryl-alkyl ($C_2$-$C_5$) radicals, wherein if Sp is a trivalent radical, it can be additionally substituted by amino, heteroarylamino, hydroxy, or thiol groups; and Q is, or can be subsequently converted to, carboxyl, lower alkyldicarboxylanhydride, -CONHNH$_2$, or

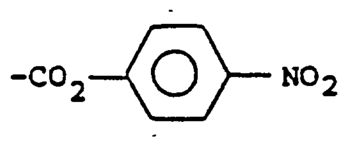

to produce an intermediate of general formula Q-Sp-SS-W, wherein Q, Sp, and W are as hereinbefore defined, reacting Q-Sp-SS-W with a molecule of the formula Tu-$(Y)_n$ wherein Tu is a monoclonal antibody which exhibits preferential reactivity with a human tumor-associated antigen, Y is a side-chain amino group on the antibody, or an aldehyde generated by oxidation of the carbohydrate groups of the antibody, and n is an integer of from 1 to 100, to produce a compound of the formula:

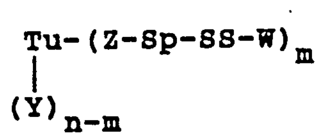

wherein Tu, Y, Sp, W, and n are as hereinbefore defined, and Z is formed from covalent reaction of the groups Q and Y directly or after subsequent reduction, and Z is -CONH-, -CONHN=CH-, -$CONHNHCH_2$-, or

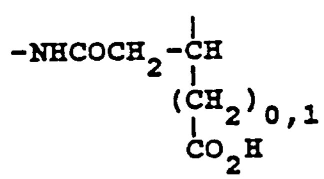

and m is 0.1 to 15, for use in tumor therapy for in vivo delivery of the conjugate to an antigenic site.

**Claims for the following Contracting States : ES, GR**

1. A process for producing a substituted disulfide of formula Q-Sp-SS-W from a compound of formula $CH_3SSS$-W which is an N-acyl derivative of an antitumor antibiotic designated as LL-E33288, $\alpha_1$-I, $\alpha_2$-Br, $\alpha_2$-I, $\beta_1$-Br, $\beta_1$-I, $\gamma_1$-Br, $\gamma_1$-I, $\delta_1$-I, BBM-1675, FR-900405, FR-900406, PD114759, PD115028, CL-1577A, CL-1577B, CL-1577D, CL-1577E, CL-1724, wherein

W is 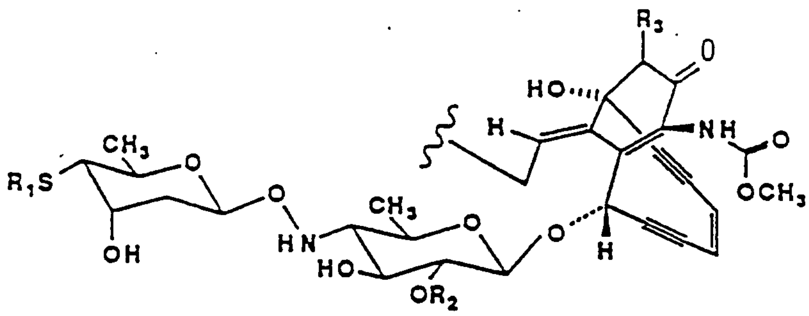

$R_1$ is 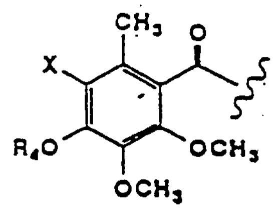 or $CH_3$;

$R_2$ is 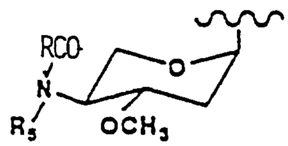

$R_3$ is 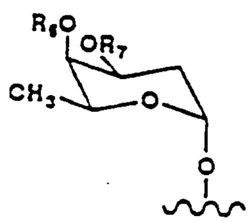 or H;

$R_4$ is 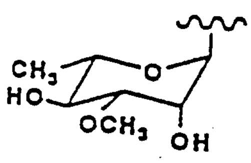 or H;

$R_6$ or $R_7$ is H or 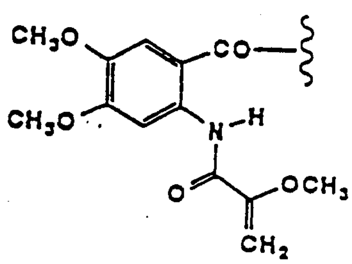

$R_5$ is $CH_3$, $C_2H_5$, or $(CH_3)_2CH$;

X is an iodine or bromine atom;

R is hydrogen or a branched or unbranched alkyl ($C_1$-$C_{10}$) or alkylene ($C_1$-$C_{10}$) group, an aryl or heteroaryl group, or an aryl-alkyl ($C_1$-$C_5$) or heteroaryl-alkyl ($C_1$-$C_5$) group, all optionally substituted by one or more hydroxy, amino, carboxy, halo, nitro, lower ($C_1$-$C_3$) alkoxy, or lower ($C_1$-$C_5$) thioalkoxy groups; Sp is straight or branched-chain divalent or trivalent ($C_1$-$C_{18}$) radicals, divalent or trivalent aryl or heteroaryl radicals, divalent or trivalent ($C_3$-$C_{18}$) cycloalkyl or heterocyclo-alkyl radicals, divalent or trivalent aryl- or heteroaryl-alkyl ($C_1$-$C_{18}$) radicals, divalent or trivalent cycloalkyl- or heterocycloalkyl-alkyl ($C_1$-$C_{18}$) radicals, or divalent or trivalent ($C_2$-$C_{18}$) unsaturated alkyl radicals, wherein if Sp is a trivalent radical, it can be additionally substituted by amino, alkylamino, arylamino, heteroarylamino, carboxyl, lower alkoxy, hydroxy, thiol, or lower alkylthio groups;

Q is hydrogen, halogen, amino, alkylamino, dialkylamino, arylamino, heteroarylamino, piperidino, pyrrolidino, piperazino, carboxyl, nitrophenyloxylcarbonyl, carboxaldehyde, lower alkoxy, hydroxy, thiol, lower alkyldicarboxyl, $-CONHNH_2$, $-NHCONHNH_2$, $-CSNHNH_2$, $-NHCSNHNH_2$, or $-ONH_2$; with the provisos that when Sp is ethylidene, Q cannot be hydrogen; and when $CH_3$-SSS-W is LL-E33288 , $\alpha_1$-I, $\alpha_2$-Br, $\alpha_2$-I, $\beta_1$-Br, $\beta_1$-I, $\gamma_1$-Br, $\gamma_1$-I, $\delta_1$-I, BBM-1675, FR-900405, FR-900406, PD114759, PR115028, CL-1577A, CL-1577B, CL-1577D, CL-1577E, or CL-1724 and Sp is divalent, Q can not be hydrogen, halogen, amino, alkylamino, dialkylamino, arylamino, heteroarylamino, piperidino, pyrrolidino, piperazino, or carboxyl;

which comprises reacting a methyl trisulfide antitumor antibiotic from those of the above with an appropriately substituted mercaptan in acetonitrile or a combination of acetonitrile and tetrahydrofuran or acetonitrile and dimethylformamide, at a temperature of between -20° to about +40°C, for a period of from one hour to six days, preferably in the presence of one equivalent of a tertiary amine base or a mixture of one equivalent of a tertiary amine base and one equivalent of an organic carboxylic acid.

2. The process according to Claim 1 wherein a substituted disulfide of formula Q-Sp-SS-W is prepared from a compound of formula $CH_3SSS$-W wherein:

W is 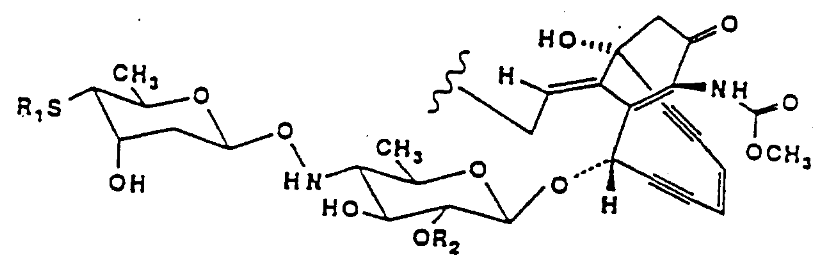

$R_1$ is 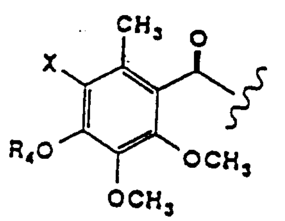 ;

$R_2$ is 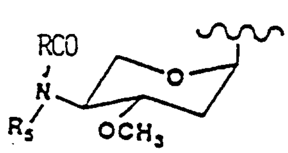 ;

$R_4$ is 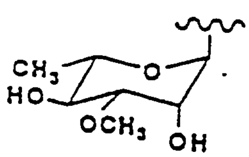 or H;

$R_5$ is $CH_3$, $C_2H_5$, or $(CH_3)_2CH$; X is an iodine or bromine atom;
wherein R is hydrogen, $CH_3$, or a mono-substituted aryl group; and Sp and Q are as defined.

**3.** A process for preparing the targeted derivatives

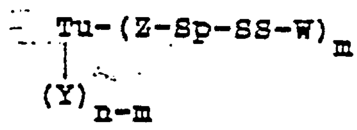

of compounds of formula $CH_3SSS$-W wherein $CH_3SSS$-W is an N-acyl derivative of an antitumor antibiotic LL-E33288, $\alpha_1^{I}$, $\alpha_2^{Br}$, $\alpha_2^{I}$, $\beta_1^{Br}$, $\beta_1^{I}$, $\gamma_1^{Br}$, $\gamma_1^{I}$, $\delta_1^{I}$, BBM-1675, FR-900405, FR-900406, PD 114759, PD 115028, CL-1577A, CL-1577B, CL-1577D, CL-1577E, CL-1724, comprising

reacting $CH_3SSS$-W with a compound of formula Q-Sp-SH, wherein Sp is a straight or branched-chain divalent or trivalent ($C_1$-$C_{18}$) radical, divalent or trivalent aryl or heteroaryl radical, divalent or trivalent ($C_3$-$C_{18}$) cycloalkyl or heterocycloalkyl radical, divalent or trivalent aryl- or heteroaryl-alkyl ($C_1$-$C_{18}$) radical, divalent or trivalent cycloalkyl- or heterocycloalkyl-alkyl ($C_1$-$C_{18}$) radical or divalent or trivalent ($C_2$-$C_{18}$) unsaturated alkyl radical, wherein if Sp is a trivalent radical, it can be additionally substituted by amino, alkylamino, arylamino, heteroarylamino, carboxyl, lower alkoxy, hydroxy, thiol, or lower alkylthio groups, with the proviso that when $CH_3$-SSS-W is the parent antitumor antibiotic LL-E33288, $\alpha$1-I, $\alpha_2$-Br, $\alpha_2$-I, $\beta_1$-Br, $\beta_1$-I, $\gamma_1$-Br, $\gamma_1$-I, $\delta_1$-I, BBM-1675, FR-900405, FR-900406, PD114759, PR115028, CL-1577A, CL-1577B, CL-1577D, CL-1577E, or CL-1724 itself, Sp can not be divalent; and Q is halogen, amino, alkylamino, carboxyl, carboxaldehyde, hydroxy, lower alkyldicarboxyl anhydride, -$CONHNH_2$, -$NHCONHNH_2$, -$NHCSNHNH_2$, -$ONH_2$, or

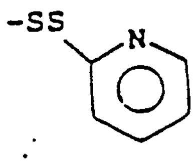

in acetonitrile in the presence of one equivalent of triethylamine or one equivalent of triethylamine and one equivalent of acetic acid at -10° to -30°C for 1-48 hours,
isolating the intermediate of formula Q-Sp-SS-W, wherein Q, Sp, and W are as hereinbefore defined in claim 1, then
reacting the compound of formula Q-Sp-SS-W, wherein Sp and W are as hereinbefore defined and Q is halogen, amino, alkylamino, carboxyl, carboxaldehyde, hydroxy, or lower alkyldicarboxylic anhydride with a molecule of the formula Tu-$(Y)_n$ wherein Tu is a mono- or polyclonal antibody, its fragments, its chemically or genetically manipulated counterparts, or growth factors or steroids; Y is a side-chain amino or carboxy functionality; n is 1-100, in aqueous buffer at a pH of between 6.5 and 9, at 4° to 40°C either directly or in the presence of a water-soluble carbodiimide, to generate the compound

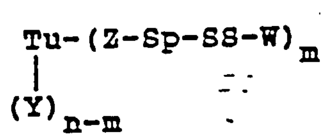

wherein Tu, Sp, W, n, and Y are as hereinbefore defined; m is 1-15 and Z is formed from covalent reaction of the groups Q and Y and is -CONH-, -NH-,

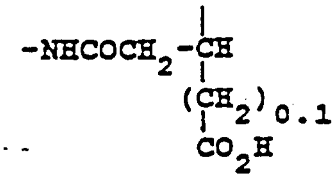

-N=CH-, or $-CO_2-$

or

reacting the compound of formula Q-Sp-SS-W, wherein Sp and W are as hereinbefore defined and Q is a carboxylic acid, with N-hydroxysuccinimide, 2,3,5,6-tetrafluorophenol, pentafluorophenol, or 4-nitrophenol in the presence of a carboxyl activating agent such as a carbodiimide to generate a compound of formula Q-Sp-SS-W wherein Sp and W are as hereinbefore defined and Q is

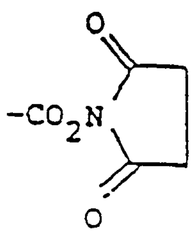 , 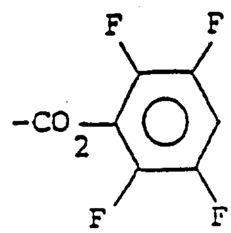 , 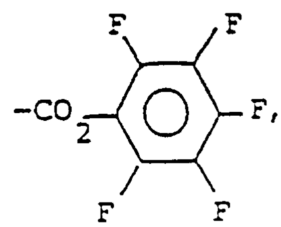

or 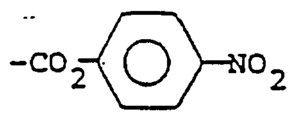

with a molecule of formula $Tu-(Y)_n$,

where Tu and n are as hereinbefore defined, and Y is a side-chain amino group, in an aqueous buffered solution at a pH between 6.5 and 9, at a temperature of between 4° and 40°C, inclusive, to generate compounds of the formula:

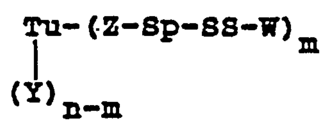

wherein Tu, Sp, Y, and n are as hereinbefore defined, m is 1-15, and Z is formed from covalent reaction between Q and Y and is defined as -CONH-

or

reacting a compound of formula Q-Sp-SS-W, wherein Sp and W are as hereinbefore defined and Q is $-CONHNH_2$ with nitrous acid in aqueous acetonitrile to generate a compound of formula Q-Sp-SS-W, wherein Sp and W are as hereinbefore defined and Q is $-CON_3$ with a compound of formula $Tu-(Y)_n$, wherein Tu, Y, and n are as hereinabove defined to produce a compound of the formula

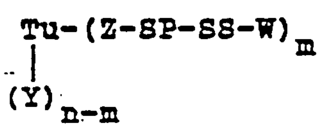

wherein Tu, Z, Sp, W, m, Y, and n are as hereinabove defined;

or

reacting a compound of formula Q-Sp-SS-W wherein Sp and W are as hereinbefore defined and Q is hydroxy, with an alpha-haloacetic anhydride to produce a compound wherein Q is α-haloacetyloxy, and reacting the α-haloacetyloxy-Sp-SS-W, or a compound of formula Q-Sp-SS-W, wherein Sp and W are as hereinbefore defined and Q is

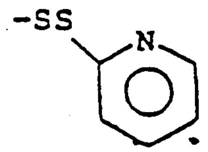 , 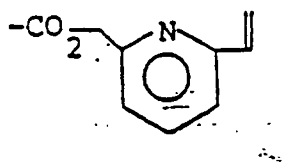 , or 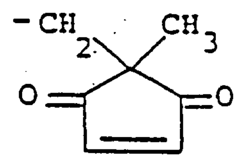

with a molecule of the formula Tu-$(Y)_n$ wherein Tu is as hereinbefore defined, Y is a side-chain thiol of a protein, or an amidoalkylthio group introduced on an amine of Tu using reagents such as 3-(2-dithiopyridyl)propionic acid hydroxysuccinimide ester followed by reduction with an agent such as dithiothreitol, or an amidoalkylthio group introduced on an amine of Tu using 2-iminothiolane, and n is 1-10, under aqueous buffered conditions at a pH between 4.5 and 7, at a temperature between 4° and 40°C, inclusive, to produce a compound of formula:

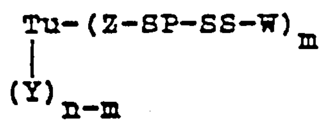

wherein Tu, Sp, W, and n are as hereinbefore defined, and Z is formed from covalent reaction of the groups Q and Y and Z is
-SS-,

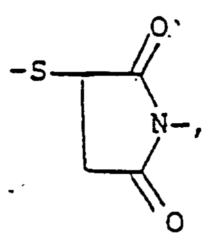 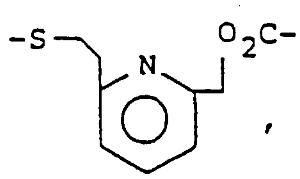 , or 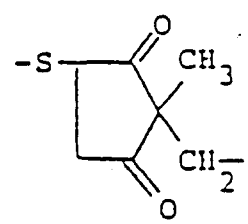

and n is 0.1 to 10;

or

reacting a compound of the formula Q-Sp-SS-W wherein Sp and W are as hereinbefore defined and Q is $-NH_2$, $-CONHNH_2$, $-NHCONHNH_2$, $-NHCSNHNH_2$, or $-ONH_2$ with a molecule of formula Tu-$(Y)_n$ wherein Tu is as hereinbefore defined, Y is an aldehyde generated from carbohydrate residues on Tu by oxidation in the presence of an alkaline earth periodate, in an aqueous buffer at a pH between 4.0 and 6.5, at 4° to 40°C, inclusive, and n is 1 to 20 to generate a compound of formula:

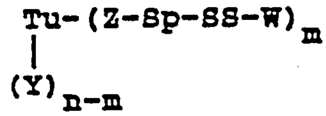

wherein Tu, Sp, W, Y, and n are as hereinbefore defined and Z is formed from the covalent reaction of Q and Y and is -ON=CH-, -N=CH-, -CONHN=CH-, -NHCONHN=CH-, or -NHCSNHN=CH-, and m is 0.1 to 15; or treating the compound immediately hereinabove of formula:

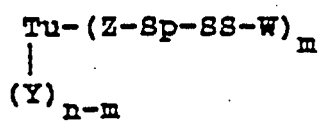

wherein Tu, Z, Sp, W, Y, n, and m are as immediately hereinabove defined with acetylhydrazine or tyrosine hydrazine in an aqueous buffer at a pH between 4.0 and 6.5, at 4° to 40°C, inclusive, to generate a compound of formula:

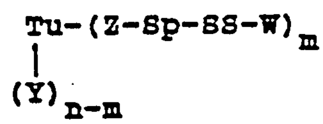

wherein Tu, Z, Sp, W, n, and m are as immediately hereinabove defined and Y is $-CH{=}NNHCOCH_3$ or

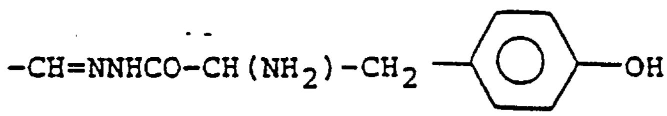

and

reacting this compound with sodium cyanoborohydride or sodium borohydride, in an aqueous buffer at a pH of 4.0 to 6.5, at a temperature of 4° to 40°C, inclusive, to generate a compound of formula:

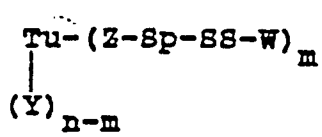

wherein Tu, Sp, W, m, and n are as hereinabove defined, Z is $-NH-CH_2-$, $-CONHNHCH_2-$, $-NHCONHNHCH_2-$, or $-NHCSNHNHCH_2-$, and Y is $-CH_2NHNHCOCH_3$ or

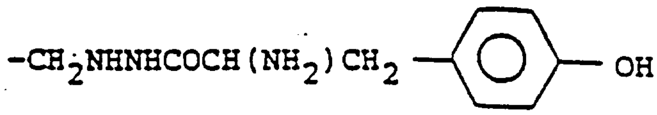

**4.** A process for preparing a carrier-drug conjugate of the formula

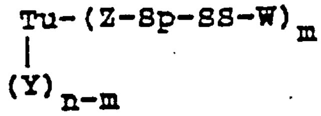

prepared from a compound of formula $CH_3SSS$-W wherein $CH_3SSS$-W is an N-acyl derivative of an antitumor antibiotic designated as LL-E33288, $\alpha_1{}^{I}$, $\alpha_2{}^{Br}$, $\alpha_2{}^{I}$, $\beta_1{}^{Br}$, $\beta_1{}^{I}$, $\gamma_1{}^{Br}$, $\gamma_1{}^{I}$, $\delta_1{}^{I}$, BBM-1675, FR-900405, FR-900406, PD114759, PD115028, CL-1577A, CL-1577B, CL-1577D, CL-1577E, CL-1724, wherein W is as hereinbefore described in claim 1 comprising:

reacting $CH_3SSS$-W with a compound of general formula Q-Sp-SH, wherein Sp is a straight or branched-chain divalent or trivalent ($C_1$-$C_{18}$) radical, divalent or trivalent aryl or heteroaryl radical, divalent or trivalent ($C_3$-$C_{18}$) cycloalkyl or heterocycloalkyl radical, divalent or trivalent aryl- or heteroaryl-alkyl ($C_1$-$C_{18}$) radical, divalent or trivalent cycloalkyl- or heterocycloalkyl-alkyl ($C_1$-$C_{18}$) radical or divalent or trivalent ($C_2$-$C_{18}$) unsaturated alkyl radical, wherein if Sp is a trivalent radical, it can be additionally substituted by amino, alkylamino, arylamino heteroarylamino, carboxyl, lower alkoxy, hydroxy, thiol, or lower alkylthio groups, with the proviso that when $CH_3$-SSS-W is the parent antitumor antibiotic LL-E33288$\alpha_1$-Br, $\alpha_1$-I, $\alpha_2$-Br, $\alpha_2$-I, $\beta_1$-Br, $\beta_1$-I, $\gamma_1$-Br, $\gamma_1$-I, $\delta_1$-I, BBM-1675, FR-900405, FR-900406, PD114759, PR115028, CL-1577A, CL-1577B, CL-1577D, CL-1577E, or CL-1724 itself, Sp can not be divalent; and Q is, or can be subsequently converted to, halogen, amino, alkylamino, carboxyl, carboxaldehyde, hydroxy, thiol, $\alpha$-haloacetyloxy, lower alkyldicarboxyl, $-CONHNH_2$, $-NHCONHNH_2$, $-NHCSNHNH_2$, $-ONH_2$, $-CON_3$,

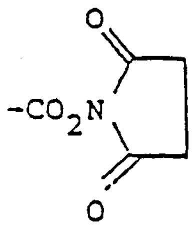, 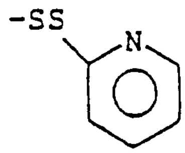, 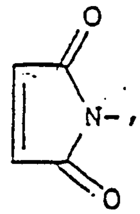, 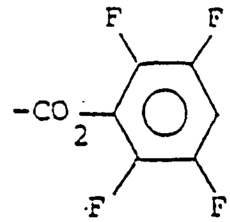,

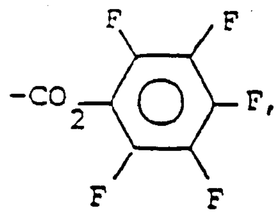, 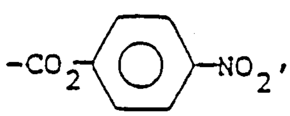, 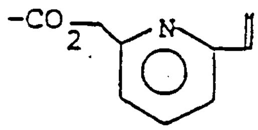, 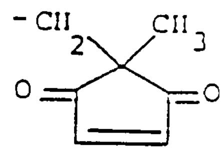

to produce an intermediate of formula Q-Sp-SS-W, wherein Q, Sp, and W are as hereinbefore defined, reacting Q-Sp-SS-W with a molecule of the formula TU-$(Y)_n$ wherein the carrier Tu is defined as a mono- or polyclonal antibody, its fragments, its chemically or genetically manipulated counterparts, or growth factors or steroids; Y is a side-chain amino, carboxy, or thiol group of a protein, an aldehyde derived from glycoprotein carbohydrate residues, or an amidoalkylthio group; and n is an integer of from 1 to 100, to produce a compound of the formula:

$$\underset{(Y)_{n-m}}{\overset{|}{Tu}}-(Z-Sp-SS-W)_m$$

wherein Tu, Y, Sp, W, and n are as hereinbefore defined, and Z is formed from covalent reaction of the groups Q and Y directly or after subsequent reduction, and Z is -CONH-, -CONHN=CH-, -CONHNHCH$_2$-, -NHCONHN=CH-, -NHCONHNHCH$_2$-, -NHCSNHN=CH-, -NHCSNHNHCH$_2$-, -ON=CH-, -NH-, -NHCH$_2$-, -N=CH-, -CO$_2$-, -NHCH$_2$CO$_2$-, -SS-,

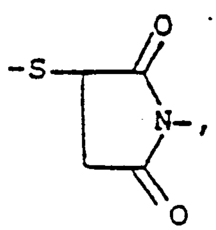 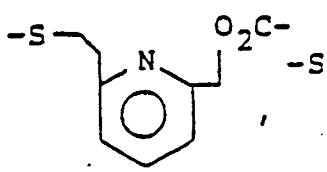, 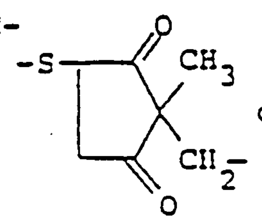 or 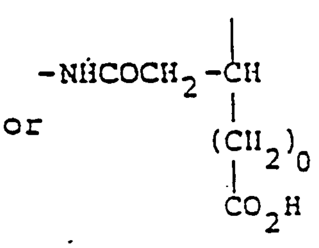

and m is 0.1 to 15.

5. A process for preparing a protein-drug conjugate as prepared in Claim 4 of the formula

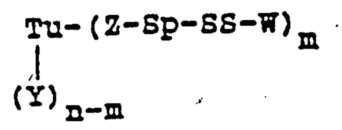

prepared from the class of N-acyl derivatives of antitumor antibiotics designated LL-E33288 ($CH_3SSS$-W) comprising:

displacing the dithiomethyl moiety with a compound of formula Q-Sp-SH, wherein Sp is straight or branched-chain divalent or trivalent ($C_2$-$C_{10}$) radicals or divalent or trivalent aryl- or heteroaryl-alkyl ($C_2$-$C_5$) radicals, wherein if Sp is a trivalent radical, it can be additionally substituted by amino, heteroarylamino, hydroxy, or thiol groups ; and Q is, or can be subsequently converted to, carboxyl , lower alkyldicarboxylanhydride, $-CO_2Su$, -$CONHNH_2$, or

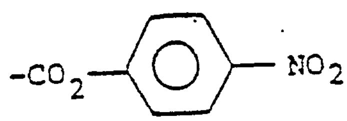

to produce an intermediate of general formula Q-Sp-SS-W, wherein Q, Sp, and W are as hereinbefore defined, reacting Q-Sp-SS-W with a molecule of the formula Tu-$(Y)_n$ wherein Tu is a monoclonal antibody which exhibits preferential reactivity with a human tumor-associated antigen, Y is a side-chain amino group on the antibody, or an aldehyde generated by oxidation of the carbohydrate groups of the antibody, and n is an integer of from 1 to 100, to produce a compound of the formula:

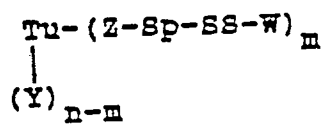

wherein Tu, Y, Sp, W, and n are as hereinbefore defined, and Z is formed from covalent reaction of the groups Q and Y directly or after subsequent reduction, and Z is -CONH-, -CONHN=CH-, $-CONHNHCH_2-$, or

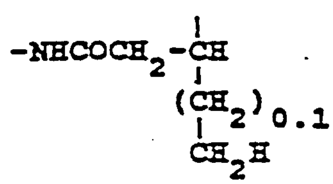

and m is 0.1 to 15.

**6.** A process for preparing a protein-drug conjugate of the formula

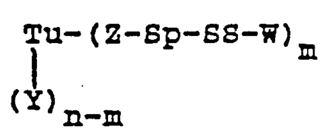

prepared from the antitumor antibiotic designated N-acetyl LL-E33288$\gamma_1{}^I$ ($CH_3SSS$-W) having

a) ultraviolet spectrum as shown in Figure 1;
b) an infrared spectrum as shown in Figure 2;
c) a proton magnetic resonance spectrum as shown in Figure 3; and
d) a carbon-13 nuclear magnetic resonance spectrum as shown in Figure 4;

which process comprises

displacing the dithiomethyl moiety with a compound of formula Q-Sp-SH, wherein Sp is straight or branched-chain divalent or trivalent ($C_2$-$C_5$) radicals or divalent or trivalent aryl- or heteroaryl-alkyl ($C_2$-$C_5$) radicals, wherein if Sp is a trivalent radical, it can be additionally substituted by amino, heteroarylamino, hydroxy, or thiol groups; and Q is, or can be subsequently converted to, carboxyl, lower alkyldicarboxylanhydride, $-CONHNH_2$, or

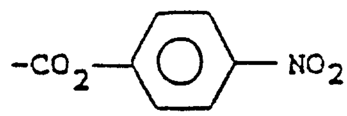

to produce an intermediate of general formula Q-Sp-SS-W, wherein Q, Sp, and W are as hereinbefore defined, reacting Q-Sp-SS-W with a molecule of the formula Tu-$(Y)_n$ wherein Tu is a monoclonal antibody which exhibits preferential reactivity with a human tumor-associated antigen, Y is a side-chain amino group on the antibody, or an aldehyde generated by oxidation of the carbohydrate groups of the antibody, and n is an integer of from 1 to 100, to produce a compound of the formula:

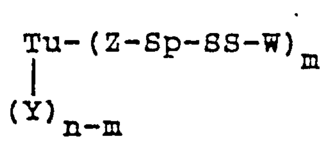

wherein Tu, Y, Sp, W, and n are as hereinbefore defined, and Z is formed from covalent reaction of the groups Q and Y directly or after subsequent reduction, and Z is -CONH-, -CONHN=CH-, $-CONHNHCH_2-$, or

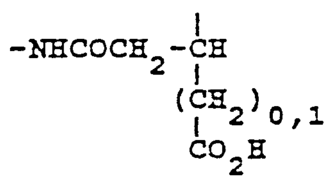

and m is 0.1 to 15.

## Patentansprüche

### Patentansprüche für folgende Vertragsstaaten : AT, BE, CH, DE, DK, FR, GB, IT, LI, NL, SE

1. Substituiertes Disulfid der Formel Q-Sp-SS-W, hergestellt aus einer Verbindung der Formel $CH_3SSS$-W, welche ein N-Acyl-Derivat eines Antitumor-Antibiotikums ist, das als LL-E33288, $\alpha_1$-I, $\alpha_2$-Br, $\alpha_2$-I, $\beta_1$-Br, $\beta_1$-I, $\gamma_1$-Br, $\gamma_1$-I, $\delta_1$-I, BBM-1675, FR-900405, FR-900406, PD114759, PD115028, CL-1577A, CL-1577B, CL-1577D, CL-1577E, CL-1724 bezeichnet wird, worin

W

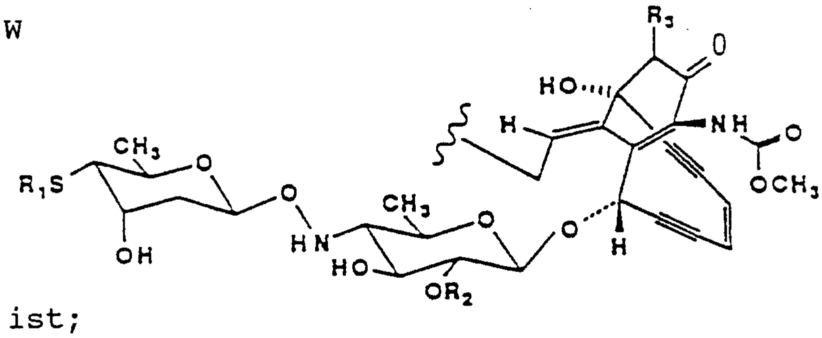

ist;

$R_1$

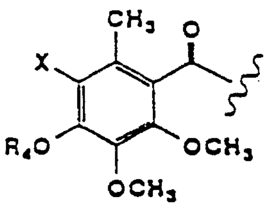

oder $CH_3$ ist;

$R_2$

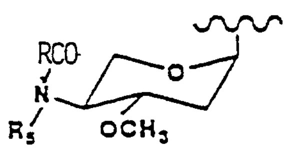

ist;

$R_3$

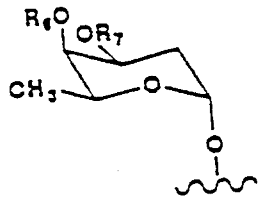

oder H ist;

$R_4$

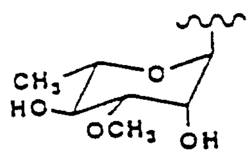

oder H ist;

$R_6$ oder $R_7$ für H oder

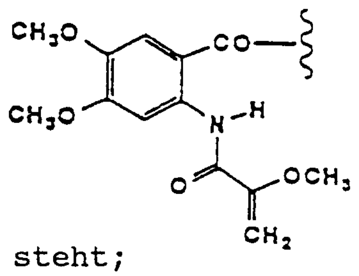

steht;

$R_5$ $CH_3$, $C_2H_5$ oder $(CH_3)_2CH$ ist;

X ein Iod- oder Bromatom ist;

R Wasserstoff oder eine verzweigte oder unverzweigte $(C_1-C_{10})$-Alkyl- oder $(C_1-C_{10})$-Alkylengruppe, eine Aryl- oder Heteroarylgruppe oder eine Aryl-$(C_1-C_5)$-alkyl- oder Heteroaryl-$(C_1-C_5)$-alkylgruppe ist, die alle gegebenenfalls mit einer oder mehreren Hydroxy-, Amino-, Carboxy-, Halogen-, Nitro-, $(C_1-C_3)$-Niederalkoxy- oder $(C_1-C_5)$-Niederthioalkoxygruppen substituiert sind;

Sp ein gerad- oder verzweigtkettiger zweiwertiger oder dreiwertiger $(C_1-C_{18})$-Rest, zweiwertiger oder dreiwertiger Aryl- oder Heteroarylrest, zweiwertiger oder dreiwertiger $(C_3-C_{18})$-Cycloalkyl- oder Heterocycloalkylrest, zweiwertiger oder dreiwertiger Aryl- oder Heteroaryl-$(C_1-C_{18})$-alkylrest, zweiwertiger oder dreiwertiger Cycloalkyl- oder Heterocycloalkyl-$(C_1-C_{18})$-alkylrest oder zweiwertiger oder dreiwertiger ungesättigter $(C_2-C_{18})$-Alkylrest ist, worin, falls Sp ein dreiwertiger Rest ist, es zusätzlich mit Amino-, Alkylamino-, Arylamino-, Heteroarylamino-, Carboxyl-, Niederalkoxy-, Hydroxy-, Thiol- oder Niederalkylthiogruppen substituiert sein kann;

Q Wasserstoff, Halogen, Amino, Alkylamino, Dialkylamino, Arylamino, Heteroarylamino, Piperidino, Pyrrolidino, Piperazino, Carboxyl, Nitrophenyloxycarbonyl, Carboxaldehyd, Niederalkoxy, Hydroxy, Thiol, Niederalkyldicarboxyl, $-CONHNH_2$, $-NHCONHNH_2$, $-CSNHNH_2$, $-NHCSNHNH_2$ oder $-ONH_2$ ist; mit den Maßgaben, daß, wenn Sp Ethyliden ist, Q nicht Wasserstoff sein kann, und wenn $CH_3$-SSS-W LL-E33288, $\alpha_1$-I, $\alpha_2$-Br, $\alpha_2$-I, $\beta_1$-Br, $\beta_1$-I, $\gamma_1$-Br, $\gamma_1$-I, $\delta_1$-I, BBM-1675, FR-900405, FR-900406, PD114759, PR115028, CL-1577A, CL-1577B, CL-1577D, CL-1577E oder CL-1724 ist und Sp zweiwertig ist, Q nicht Wasserstoff, Halogen, Amino, Alkylamino, Dialkylamino, Arylamino, Heteroarylamino, Piperidino, Pyrrolidino, Piperazino oder Carboxyl sein kann.

2. Substituiertes Disulfid der Formel Q-Sp-SS-W nach Anspruch 1, hergestellt aus einer Verbindung der Formel $CH_3SSS$-W, in der:

W

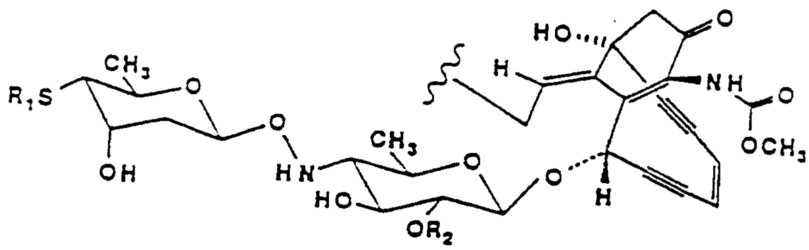

ist;

$R_1$

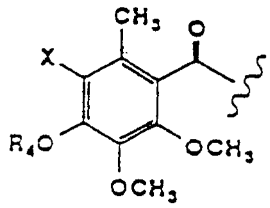

ist;

$R_2$

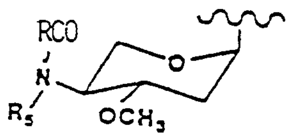

ist;

$R_4$

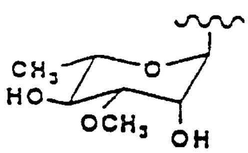

oder H ist;

$R_5$ $CH_3$, $C_2H_5$ oder $(CH_3)_2CH$ ist; X ein Iod- oder Bromatom ist; worin R Wasserstoff, $CH_3$ oder eine monosubstituierte Arylgruppe ist; und Sp und Q wie definiert sind.

**3.** Träger-Arzneistoff-Konjugat der Formel

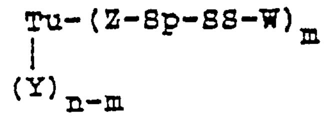

hergestellt aus einer Verbindung der Formel $CH_3SSS$-W, in der $CH_3SSS$-W ein N-Acyl-Derivat eines Antitumor-Antibiotikums ist, das als LL-E33288, $\alpha_1^I$, $\alpha_2^{Br}$, $\alpha_2^I$, $\beta_1^{Br}$, $\beta_1^I$, $\gamma_1^{Br}$, $\gamma_1^I$, $\delta_1^I$, BBM-1675, FR-900405, FR-900406, PD114759, PD115028, CL-1577A, CL-1577B, CL-1577D, CL-1577E, CL-1724 bezeichnet wird, worin W wie vorstehend in Anspruch 1 beschrieben ist, umfassend:

das Umsetzen von $CH_3SSS$-W mit einer Verbindung der allgemeinen Formel Q-Sp-SH, worin Sp ein gerad- oder verzweigtkettiger zweiwertiger oder dreiwertiger ($C_1$-$C_{18}$)-Rest, zweiwertiger oder dreiwertiger Aryl- oder Heteroarylrest, zweiwertiger oder dreiwertiger ($C_3$-$C_{18}$)-Cycloalkyl- oder Heterocycloalkylrest, zweiwertiger oder dreiwertiger Aryl- oder Heteroaryl-($C_1$-$C_{18}$)-alkylrest, zweiwertiger oder dreiwertiger Cycloalkyl- oder Heterocycloalkyl-($C_1$-$C_{18}$)-alkylrest oder zweiwertiger oder dreiwertiger ungesättigter ($C_2$-$C_{18}$)-Alkylrest ist, worin, falls Sp ein dreiwertiger Rest ist, es zusätzlich durch Amino-, Alkylamino-, Arylamino-, Heteroarylamino-, Carboxyl-, Niederalkoxy-, Hydroxy-, Thiol- oder Niederalkylthiogruppen substituiert sein kann, mit der Maßgabe, daß, wenn $CH_3SSS$-W in dem Ausgangs-Antitumor-Antibiotikum LL-E33288 $\alpha_1$-Br, $\alpha_1$-I, $\alpha_2$-Br, $\alpha_2$-I, $\beta_1$-Br, $\beta_1$-I, $\gamma_1$-Br, $\gamma_1$-I, $\delta_1$-I, BBM-1675, FR-900405, FR-900406, PD114759, PR115028, CL-1577A, CL-1577B, CL-1577D, CL-1577E oder CL-1724 selbst ist, Sp nicht zweiwertig sein kann; und Q Halogen, Amino, Alkylamino, Carboxyl, Carboxaldehyd, Hydroxy, Thiol, $\alpha$-Halogenacetyloxy, Niederalkyldicarboxyl, -$CONHNH_2$, -$NHCONHNH_2$, -$NHCSNHNH_2$, -$ONH_2$, -$CON_3$,

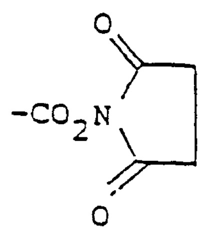 , 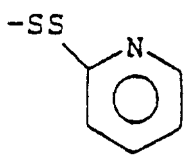 , 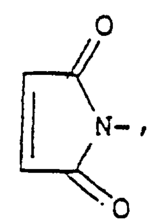 , 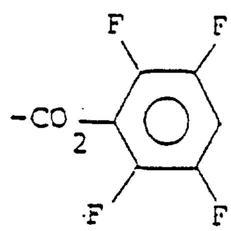 ,

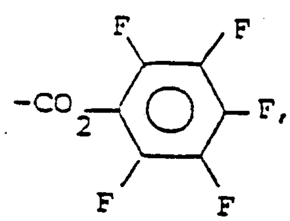 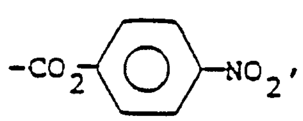 , 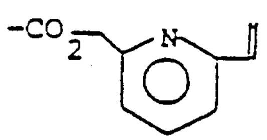 , 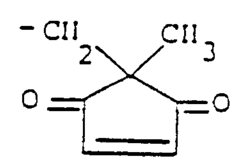

ist oder anschließend dazu umgewandelt werden kann, um ein Zwischenprodukt der Formel Q-Sp-SS-W herzustellen, in der Q, Sp und W wie vorstehend definiert sind,

das Umsetzen von Q-Sp-SS-W mit einem Molekül der Formel Tu-$(Y)_n$, worin der Träger Tu als ein mono- oder polyklonaler Antikörper, dessen Fragmente, dessen chemisch oder genetisch manipulierte Gegenstücke oder Wachstumsfaktoren oder Steroide definiert ist; Y eine Seitenketten-Amino-, -Carboxy- oder -Thiolgruppe eines Proteins, ein Aldehyd, der von Glycoproteinkohlehydrat-Resten abstammt, oder eine Amidoalkylthiogruppe ist; und n eine ganze Zahl von 1 bis 100 ist, um eine Verbindung der Formel:

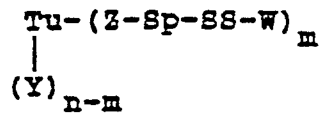

herzustellen, in der Tu, Y, Sp, W und n wie vorstehend definiert sind und Z aus einer kovalenten Reaktion der Gruppen Q und Y direkt oder nach anschließender Reduktion gebildet ist und Z -CONH-, -CONHN=CH-, -CONHNHCH$_2$-, -NHCONHN=CH-, -NHCONHNHCH$_2$-, -NHCSNHN=CH-, -NHCSNHNHCH$_2$-, -ON=CH-, -NH-, -NHCH$_2$-, -N=CH-, -CO$_2$-, -NHCH$_2$CO$_2$-, -SS-,

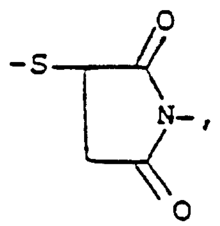 , 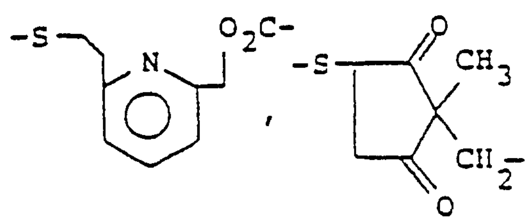 oder 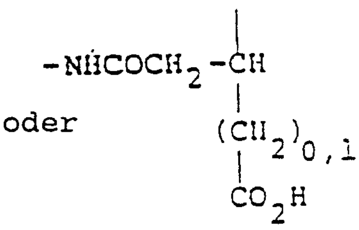

ist und m 0,1 bis 15 ist.

**4.** Protein-Arzneistoff-Konjugat nach Anspruch 3 der Formel

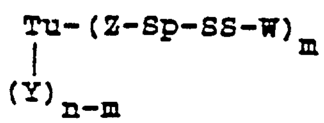

hergestellt aus der Klasse von N-Acyl-Derivaten von Antitumor-Antiobiotika, die als LL-E33288 (CH$_3$SSS-W) bezeichnet werden, umfassend:

das Verdrängen der Dithiomethyl-Einheit durch eine Verbindung der Formel Q-Sp-SH, in der Sp ein gerad- oder verzweigtkettiger zweiwertiger oder dreiwertiger (C$_2$-C$_{10}$)-Rest oder zweiwertiger oder dreiwertiger Aryl- oder Heteroaryl-(C$_2$-C$_5$)-alkylrest ist, worin, falls Sp ein dreiwertiger Rest ist, es zusätzlich durch Amino-, Heteroarylamino-, Hydroxy- oder Thiolgruppen substituiert sein kann; und Q Carboxyl, Niederalkyldicarboxylanhydrid, -CO$_2$Su, -CONHNH$_2$ oder

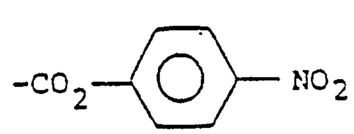

ist oder anschließend dazu umgewandelt werden kann, um ein Zwischenprodukt der allgemeinen Formel Q-Sp-SS-W herzustellen, in der Q, Sp und W wie vorstehend definiert sind,

das Umsetzen von Q-Sp-SS-W mit einem Molekül der Formel Tu-(Y)$_n$, in der Tu ein monoklonaler Antikörper ist, der eine bevorzugte Reaktivität mit einem menschlichen Tumor-assoziierten Antigen aufweist, Y eine Seitenketten-Aminogruppe am Antikörper oder ein Aldehyd ist, der durch Oxidation der Kohlehydratgruppen des Antikörpers erzeugt wird, und n eine ganze Zahl von 1 bis 100 ist, um eine Verbindung der Formel

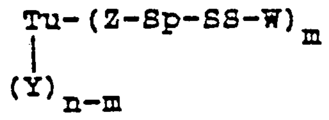

herzustellen, in der Tu, Y, Sp, W und n wie vorstehend definiert sind und Z aus einer kovalenten Reaktion der Gruppen Q und Y direkt oder nach anschließender Reduktion gebildet wird und Z -CONH-, -CONHN=CH-, -CONHNHCH$_2$- oder

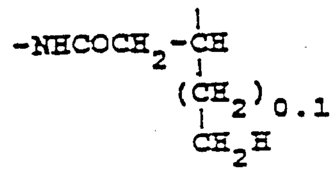

ist und m 0,1 bis 15 ist.

**5.** Verfahren zur Herstellung eines substituierten Disulfids der Formel Q-Sp-SS-W aus einer Verbindung der Formel $CH_3$SSS-W, welche ein N-Acyl-Derivat eines Antitumor-Antibiotikums ist, das als LL-E33288, $\alpha_1$-I, $\alpha_2$-Br, $\alpha_2$-I, $\beta_1$-Br, $\beta_1$-I, $\gamma_1$-Br, $\gamma_1$-I, $\delta_1$-I, BBM-1675, FR-900405, FR-900406, PD114759, PD115028, CL-1577A, CL-1577B, CL-1577D, CL-1577E, CL-1724 bezeichnet wird, worin

W

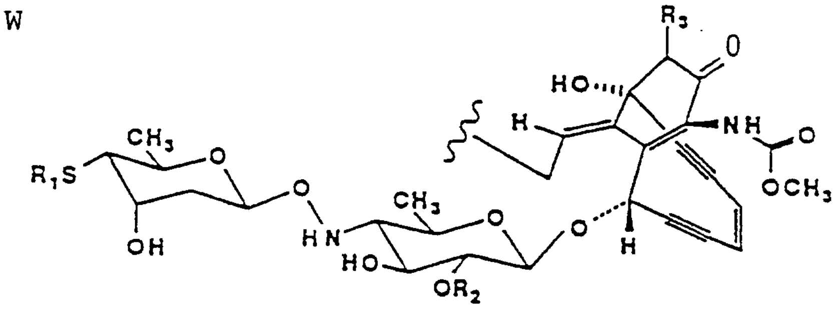

ist;

$R_1$

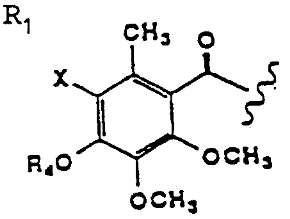

oder $CH_3$ ist;

$R_2$

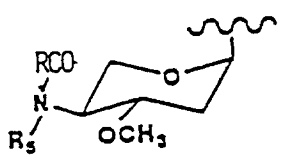

ist;

$R_3$

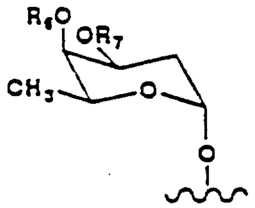

oder H ist;

$R_4$

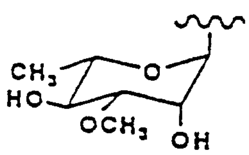

oder H ist;

$R_6$ oder $R_7$ für H oder

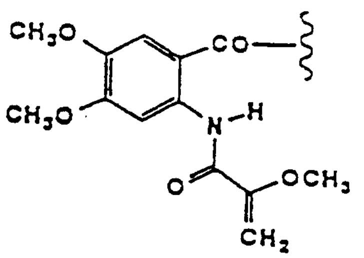

steht;

$R_5$ $CH_3$, $C_2H_5$ oder $(CH_3)_2CH$ ist;

X ein Iod- oder Bromatom ist;

R Wasserstoff oder eine verzweigte oder unverzweigte $(C_1$-$C_{10})$-Alkyl- oder $(C_1$-$C_{10})$-Alkylengruppe, eine Aryl- oder Heteroarylgruppe oder eine Aryl-$(C_1$-$C_5)$-alkyl- oder Heteroaryl-$(C_1$-$C_5)$-alkylgruppe ist, die alle gegebenenfalls mit einer oder mehreren Hydroxy-, Amino-, Carboxy-, Halogen-, Nitro-, $(C_1$-$C_3)$-Niederalkoxy- oder $(C_1$-$C_5)$-Niederthioalkoxygruppen substituiert sind;

Sp ein gerad- oder verzweigtkettiger zweiwertiger oder dreiwertiger $(C_1$-$C_{18})$-Rest, zweiwertiger oder dreiwertiger Aryl- oder Heteroarylrest, zweiwertiger oder dreiwertiger $(C_3$-$C_{18})$-Cycloalkyl- oder Heterocycloalkylrest, zweiwertiger oder dreiwertiger Aryl- oder Heteroaryl-$(C_1$-$C_{18})$-alkylrest, zweiwertiger oder dreiwertiger Cycloalkyl- oder Heterocycloalkyl-$(C_1$-$C_{18})$-alkylrest oder zweiwertiger oder dreiwertiger ungesättigter $(C_2$-$C_{18})$-Alkylrest ist, worin, falls Sp ein dreiwertiger Rest ist, es zusätzlich mit Amino-, Alkylamino-, Arylamino-, Heteroarylamino-, Carboxyl-, Niederalkoxy-, Hydroxy-, Thiol- oder Niederalkylthiogruppen substituiert sein kann;

Q Wasserstoff, Halogen, Amino, Alkylamino, Dialkylamino, Arylamino, Heteroarylamino, Piperidino, Pyrrolidino, Piperazino, Carboxyl, Nitrophenyloxycarbonyl, Carboxaldehyd, Niederalkoxy, Hydroxy, Thiol, Niederalkyldicarboxyl, $-CONHNH_2$, $-NHCONHNH_2$, $-CSNHNH_2$, $-NHCSNHNH_2$ oder $-ONH_2$ ist; mit den Maßgaben, daß, wenn Sp Ethyliden ist, Q nicht Wasserstoff sein kann; und wenn $CH_3$-SSS-W LL-E33288, $\alpha_1$-I, $\alpha_2$-Br, $\alpha_2$-I, $\beta_1$-Br, $\beta_1$-I, $\gamma_1$-Br, $\gamma_1$-I, $\delta_1$-I, BBM-1675, FR-900405, FR-900406, PD114759, PR115028, CL-1577A, CL-1577B, CL-1577D, CL-1577E oder CL-1724 ist und Sp zweiwertig ist, Q nicht Wasserstoff, Halogen, Amino, Alkylamino, Dialkylamino, Arylamino, Heteroarylamino, Piperidino, Pyrrolidino, Piperazino oder Carboxyl sein kann;

umfassend das Umsetzen eines Methyltrisulfid-Antitumor-Antibiotikums aus denjenigen der obigen mit einem geeignet substituierten Mercaptan in Acetonitril oder einer Kombination von Acetonitril und Tetrahydrofuran oder Acetonitril und Dimethylformamid bei einer Temperatur von zwischen -20 bis etwa +40°C über einen Zeitraum von einer Stunde bis sechs Tagen, vorzugsweise in Anwesenheit eines Äquivalents einer tertiären Amin-Base oder einer Mischung eines Äquivalents einer tertiären Amin-Base und eines Äquivalents einer organischen Carbonsäure.

**6.** Verfahren zur Herstellung der Ziel-Derivate

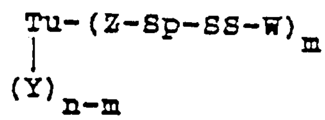

von Verbindungen der Formel $CH_3SSS$-W, in der $CH_3SSS$-W ein N-Acyl-Derivat eines Antitumor-Antibiotikums LL-E33288, $\alpha_1{}^I$, $\alpha_2{}^{Br}$, $\alpha_2{}^I$, $\beta_1{}^{Br}$, $\beta_1{}^I$, $\gamma_1{}^{Br}$, $\gamma_1{}^I$, $\delta_1{}^I$, BBM-1675, FR-900405, FR-900406, PD114759, PD115028, CL-1577A, CL-1577B, CL-1577D, CL-1577E, CL-1724 ist, umfassend

das Umsetzen von $CH_3SSS$-W mit einer Verbindung der Formel Q-Sp-SH, in der Sp ein gerad- oder verzweigtkettiger zweiwertiger oder dreiwertiger $(C_1$-$C_{18})$-Rest, zweiwertiger oder dreiwertiger Aryl- oder Heteroarylrest, zweiwertiger oder dreiwertiger $(C_3$-$C_{18})$-Cycloalkyl- oder Heterocycloalkylrest, zweiwertiger oder dreiwertiger Aryl- oder Heteroaryl-$(C_1$-$C_{18})$-alkylrest, zweiwertiger oder dreiwertiger Cycloalkyl- oder Heterocycloalkyl-$(C_1$-$C_{18})$-alkylrest oder zweiwertiger oder dreiwertiger ungesättigter $(C_2$-$C_{18})$-Alkylrest ist, worin, falls Sp ein dreiwertiger Rest ist, es zusätzlich mit Amino-, Alkylamino-, Arylamino-, Heteroarylamino-, Carboxyl-, Niederalkoxy-, Hydroxy-, Thiol- oder Niederalkylthiogruppen substituiert sein kann, mit der Maßgabe, daß, wenn $CH_3$-SSS-W das Ausgangs-Antitumor-Antibiotikum LL-E33288 , $\alpha_1$-I, $\alpha_2$-Br, $\alpha_2$-I, $\beta_1$-Br, $\beta_1$-I, $\gamma_1$-Br, $\gamma_1$-I, $\delta_1$-I, BBM-1675, FR-900405, FR-900406, PD114759, PR115028, CL-1577A, CL-1577B, CL-1577D, CL-1577E oder CL-1724 selbst ist, Sp nicht zweiwertig sein kann; und Q Halogen, Amino, Alkylamino, Carboxyl, Carboxaldehyd, Hydroxy, Niederalkyldicarboxylanhydrid, $-CONHNH_2$, $-NHCONHNH_2$, $-NHCSNHNH_2$, $-ONH_2$ oder

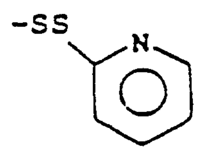

ist, in Acetonitril in Anwesenheit eines Äquivalents Triethylamin oder eines Äquivalents Triethylamin und eines Äquivalents Essigsäure bei -10 bis -30°C über 1 - 48 Stunden,

das Isolieren des Zwischenproduktes der Formel Q-Sp-SS-W, in der Q, Sp und W wie vorstehend definiert sind, dann

das Umsetzen der Verbindung der Formel Q-Sp-SS-W, in der Sp und W wie vorstehend definiert sind und Q Halogen, Amino, Alkylamino, Carboxyl, Carboxaldehyd, Hydroxy oder Niederalkyldicarbonsäureanhydrid ist, mit einem Molekül der Formel $Tu$-$(Y)_n$, worin es sich bei Tu um einen mono- oder polyklonalen Antikörper, dessen Fragmente, dessen chemisch oder genetisch manipulierte Gegenstücke oder Wachstumsfaktoren oder

Steroide handelt; Y eine Seitenketten-Amino- oder Carboxy-Funktionalität ist; n 1 - 100 ist, in wäßrigem Puffer bei einem pH zwischen 6,5 und 9 bei 4 bis 40°C entweder direkt oder in Anwesenheit eines wasserlöslichen Carbodiimids, um die Verbindung

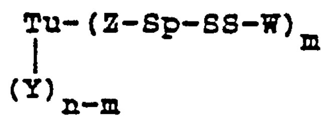

herzustellen, in der Tu, Sp, W, n und Y wie vorstehend definiert sind, m 1 - 15 ist und Z aus einer kovalenten Reaktion der Gruppen Q und Y gebildet ist und -CONH-, -NH-,

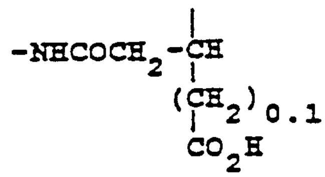

-N=CH- oder $-CO_2-$ ist,
oder

das Umsetzen der Verbindung der Formel Q-Sp-SS-W, in der Sp und W wie vorstehend definiert sind und Q eine Carbonsäure ist, mit N-Hydroxysuccinimid, 2,3,5,6-Tetrafluorphenol, Pentafluorphenol oder 4-Nitrophenol in Anwesenheit eines Carboxyl-aktivierenden Mittels, wie eines Carbodiimids, um eine Verbindung der Formel Q-Sp-SS-W herzustellen, in der Sp und W wie vorstehend definiert sind und Q

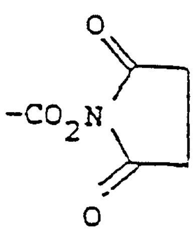 , 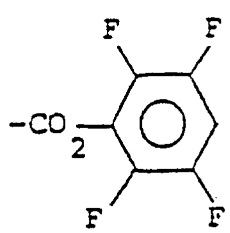 , 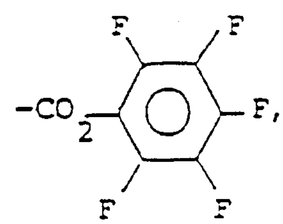

oder

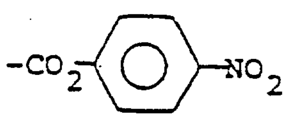

ist, dann mit einem Molekül der Formel $Tu-(Y)_n$, in der Tu und n wie vorstehend definiert sind und Y eine Seitenketten-Aminogruppe ist, in einer wäßrigen gepufferten Lösung bei einem pH zwischen 6,5 und 9 bei einer Temperatur zwischen 4 und 40°C einschließlich, um Verbindungen der Formel:

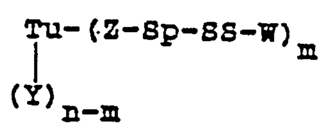

herzustellen, in der Tu, Sp, Y und n wie vorstehend definiert sind, m 1 - 15 ist und Z aus einer kovalenten Reaktion zwischen Q und Y gebildet ist und als -CONH- definiert ist
oder

das Umsetzen einer Verbindung der Formel Q-Sp-SS-W, in der Sp und W wie vorstehend definiert sind und Q -$CONHNH_2$ ist, mit salpetriger Säure in wäßrigem Acetonitril, um eine Verbindung der Formel Q-Sp-SS-W herzustellen, in der Sp und W wie vorstehend definiert sind und Q -$CON_3$ ist, dann mit einer Verbindung der Formel Tu-$(Y)_n$, in der Tu, Y und n wie vorstehend definiert sind, um eine Verbindung der Formel

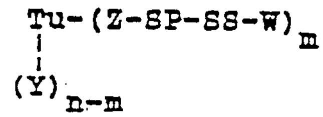

herzustellen, in der Tu, Z, Sp, W, m, Y und n wie vorstehend definiert sind;
oder

das Umsetzen einer Verbindung der Formel Q-Sp-SS-W, in der Sp und W wie vorstehend definiert sind und Q Hydroxy ist, mit einem alpha-Halogenacetanhydrid, um eine Verbindung herzustellen, in der Q $\alpha$-Halogenacetyloxy ist, und das Umsetzen des $\alpha$-Halogenacetyloxy-Sp-SS-W oder einer Verbindung der Formel Q-Sp-SS-W, in der Sp und W wie vorstehend definiert sind und Q

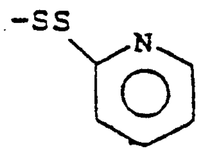 , 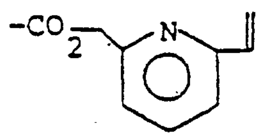 oder 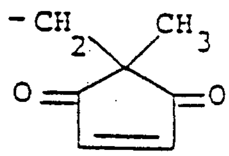

ist, mit einem Molekül der Formel Tu-$(Y)_n$, in der Tu wie vorstehend definiert ist, Y für ein Seitenketten-Thiol eines Proteins oder eine Amidoalkylthiogruppe, die an einem Amin von Tu unter Verwendung von Reagenzien wie 3-(2-Dithiopyridyl)propionsäurehydroxysuccinimidester, gefolgt von der Reduktion mit einem Mittel wie Dithiothreit, eingeführt wurde, oder eine Amidoalkylthiogruppe steht, die unter Verwendung von 2-Iminothiolan an einem Amin von Tu eingeführt wurde, und n 1 - 10 ist, unter wäßrigen gepufferten Bedingungen bei einem pH zwischen 4,5 und 7 bei einer Temperatur zwischen 4 und 40°C einschließlich, um eine Verbindung der Formel:

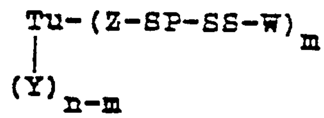

herzustellen, in der Tu, Sp, W und n wie vorstehend definiert sind und Z aus einer kovalenten Reaktion der Gruppen Q und Y gebildet ist und Z
-SS-,

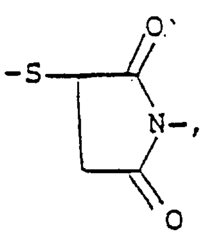 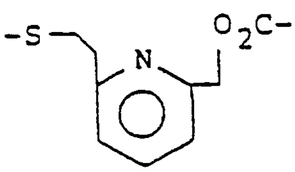 oder 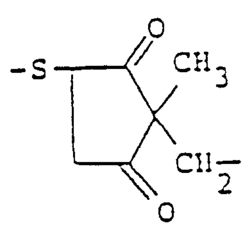

ist und n 0,1 bis 10 ist;
oder

das Umsetzen einer Verbindung der Formel Q-Sp-SS-W, in der Sp und W wie vorstehend definiert sind und Q $-NH_2$, $-CONHNH_2$, $-NHCONHNH_2$, $-NHCSNHNH_2$ oder $-ONH_2$ ist, mit einem Molekül der Formel $Tu-(Y)_n$, in der Tu wie vorstehend definiert ist, Y ein Aldehyd ist, der aus Kohlehydrat-Resten an Tu durch Oxidation in Anwesenheit eines Erdalkaliperiodats erzeugt worden ist, in einem wäßrigen Puffer bei einem pH zwischen 4,0 und 6,5 bei 4 bis 40°C einschließlich, und n 1 bis 20 ist, um eine Verbindung der Formel:

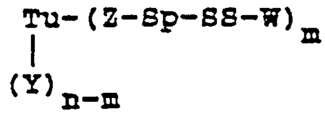

herzustellen, in der Tu, Sp, W, Y und n wie vorstehend definiert sind und Z aus der kovalenten Reaktion von Q und Y gebildet ist und -ON=CH-, -N=CH-, -CONHN=CH-, -NHCONHN=CH- oder -NHCSNHN=CH- ist und m 0,1 bis 15 ist; oder das Behandeln der unmittelbar vorstehenden Verbindung der Formel:

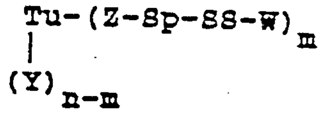

in der Tu, Z, Sp, W, Y, n und m wie unmittelbar vorstehend definiert sind, mit Acetylhydrazin oder Tyrosinhydrazin in einem wäßrigen Puffer bei einem pH zwischen 4,0 und 6,5 bei 4 bis 40°C einschließlich, um eine Verbindung der Formel:

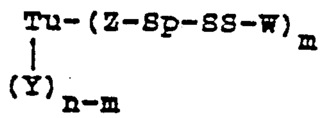

herzustellen, in der Tu, Z, Sp, W, n und m wie unmittelbar vorstehend definiert sind und Y $-CH=NNHCOCH_3$ oder

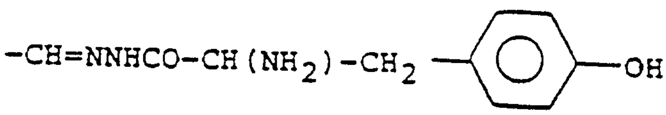

ist, und

das Umsetzen dieser Verbindung mit Natriumcyanborhydrid oder Natriumborhydrid in einem wäßrigen Puffer bei einem pH von 4,0 bis 6,5 bei einer Temperatur von 4 bis 40°C einschließlich, um eine Verbindung der Formel:

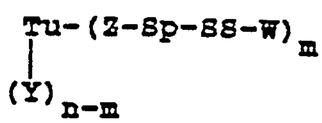

herzustellen, in der Tu, Sp, W, m und n wie vorstehend definiert sind, Z $-NH-CH_2-$, $-CONHNHCH_2-$, $-NHCONHNHCH_2-$ oder $-NHCSNHNHCH_2-$ ist und Y $-CH_2NHNHCOCH_3$ oder

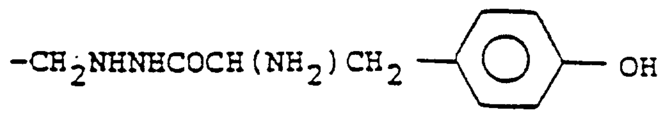

ist.

7. Produkt, das ein substituiertes Disulfid-Analogon der Formel Q-Sp-SS-W enthält, welches aus einer Verbindung der Formel $CH_3SSS$-W hergestellt ist, bei der es sich um ein N-Acyl-Derivat von LL-E33288, $\alpha_1^{I}$, $\alpha_2^{Br}$, $\alpha_2^{I}$, $\beta_1^{Br}$, $\beta_1^{I}$, $\gamma_1^{Br}$, $\gamma_1^{I}$, $\delta_1^{I}$, BBM-1675, FR-900405, FR-900406, PD114759, PD115028, CL-1577A, CL-1577B, CL-1577D, CL-1577E, CL-1724 handelt, zur Verwendung bei der Therapie bakterieller Infektionen, worin

W

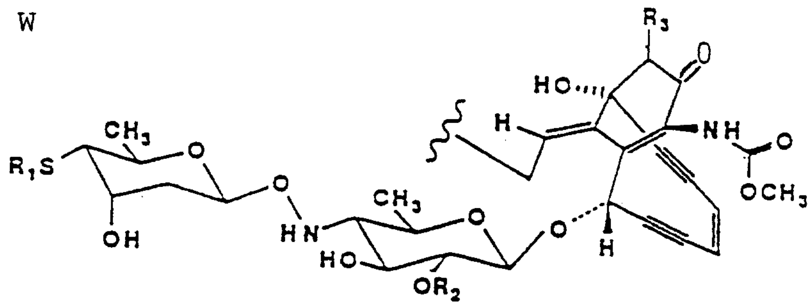

ist;

$R_1$

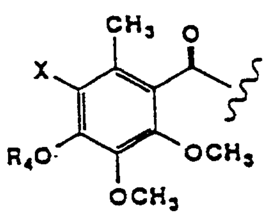

oder $CH_3$ ist;

$R_2$

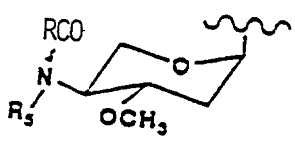

ist;

$R_3$

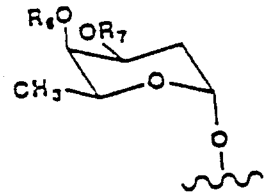

oder H ist;

$R_4$

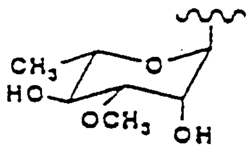

oder H ist;

$R_6$ oder $R_7$ für H oder

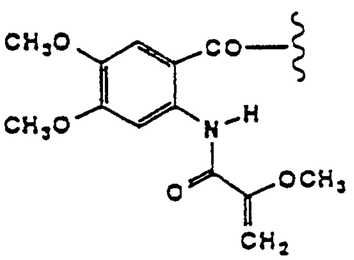

steht;

$R_5$ $CH_3$, $C_2H_5$ oder $(CH_3)_2CH$ ist;

X ein Iod- oder Bromatom ist;

R Wasserstoff oder eine verzweigte oder unverzweigte ($C_1$-$C_{10}$)-Alkyl- oder ($C_1$-$C_{10}$)-Alkylengruppe, eine Aryl- oder Heteroarylgruppe oder eine Aryl-($C_1$-$C_5$)-alkyl- oder Heteroaryl-($C_1$-$C_5$)-alkylgruppe ist, die alle gegebenenfalls mit einer oder mehreren Hydroxy-, Amino-, Carboxy-, Halogen-, Nitro-, ($C_1$-$C_3$)-Niederalkoxy- oder ($C_1$-$C_5$)-Niederthioalkoxygruppen substituiert sind;

Sp ein gerad- oder verzweigtkettiger zweiwertiger oder dreiwertiger ($C_1$-$C_{18}$)-Rest, zweiwertiger oder dreiwertiger Aryl- oder Heteroarylrest, zweiwertiger oder dreiwertiger ($C_3$-$C_{18}$)-Cycloalkyl- oder Heterocycloalkylrest, zweiwertiger oder dreiwertiger Aryl- oder Heteroaryl-($C_1$-$C_{18}$)-alkylrest, zweiwertiger oder dreiwertiger Cycloalkyl- oder Heterocycloalkyl-($C_1$-$C_{18}$)-alkylrest oder zweiwertiger oder dreiwertiger ungesättigter ($C_2$-$C_{18}$)-Alkylrest ist, worin, falls Sp ein dreiwertiger Rest ist, es zusätzlich mit Amino-, Alkylamino-, Arylamino-, Heteroarylamino-, Carboxyl-, Niederalkoxy-, Hydroxy-, Thiol- oder Niederalkylthiogruppen substituiert sein kann;

Q Wasserstoff, Halogen, Amino, Alkylamino, Dialkylamino, Arylamino, Heteroarylamino, Piperidino, Pyrrolidino, Piperazino, Carboxyl, Nitrophenyloxycarbonyl, Carboxaldehyd, Niederalkoxy, Hydroxy, Thiol, Niederalkyldicarboxyl, $-CONHNH_2$, $-NHCONHNH_2$, $-CSNHNH_2$, $-NHCSNHNH_2$ oder $-ONH_2$ ist; mit den Maßgaben, daß, wenn Sp Ethyliden ist, Q nicht Wasserstoff sein kann, und wenn $CH_3$-SSS-W LL-E33288, $\alpha_1$-I, $\alpha_2$-Br, $\alpha_2$-I, $\beta_1$-Br, $\beta_1$-I, $\gamma_1$-Br, $\gamma_1$-I, $\delta_1$-I, BBM-1675, FR-900405, FR-900406, PD114759, PR115028, CL-1577A, CL-1577B, CL-1577D, CL-1577E oder CL-1724 ist und Sp zweiwertig ist, Q nicht Wasserstoff, Halogen, Amino, Alkylamino, Dialkylamino, Arylamino, Heteroarylamino, Piperidino, Pyrrolidino, Piperazino oder Carboxyl sein kann.

**8.** Produkt, das ein substituiertes Disulfid der Formel Q-Sp-SS-W enthält, welches aus einer Verbindung der Formel $CH_3$SSS-W hergestellt ist, bei der es sich um ein N-Acyl-Derivat von LL-E33288, $\alpha_1^I$, $\alpha_2^{Br}$, $\alpha_2^I$, $\beta_1^{Br}$, $\beta_1^I$, $\gamma_1^{Br}$, $\gamma_1^I$, $\delta_1^I$, BBM-1675, FR-900405, FR-900406, PD114759, PD115028, CL-1577A, CL-1577B, CL-1577D, CL-1577E, CL-1724 handelt, zur Verwendung bei der Tumor-Therapie, worin

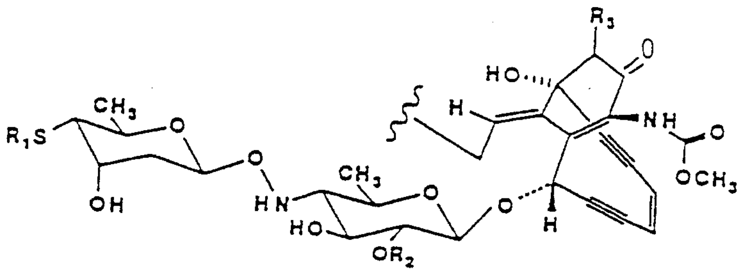

ist;

$R_1$

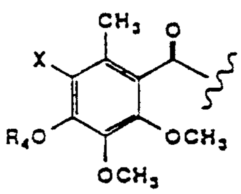

oder $CH_3$ ist;

$R_2$

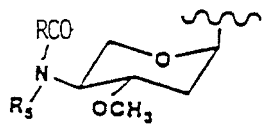

ist;

$R_3$

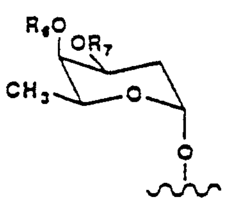

oder H ist;

$R_4$

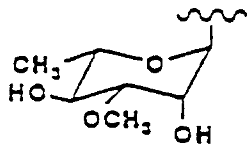

oder H ist;

$R_6$ oder $R_7$ für H oder

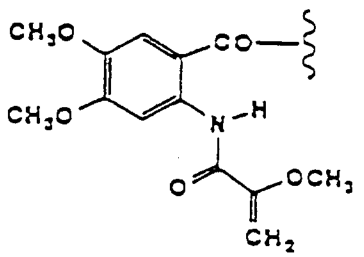

steht;

$R_5$ $CH_3$, $C_2H_5$ oder $(CH_3)_2CH$ ist;

X ein Iod- oder Bromatom ist;

worin R Wasserstoff oder eine verzweigte oder unverzweigte ($C_1$-$C_{10}$)-Alkyl- oder ($C_1$-$C_{10}$)-Alkylengruppe, eine Aryl- oder Heteroarylgruppe oder eine Aryl-($C_1$-$C_5$)-alkyl- oder Heteroaryl-($C_1$-$C_5$)-alkylgruppe ist, die alle gegebenenfalls mit einer oder mehreren Hydroxy-, Amino-, Carboxy-, Halogen-, Nitro-, ($C_1$-$C_3$)-Niederalkoxy- oder ($C_1$-$C_5$)-Niederthioalkoxygruppen substituiert sind;

Sp ein gerad- oder verzweigtkettiger zweiwertiger oder dreiwertiger ($C_1$-$C_{18}$)-Rest, zweiwertiger oder dreiwertiger Aryl- oder Heteroarylrest, zweiwertiger oder dreiwertiger ($C_3$-$C_{18}$)-Cycloalkyl- oder Heterocycloalkylrest, zweiwertiger oder dreiwertiger Aryl- oder Heteroaryl-($C_1$-$C_{18}$)-alkylrest, zweiwertiger oder dreiwertiger Cycloalkyl- oder Heterocycloalkyl-($C_1$-$C_{18}$)-alkylrest oder zweiwertiger oder dreiwertiger ungesättigter ($C_2$-$C_{18}$)-Alkylrest ist, worin, falls Sp ein dreiwertiger Rest ist, es zusätzlich mit Amino-, Alkylamino-, Arylamino-, Heteroarylamino-, Carboxyl-, Niederalkoxy-, Hydroxy-, Thiol- oder Niederalkylthiogruppen substituiert sein kann;

Q Wasserstoff, Halogen, Amino, Alkylamino, Dialkylamino, Arylamino, Heteroarylamino, Piperidino, Pyrrolidino, Piperazino, Carboxyl, Nitrophenyloxycarbonyl, Carboxaldehyd, Niederalkoxy, Hydroxy, Thiol, Niederalkyldicarboxyl, $-CONHNH_2$, $-NHCONHNH_2$, $-CSNHNH_2$, $-NHCSNHNH_2$ oder $-ONH_2$ ist; mit den Maßgaben, daß, wenn Sp Ethyliden ist, Q nicht Wasserstoff sein kann, und wenn $CH_3$-SSS-W LL-E33288, $\alpha_1$-I, $\alpha_2$-Br, $\alpha_2$-I, $\beta_1$-Br, $\beta_1$-I, $\gamma_1$-Br, $\gamma_1$-I, $\delta_1$-I, BBM-1675, FR-900405, FR-900406, PD114759, PR115028, CL-1577A, CL-1577B, CL-1577D, CL-1577E oder CL-1724 ist und Sp zweiwertig ist, Q nicht Wasserstoff, Halogen, Amino, Alkylamino, Dialkylamino, Arylamino, Heteroarylamino, Piperidino, Pyrrolidino, Piperazino oder Carboxyl sein kann.

**9.** Produkt, das eine Verbindung

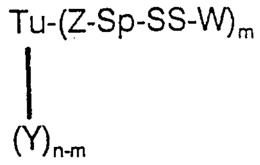

enthält, die hergestellt ist aus einer Verbindung der allgemeinen Formel $CH_3SSS$-W, wobei $CH_3SSS$-W ein N-Acyl-Derivat eines Antitumor-Antibiotikums ist, das als LL-E33288, $\alpha_1^I$, $\alpha_2^{Br}$, $\alpha_2^I$, $\beta_1^{Br}$, $\beta_1^I$, $\gamma_1^{Br}$, $\gamma_1^I$, $\delta_1^I$, BBM-1675, FR-900405, FR-900406, PD114759, PD115028, CL-1577A, CL-1577B, CL-1577D, CL-1577E, CL-1724 bezeichnet wird, durch ein Verfahren, welches umfaßt:

das Umsetzen von $CH_3SSS$-W mit einer Verbindung der allgemeinen Formel Q-Sp-SH, in der Sp ein gerad- oder verzweigtkettiger zweiwertiger oder dreiwertiger ($C_1$-$C_{18}$)-Rest, zweiwertiger oder dreiwertiger Aryl- oder Heteroarylrest, zweiwertiger oder dreiwertiger ($C_3$-$C_{18}$)-Cycloalkyl- oder Heterocycloalkylrest, zweiwertiger oder dreiwertiger Aryl- oder Heteroaryl-($C_1$-$C_{18}$)-alkylrest, zweiwertiger oder dreiwertiger Cycloalkyl- oder Heterocycloalkyl-($C_1$-$C_{18}$)-alkylrest oder zweiwertiger oder dreiwertiger ungesättigter ($C_2$-$C_{18}$)-Alkylrest ist, worin, falls Sp ein dreiwertiger Rest ist, es zusätzlich mit Amino-, Alkylamino-, Arylamino-, Heteroarylamino-, Carboxyl-, Niederalkoxy-, Hydroxy-, Thiol-, Niederalkylthiogruppen substituiert sein kann, mit der Maßgabe, daß, wenn $CH_3$-SSS-W das Ausgangs-Antitumor-Antibiotikum LL-E33288 $\alpha_1$-I, $\alpha_2$-Br, $\alpha_2$-I, $\beta_1$-Br, $\beta_1$-I, $\gamma_1$-Br, $\gamma_1$-I, $\delta_1$-I, BBM-1675, FR-900405, FR-900406, PD114759, PR115028, CL-1577A, CL-1577B, CL-1577D, CL-1577E oder CL-1724 selbst ist, Sp nicht zweiwertig sein kann; und Q Halogen, Amino, Alkylamino, Carboxyl, Carboxaldehyd, Hydroxy, Thiol, $\alpha$-Halogenacetyloxy, Niederalkyldicarboxyl, -$CONHNH_2$, -$NHCONHNH_2$, -$NHCSNHNH_2$, -$ONH_2$, -$CON_3$,

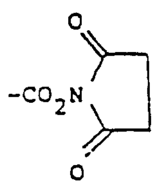 , 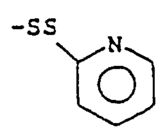 , 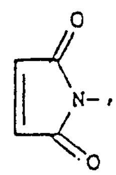 , 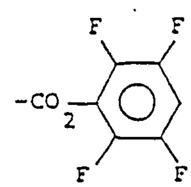 ,

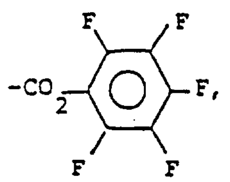 , 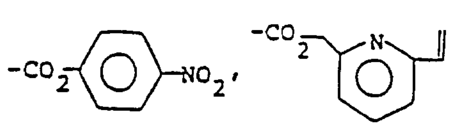 , 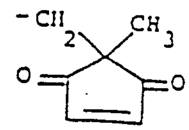

ist oder anschließend dazu umgewandelt werden kann, um ein Zwischenprodukt der Formel Q-Sp-SS-W herzustellen, in der Q, Sp und W wie vorstehend definiert sind,

das Umsetzen von Q-Sp-SS-W mit einem Molekül der Formel Tu-$(Y)_n$, in der Tu als mono- oder polyklonaler Antikörper, dessen Fragmente, dessen chemisch oder genetisch manipulierte Gegenstücke, Wachstumsfaktoren oder Steroide definiert ist; Y eine Seitenketten-Amino, -Carboxy- oder -Thiolgruppe eines Proteins, ein Aldehyd, der von Glycoproteinkohlehydrat-Resten abstammt, oder eine Amidoalkylthiogruppe ist; und n eine ganze Zahl von 1 bis 100 ist, um eine Verbindung der Formel:

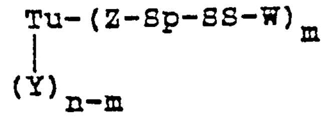

herzustellen, in der Tu, Y, Sp, W und n wie vorstehend definiert sind und Z direkt oder nach anschließender Reduktion aus einer kovalenten Reaktion der Gruppen Q und Y gebildet ist und Z -CONH-, -CONHN=CH-, -CONHNHCH$_2$-, -NHCONHN=CH-, -NHCONHNHCH$_2$-, -NHCSNHN=CH-, -NHCSNHNHCH$_2$-, -ON=CH-, -NH-, -NHCH$_2$-, -N=CH-, -CO$_2$-, -NHCH$_2$CO$_2$-, -SS-,

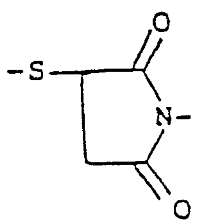 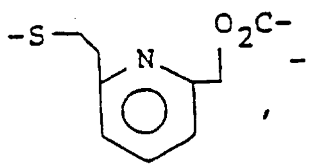 , 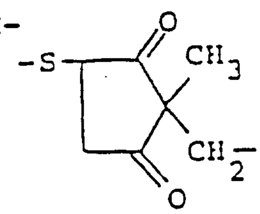 oder 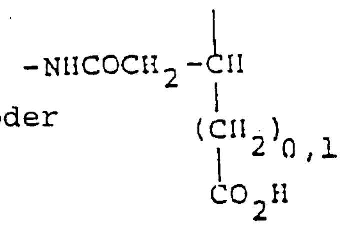

ist und m 0,1 bis 15 ist, zur Verwendung bei der Tumor-Therapie.

**10.** Produkt eines Protein-Arzneistoff-Konjugats der Formel

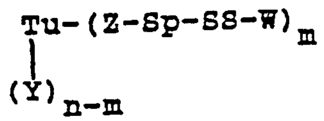

hergestellt aus dem Antitumor-Antibiotikum, welches als N-Acetyl-LL-E33288$\gamma_1{}^{I}$ (CH$_3$SSS-W) bezeichnet wird, mit

a) einem Ultraviolett-Spektrum, wie in Figur 1 gezeigt;
b) einem Infrarot-Spektrum, wie in Figur 2 gezeigt;
c) einem magnetischen Protonenresonanzspektrum, wie in Figur 3 gezeigt; und
d) einem kernmagnetischen Kohlenstoff-13-Resonanzspektrum, wie in Figur 4 gezeigt;

durch ein Verfahren, welches umfaßt:

das Verdrängen der Dithiomethyl-Einheit durch eine Verbindung der Formel Q-Sp-SH, in der Sp ein gerad- oder verzweigtkettiger zweiwertiger oder dreiwertiger (C$_2$-C$_5$)-Rest oder zweiwertiger oder dreiwertiger Aryl- oder Heteroaryl-(C$_2$-C$_5$)-alkylrest ist, worin, falls Sp ein dreiwertiger Rest ist, es zusätzlich mit Amino-, Heteroarylamino-, Hydroxy- oder Thiolgruppen substituiert sein kann; und Q Carboxyl, Niederalkyldicarboxylanhydrid, -CONHNH$_2$ oder

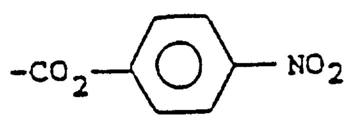

ist oder anschließend dazu umgewandelt werden kann, um ein Zwischenprodukt der allgemeinen Formel Q-Sp-SS-W herzustellen, in der Q, Sp und W wie vorstehend definiert sind,

das Umsetzen von Q-Sp-SS-W mit einem Molekül der Formel Tu-$(Y)_n$, in der Tu ein monoklonaler Antikörper ist, der eine bevorzugte Reaktivität mit einem menschlichen Tumorassoziierten Antigen aufweist, Y eine Seitenketten-Aminogruppe an dem Antikörper oder ein Aldehyd ist, der durch Oxidation der Kohlehydratgruppen des Antikörpers erzeugt ist, und n eine ganze Zahl von 1 bis 100 ist, um eine Verbindung der Formel:

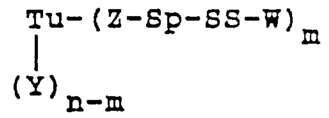

herzustellen, in der Tu, Y, Sp, W und n wie vorstehend definiert sind und Z direkt oder nach anschließender Reduktion aus einer kovalenten Reaktion der Gruppen Q und Y gebildet ist und Z -CONH-, -CONHN=CH-, -$CONHNHCH_2$- oder

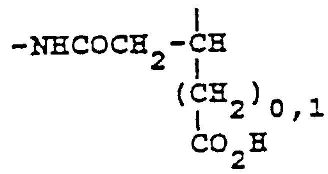

ist und m 0,1 bis 15 ist, zur Verwendung bei der Tumor-Therapie für die in-vivo-Zufuhr des Konjugats an einen antigenen Ort.

**Patentansprüche für folgende Vertragsstaaten : ES, GR**

1. Verfahren zur Herstellung eines substituierten Disulfids der Formel Q-Sp-SS-W aus einer Verbindung der Formel $CH_3SSS$-W, welche ein N-Acyl-Derivat eines Antitumor-Antibiotikums ist, das als LL-E33288, $\alpha_1$-I, $\alpha_2$-Br, $\alpha_2$-I, $\beta_1$-Br, $\beta_1$-I, $\gamma_1$-Br, $\gamma_1$-I, $\delta_1$-I, BBM-1675, FR-900405, FR-900406, PD114759, PD115028, CL-1577A, CL-1577B, CL-1577D, CL-1577E, CL-1724 bezeichnet wird, worin

W

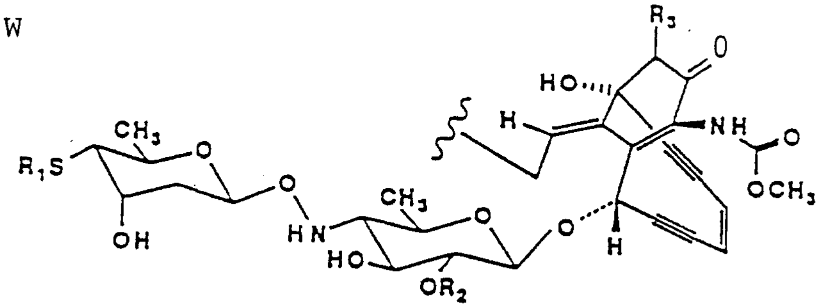

ist;

$R_1$

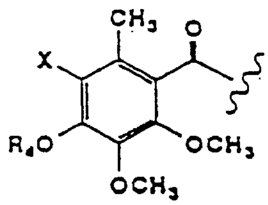

oder $CH_3$ ist;

$R_2$

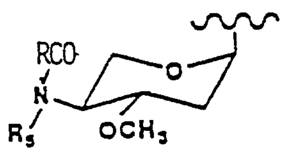

ist;

$R_3$

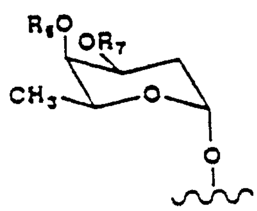

oder H ist;

$R_4$

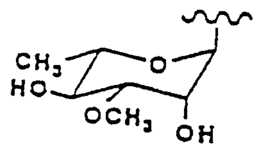

oder H ist;

$R_6$ oder $R_7$ für H oder

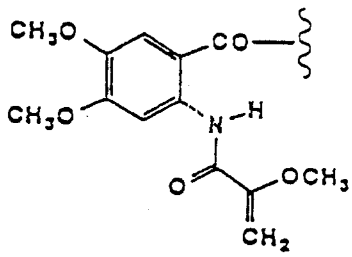

steht;

$R_5$ $CH_3$, $C_2H_5$ oder $(CH_3)_2CH$ ist;

X ein lod- oder Bromatom ist;

R Wasserstoff oder eine verzweigte oder unverzweigte $(C_1-C_{10})$-Alkyl- oder $(C_1-C_{10})$-Alkylengruppe, eine Aryl- oder Heteroarylgruppe oder eine Aryl-$(C_1-C_5)$-alkyl- oder Heteroaryl-$(C_1-C_5)$-alkylgruppe ist, die alle gegebenenfalls mit einer oder mehreren Hydroxy-, Amino-, Carboxy-, Halogen-, Nitro-, $(C_1-C_3)$-Niederalkoxy- oder $(C_1-C_5)$-Niederthioalkoxygruppen substituiert sind;

Sp ein gerad- oder verzweigtkettiger zweiwertiger oder dreiwertiger $(C_1-C_{18})$-Rest, zweiwertiger oder dreiwertiger Aryl- oder Heteroarylrest, zweiwertiger oder dreiwertiger $(C_3-C_{18})$-Cycloalkyl- oder Heterocycloalkylrest, zweiwertiger oder dreiwertiger Aryl- oder Heteroaryl-$(C_1-C_{18})$-alkylrest, zweiwertiger oder dreiwertiger Cycloalkyl- oder Heterocycloalkyl-$(C_1-C_{18})$-alkylrest oder zweiwertiger oder dreiwertiger ungesättigter $(C_2-C_{18})$-Alkylrest ist, worin, falls Sp ein dreiwertiger Rest ist, es zusätzlich mit Amino-, Alkylamino-, Arylamino-, Heteroarylamino-, Carboxyl-, Niederalkoxy-, Hydroxy-, Thiol- oder Niederalkylthiogruppen substituiert sein kann;

Q Wasserstoff, Halogen, Amino, Alkylamino, Dialkylamino, Arylamino, Heteroarylamino, Piperidino, Pyrrolidino, Piperazino, Carboxyl, Nitrophenyloxycarbonyl, Carboxaldehyd, Niederalkoxy, Hydroxy, Thiol, Niederalkyldicarboxyl, $-CONHNH_2$, $-NHCONHNH_2$, $-CSNHNH_2$, $-NHCSNHNH_2$ oder $-ONH_2$ ist; mit den Maßgaben, daß, wenn Sp Ethyliden ist, Q nicht Wasserstoff sein kann; und wenn $CH_3$-SSS-W LL-E33288, $\alpha_1$-I, $\alpha_2$-Br, $\alpha_2$-I, $\beta_1$-Br, $\beta_1$-I, $\gamma_1$-Br, $\gamma_1$-I, $\delta_1$-I, BBM-1675, FR-900405, FR-900406, PD114759, PR115028, CL-1577A, CL-1577B, CL-1577D, CL-1577E oder CL-1724 ist und Sp zweiwertig ist, Q nicht Wasserstoff, Halogen, Amino, Alkylamino, Dialkylamino, Arylamino, Heteroarylamino, Piperidino, Pyrrolidino, Piperazino oder Carboxyl sein kann;

umfassend das Umsetzen eines Methyltrisulfid-Antitumor-Antibiotikums aus denjenigen der obigen mit einem geeignet substituierten Mercaptan in Acetonitril oder einer Kombination von Acetonitril und Tetrahydrofuran oder Acetonitril und Dimethylformamid bei einer Temperatur von zwischen -20 bis etwa +40°C über einen Zeitraum von einer Stunde bis sechs Tagen, vorzugsweise in Anwesenheit eines Äquivalents einer tertiären Amin-Base oder einer Mischung eines Äquivalents einer tertiären Amin-Base und eines Äquivalents einer organischen Carbonsäure.

**2.** Verfahren nach Anspruch 1, ien dem eine substituiertes Disulfid der Formel Q-Sp-SS-W hergestellt wird aus einer Verbindung der Formel $CH_3SSS$-W, in der:

W

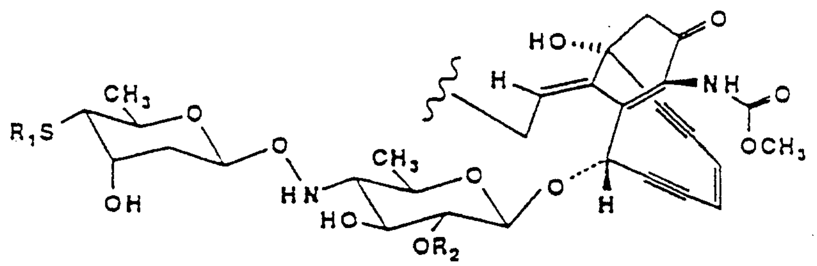

ist;

$R_1$

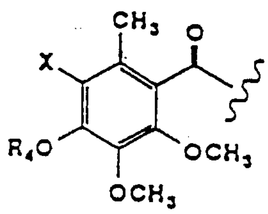

ist;

$R_2$

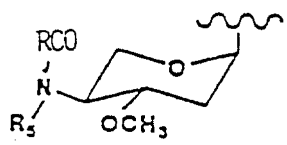

ist;

$R_4$

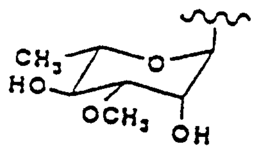

oder H ist;

$R_5$ $CH_3$, $C_2H_5$ oder $(CH_3)_2CH$ ist; X ein Iod- oder Bromatom ist; worin R Wasserstoff, $CH_3$ oder eine monosubstituierte Arylgruppe ist; und Sp und Q wie definiert sind.

3. Verfahren zur Herstellung der Ziel-Derivate

$$\begin{array}{l} Tu-(Z-Sp-SS-W)_m \\ | \\ (Y)_{n-m} \end{array}$$

von Verbindungen der Formel $CH_3SSS$-W, in der $CH_3SSS$-W ein N-Acyl-Derivat eines Antitumor-Antibiotikums LL-E33288, $\alpha_1^I$, $\alpha_2^{Br}$, $\alpha_2^I$, $\beta_1^{Br}$, $\beta_1^I$, $\gamma_1^{Br}$, $\gamma_1^I$, $\delta_1^I$, BBM-1675, FR-900405, FR-900406, PD114759, PD115028, CL-1577A, CL-1577B, CL-1577D, CL-1577E, CL-1724 ist, umfassend

das Umsetzen von $CH_3SSS$-W mit einer Verbindung der Formel Q-Sp-SH, in der Sp ein gerad- oder verzweigtkettiger zweiwertiger oder dreiwertiger $(C_1$-$C_{18})$-Rest, zweiwertiger oder dreiwertiger Aryl- oder Heteroarylrest, zweiwertiger oder dreiwertiger $(C_3$-$C_{18})$-Cycloalkyl- oder Heterocycloalkylrest, zweiwertiger oder dreiwertiger Aryl- oder Heteroaryl-$(C_1$-$C_{18})$-alkylrest, zweiwertiger oder dreiwertiger Cycloalkyl- oder Heterocycloalkyl-$(C_1$-$C_{18})$-alkylrest oder zweiwertiger oder dreiwertiger ungesättigter $(C_2$-$C_{18})$-Alkylrest ist, worin, falls Sp ein dreiwertiger Rest ist, es zusätzlich mit Amino-, Alkylamino-, Arylamino-, Heteroarylamino-, Carboxyl-, Niederalkoxy-, Hydroxy-, Thiol- oder Niederalkylthiogruppen substituiert sein kann, mit der Maßgabe, daß, wenn $CH_3$-SSS-W das Ausgangs-Antitumor-Antibiotikum LL-E33288 , $\alpha_1$-I, $\alpha_2$-Br, $\alpha_2$-I, $\beta_1$-Br, $\beta_1$-I, $\gamma_1$-Br, $\gamma_1$-I, $\delta_1$-I, BBM-1675, FR-900405, FR-900406, PD114759, PR115028, CL-1577A, CL-1577B, CL-1577D, CL-1577E oder CL-1724 selbst ist, Sp nicht zweiwertig sein kann; und Q Halogen, Amino, Alkylamino, Carboxyl, Carboxaldehyd, Hydroxy, Niederalkyldicarboxylanhydrid, $-CONHNH_2$, $-NHCONHNH_2$, $-NHCSNHNH_2$, $-ONH_2$ oder

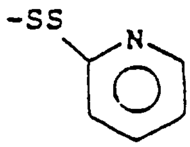

ist, in Acetonitril in Anwesenheit eines Äquivalents Triethylamin oder eines Äquivalents Triethylamin und eines Äquivalents Essigsäure bei -10 bis -30°C über 1 - 48 Stunden,

das Isolieren des Zwischenproduktes der Formel Q-Sp-SS-W, in der Q, Sp und W wie vorstehend in Anspruch 1 definiert sind, dann

das Umsetzen der Verbindung der Formel Q-Sp-SS-W, in der Sp und W wie vorstehend definiert sind und Q Halogen, Amino, Alkylamino, Carboxyl, Carboxaldehyd, Hydroxy oder Niederalkyldicarbonsäureanhydrid ist, mit einem Molekül der Formel Tu-$(Y)_n$, worin es sich bei Tu um einen mono- oder polyklonalen Antikörper, dessen Fragmente, dessen chemisch oder genetisch manipulierte Gegenstücke oder Wachstumsfaktoren oder Steroide handelt; Y eine Seitenketten-Amino- oder Carboxy-Funktionalität ist; n 1 - 100 ist, in wäßrigem Puffer bei einem pH zwischen 6,5 und 9 bei 4 bis 40°C entweder direkt oder in Anwesenheit eines wasserlöslichen Carbodiimids, um die Verbindung

$$\begin{array}{l} Tu-(Z-Sp-SS-W)_m \\ | \\ (Y)_{n-m} \end{array}$$

herzustellen, in der Tu, Sp, W, n und Y wie vorstehend definiert sind, m 1 - 15 ist und Z aus einer kovalenten

Reaktion der Gruppen Q und Y gebildet ist und -CONH-, -NH-,

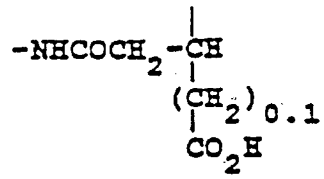

-N=CH- oder $-CO_2-$ ist,
oder

das Umsetzen der Verbindung der Formel Q-Sp-SS-W, in der Sp und W wie vorstehend definiert sind und Q eine Carbonsäure ist, mit N-Hydroxysuccinimid, 2,3,5,6-Tetrafluorphenol, Pentafluorphenol oder 4-Nitrophenol in Anwesenheit eines Carboxyl-aktivierenden Mittels, wie eines Carbodiimids, um eine Verbindung der Formel Q-Sp-SS-W herzustellen, in der Sp und W wie vorstehend definiert sind und Q

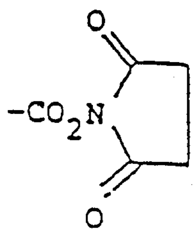 , 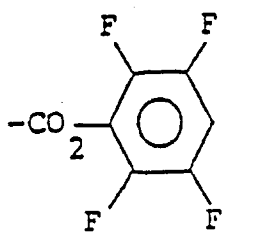 , 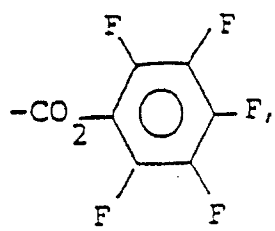

oder

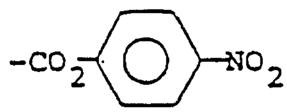

ist, dann mit einem Molekül der Formel $Tu-(Y)_n$, in der Tu und n wie vorstehend definiert sind und Y eine Seitenketten-Aminogruppe ist, in einer wäßrigen gepufferten Lösung bei einem pH zwischen 6,5 und 9 bei einer Temperatur zwischen 4 und 40°C einschließlich, um Verbindungen der Formel:

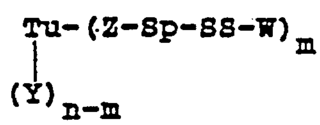

herzustellen, in der Tu, Sp, Y und n wie vorstehend definiert sind, m 1 - 15 ist und Z aus einer kovalenten Reaktion zwischen Q und Y gebildet ist und als -CONH- definiert ist
oder

das Umsetzen einer Verbindung der Formel Q-Sp-SS-W, in der Sp und W wie vorstehend definiert sind und Q $-CONHNH_2$ ist, mit salpetriger Säure in wäßrigem Acetonitril, um eine Verbindung der Formel Q-Sp-SS-W herzustellen, in der Sp und W wie vorstehend definiert sind und Q $-CON_3$ ist, dann mit einer Verbindung der Formel $Tu-(Y)_n$, in der Tu, Y und n wie vorstehend definiert sind, um eine Verbindung der Formel

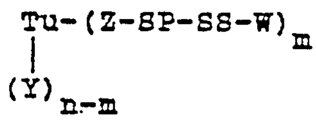

herzustellen, in der Tu, Z, Sp, W, m, Y und n wie vorstehend definiert sind;
oder

das Umsetzen einer Verbindung der Formel Q-Sp-SS-W, in der Sp und W wie vorstehend definiert sind und Q Hydroxy ist, mit einem alpha-Halogenacetanhydrid, um eine Verbindung herzustellen, in der Q α-Halogenacetyloxy ist, und das Umsetzen des α-Halogenacetyloxy-Sp-SS-W oder einer Verbindung der Formel Q-Sp-SS-W, in der Sp und W wie vorstehend definiert sind und Q

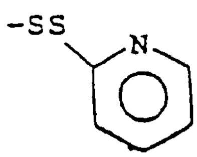 , 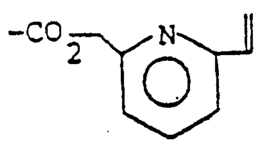 oder 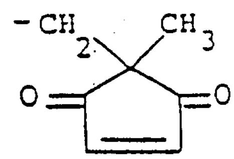

ist, mit einem Molekül der Formel Tu-$(Y)_n$, in der Tu wie vorstehend definiert ist, Y für ein seitenketten-Thiol eines Proteins oder eine Amidoalkylthiogruppe, die an einem Amin von Tu unter Verwendung von Reagenzien wie 3-(2-Dithiopyridyl)propionsäurehydroxysuccinimidester, gefolgt von der Reduktion mit einem Mittel wie Dithiothreit, eingeführt wurde, oder eine Amidoalkylthiogruppe steht, die unter Verwendung von 2-Iminothiolan an einem Amin von Tu eingeführt wurde, und n 1 - 10 ist, unter wäßrigen gepufferten Bedingungen bei einem pH zwischen 4,5 und 7 bei einer Temperatur zwischen 4 und 40°C einschließlich, um eine Verbindung der Formel:

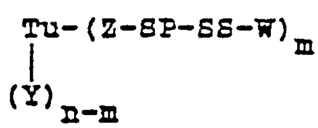

herzustellen, in der Tu, Sp, W und n wie vorstehend definiert sind und Z aus einer kovalenten Reaktion der Gruppen Q und Y gebildet ist und Z
-SS-,

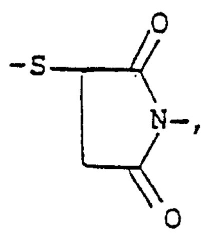 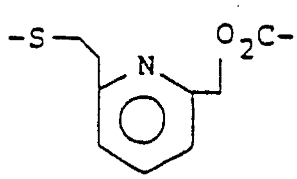 oder 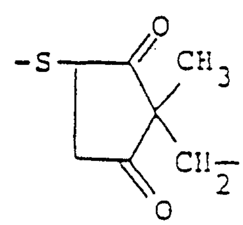

ist und n 0,1 bis 10 ist;
oder

das Umsetzen einer Verbindung der Formel Q-Sp-SS-W, in der Sp und W wie vorstehend definiert sind und Q

$-NH_2$, $-CONHNH_2$, $-NHCONHNH_2$, $-NHCSNHNH_2$ oder $-ONH_2$ ist, mit einem Molekül der Formel $Tu\text{-}(Y)_n$, in der Tu wie vorstehend definiert ist, Y ein Aldehyd ist, der aus Kohlehydrat-Resten an Tu durch Oxidation in Anwesenheit eines Erdalkaliperiodats erzeugt worden ist, in einem wäßrigen Puffer bei einem pH zwischen 4,0 und 6,5 bei 4 bis 40°C einschließlich, und n 1 bis 20 ist, um eine Verbindung der Formel:

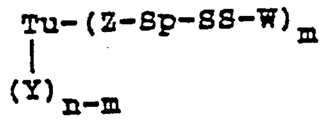

herzustellen, in der Tu, Sp, W, Y und n wie vorstehend definiert sind und Z aus der kovalenten Reaktion von Q und Y gebildet ist und -ON=CH-, -N=CH-, -CONHN=CH-, -NHCONHN=CH- oder -NHCSNHN=CH- ist und m 0,1 bis 15 ist; oder das Behandeln der unmittelbar vorstehenden Verbindung der Formel:

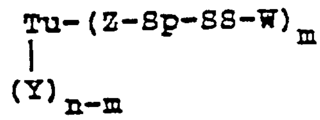

in der Tu, Z, Sp, W, Y, n und m wie unmittelbar vorstehend definiert sind, mit Acetylhydrazin oder Tyrosinhydrazin in einem wäßrigen Puffer bei einem pH zwischen 4,0 und 6,5 bei 4 bis 40°C einschließlich, um eine Verbindung der Formel:

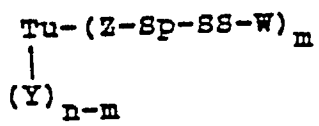

herzustellen, in der Tu, Z, Sp, W, n und m wie unmittelbar vorstehend definiert sind und Y $-CH=NNHCOCH_3$ oder

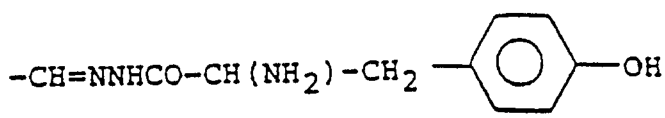

ist, und

das Umsetzen dieser Verbindung mit Natriumcyanborhydrid oder Natriumborhydrid in einem wäßrigen Puffer bei einem pH von 4,0 bis 6,5 bei einer Temperatur von 4 bis 40°C einschließlich, um eine Verbindung der Formel:

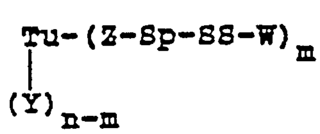

herzustellen, in der Tu, Sp, W, m und n wie vorstehend definiert sind, Z $-NH\text{-}CH_2\text{-}$, $-CONHNHCH_2\text{-}$, $-NHCONHNHCH_2\text{-}$ oder $-NHCSNHNHCH_2\text{-}$ ist und Y $-CH_2NHNHCOCH_3$ oder

$-CH_2NHNHCOCH(NH_2)CH_2$ 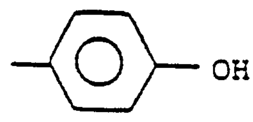

ist.

4. Verfahren zur Herstellung eines Träger-Arzneistoff-Konjugats der Formel

$$\begin{array}{l} Tu-(Z-Sp-SS-W)_m \\ | \\ (Y)_{n-m} \end{array}$$

hergestellt aus einer Verbindung der Formel $CH_3SSS$-W, in der $CH_3SSS$-W ein N-Acyl-Derivat eines Antitumor-Antibiotikums ist, das als LL-E33288, $\alpha_1^I$, $\alpha_2^{Br}$, $\alpha_2^I$, $\beta_1^{Br}$, $\beta_1^I$, $\gamma_1^{Br}$, $\gamma_1^I$, $\delta_1^I$, BBM-1675, FR-900405, FR-900406, PD114759, PD115028, CL-1577A, CL-1577B, CL-1577D, CL-1577E, CL-1724 bezeichnet wird, worin W wie vorstehend in Anspruch 1 beschrieben ist, umfassend:

das Umsetzen von $CH_3SSS$-W mit einer Verbindung der allgemeinen Formel Q-Sp-SH, worin Sp ein gerad- oder verzweigtkettiger zweiwertiger oder dreiwertiger ($C_1$-$C_{18}$)-Rest, zweiwertiger oder dreiwertiger Aryl- oder Heteroarylrest, zweiwertiger oder dreiwertiger ($C_3$-$C_{18}$)-Cycloalkyl- oder Heterocycloalkylrest, zweiwertiger oder dreiwertiger Aryl- oder Heteroaryl-($C_1$-$C_{18}$)-alkylrest, zweiwertiger oder dreiwertiger Cycloalkyl- oder Heterocycloalkyl-($C_1$-$C_{18}$)-alkylrest oder zweiwertiger oder dreiwertiger ungesättigter ($C_2$-$C_{18}$)-Alkylrest ist, worin, falls Sp ein dreiwertiger Rest ist, es zusätzlich durch Amino-, Alkylamino-, Arylamino-, Heteroarylamino-, Carboxyl-, Niederalkoxy-, Hydroxy-, Thiol- oder Niederalkylthiogruppen substituiert sein kann, mit der Maßgabe, daß, wenn $CH_3SSS$-W in dem Ausgangs-Antitumor-Antibiotikum LL-E33288 $\alpha_1$-Br, $\alpha_1$-I, $\alpha_2$-Br, $\alpha_2$-I, $\beta_1$-Br, $\beta_1$-I, $\gamma_1$-Br, $\gamma_1$-I, $\delta_1$-I, BBM-1675, FR-900405, FR-900406, PD114759, PR115028, CL-1577A, CL-1577B, CL-1577D, CL-1577E oder CL-1724 selbst ist, Sp nicht zweiwertig sein kann; und Q Halogen, Amino, Alkylamino, Carboxyl, Carboxaldehyd, Hydroxy, Thiol, $\alpha$-Halogenacetyloxy, Niederalkyldicarboxyl, $-CONHNH_2$, $-NHCONHNH_2$, $-NHCSNHNH_2$, $-ONH_2$, $-CON_3$,

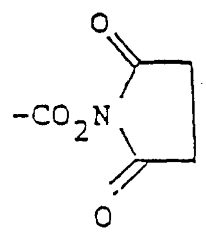 , 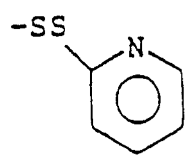 , 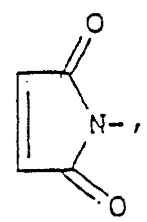 , 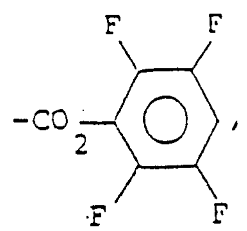 ,

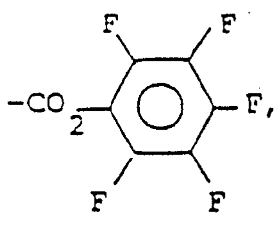 , 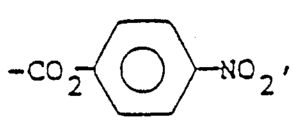 , 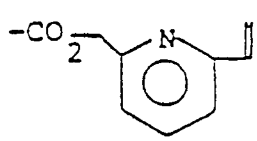 , 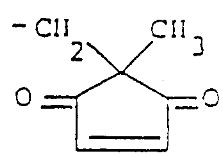

ist oder anschließend dazu umgewandelt werden kann,

um ein Zwischenprodukt der Formel Q-Sp-SS-W herzustellen, in der Q, Sp und W wie vorstehend definiert sind,

das Umsetzen von Q-Sp-SS-W mit einem Molekül der Formel Tu-$(Y)_n$, worin der Träger Tu als ein mono- oder polyklonaler Antikörper, dessen Fragmente, dessen chemisch oder genetisch manipulierte Gegenstücke oder Wachstumsfaktoren oder Steroide definiert ist; Y eine Seitenketten-Amino-, -Carboxy- oder -Thiolgruppe eines Proteins, ein Aldehyd, der von Glycoproteinkohlehydrat-Resten abstammt, oder eine Amidoalkylthiogruppe ist; und n eine ganze Zahl von 1 bis 100 ist, um eine Verbindung der Formel:

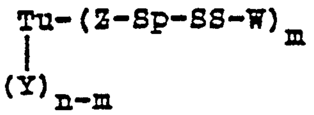

herzustellen, in der Tu, Y, Sp, W und n wie vorstehend definiert sind und Z aus einer kovalenten Reaktion der Gruppen Q und Y direkt oder nach anschließender Reduktion gebildet ist und Z -CONH-, -CONHN=CH-, -CONHNH$CH_2$-, -NHCONHN=CH-, -NHCONHNH$CH_2$-, -NHCSNHN=CH-, -NHCSNHNH$CH_2$-, -ON=CH-, -NH-, -NH$CH_2$-, -N=CH-, -$CO_2$-, -NH$CH_2CO_2$-, -SS-,

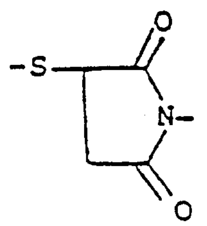 , 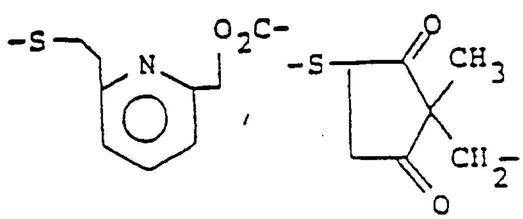 oder 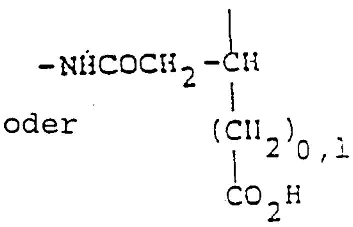

ist und m 0,1 bis 15 ist.

**5.** Verfahren zur Herstellung eines Protein-Arzneistoff-Konjugats, wie in Anspruch 4 hergestellt, der Formel

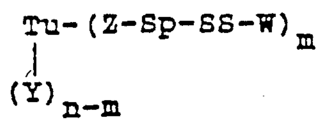

hergestellt aus der Klasse von N-Acyl-Derivaten von Antitumor-Antiobiotika, die als LL-E33288 ($CH_3$SSS-W) bezeichnet werden, umfassend:

das Verdrängen der Dithiomethyl-Einheit durch eine Verbindung der Formel Q-Sp-SH, in der Sp ein gerad- oder verzweigtkettiger zweiwertiger oder dreiwertiger ($C_2$-$C_{10}$)-Rest oder zweiwertiger oder dreiwertiger Aryl- oder Heteroaryl-($C_2$-$C_5$)-alkylrest ist, worin, falls Sp ein dreiwertiger Rest ist, es zusätzlich durch Amino-, Heteroarylamino-, Hydroxy- oder Thiolgruppen substituiert sein kann; und Q Carboxyl, Niederalkyldicarboxylanhydrid, -$CO_2$Su, -CONH$NH_2$ oder

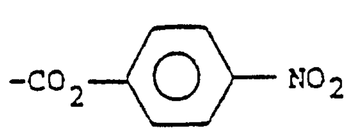

ist oder anschließend dazu umgewandelt werden kann, um ein Zwischenprodukt der allgemeinen Formel Q-Sp-SS-W herzustellen, in der Q, Sp und W wie vorstehend definiert sind,

das Umsetzen von Q-Sp-SS-W mit einem Molekül der Formel Tu-$(Y)_n$, in der Tu ein monoklonaler Antikörper ist, der eine bevorzugte Reaktivität mit einem menschlichen Tumor-assoziierten Antigen aufweist, Y eine Seitenketten-Aminogruppe am Antikörper oder ein Aldehyd ist, der durch Oxidation der Kohlehydratgruppen des Antikörpers erzeugt wird, und n eine ganze Zahl von 1 bis 100 ist, um eine Verbindung der Formel

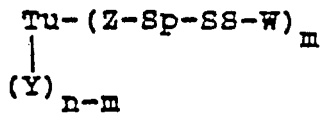

herzustellen, in der Tu, Y, Sp, W und n wie vorstehend definiert sind und Z aus einer kovalenten Reaktion der Gruppen Q und Y direkt oder nach anschließender Reduktion gebildet wird und Z -CONH-, -CONHN=CH-, -$CONHNHCH_2$- oder

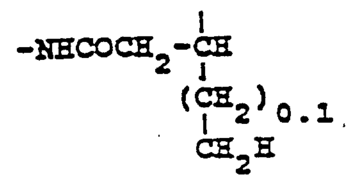

ist und m 0,1 bis 15 ist.

**6.** Verfahren zur Herstellung eines Protein-Arzneistoff-Konjugats der Formel

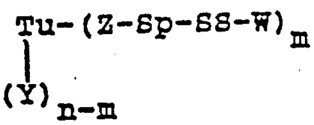

hergestellt aus dem Antitumor-Antibiotikum, welches als N-Acetyl-LL-E33288$\gamma_1{}^I$ ($CH_3$SSS-W) bezeichnet wird, mit

a) einem Ultraviolett-Spektrum, wie in Figur 1 gezeigt;
b) einem Infrarot-Spektrum, wie in Figur 2 gezeigt;
c) einem magnetischen Protonenresonanzspektrum, wie in Figur 3 gezeigt; und
d) einem kernmagnetischen Kohlenstoff-13-Resonanzspektrum, wie in Figur 4 gezeigt;

wobei das Verfahren umfaßt:

das Verdrängen der Dithiomethyl-Einheit durch eine Verbindung der Formel Q-Sp-SH, in der Sp ein gerad- oder verzweigtkettiger zweiwertiger oder dreiwertiger ($C_2$-$C_5$)-Rest oder zweiwertiger oder dreiwertiger Aryl- oder Heteroaryl-($C_2$-$C_5$)-alkylrest ist, worin, falls Sp ein dreiwertiger Rest ist, es zusätzlich mit Amino-, Heteroarylamino-, Hydroxy- oder Thiolgruppen substituiert sein kann; und Q Carboxyl, Niederalkyldicarboxylanhydrid, -$CONHNH_2$ oder

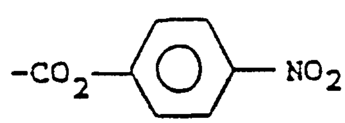

ist oder anschließend dazu umgewandelt werden kann, um ein Zwischenprodukt der allgemeinen Formel Q-Sp-SS-W herzustellen, in der Q, Sp und W wie vorstehend definiert sind,

das Umsetzen von Q-Sp-SS-W mit einem Molekül der Formel Tu-$(Y)_n$, in der Tu ein monoklonaler Antikörper ist, der eine bevorzugte Reaktivität mit einem menschlichen Tumorassoziierten Antigen aufweist, Y eine Seitenketten-Aminogruppe an dem Antikörper oder ein Aldehyd ist, der durch Oxidation der Kohlehydratgruppen des Antikörpers erzeugt ist, und n eine ganze Zahl von 1 bis 100 ist, um eine Verbindung der Formel:

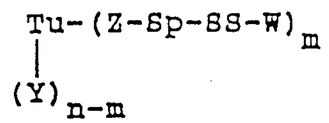

herzustellen, in der Tu, Y, Sp, W und n wie vorstehend definiert sind und Z direkt oder nach anschließender Reduktion aus einer kovalenten Reaktion der Gruppen Q und Y gebildet ist und Z -CONH-, -CONHN=CH-, -$CONHNHCH_2$- oder

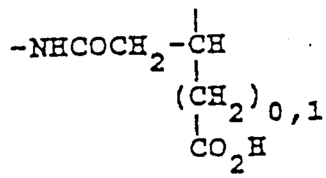

ist und m 0,1 bis 15 ist.

**Revendications**

**Revendications pour les Etats contractants suivants : AT, BE, CH, DE, DK, FR, GB, IT, LI, NL, SE**

1. Disulfure substitué de formule Q-Sp-SS-W, préparé à partir d'un composé de formule $CH_3SSS$-W qui est un dérivé N-acylé d'un antibiotique antitumoral dénommé LL-E33288,$\alpha_1$-I, $\alpha_2$-Br, $\alpha_2$-I, $\beta_1$-Br, $\beta_1$-I, $\gamma_1$-Br, $\gamma_1$-I, $\delta_1$-I, BBM-1675, FR-900405, FR-900406, PD114759, PD115028, CL-1577A, CL-1577B, CL-1577D, CL-1577E ou CL-1724, dans lequel :

W représente 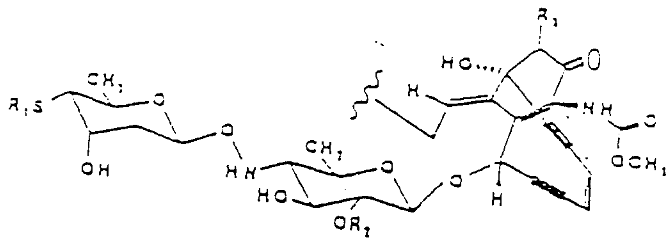

$R_1$ représente 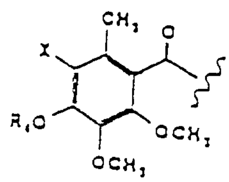 ou $CH_3$ ;

$R_2$ représente 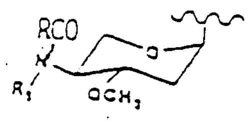 ;

$R_3$ représente 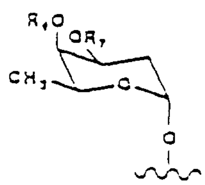 ou H ;

$R_4$ représente 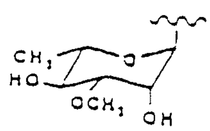 ou H ;

$R_6$ ou $R_7$ représente H ou 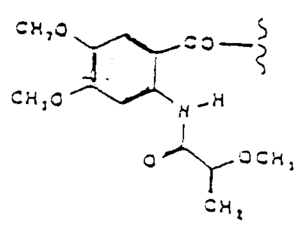

$R_5$ représente $CH_3$, $C_2H_5$ ou $(CH_3)_2CH$ ;
X représente un atome d'iode ou de brome ;
R représente un atome d'hydrogène, ou un groupe alkyle en $C_1$-$C_{10}$ ou alkylène en $C_1$-$C_{10}$ ramifié ou non ramifié, un groupe aryle ou hétéroaryle, ou un groupe aryl-(alkyle en $C_1$-$C_5$) ou hétéroaryl-(alkyle en $C_1$-$C_5$), tous étant éventuellement substitués avec un ou plusieurs groupes hydroxyle, amino, carboxy, halo, nitro, alkoxy inférieurs en $C_1$-$C_3$ ou thioalkoxy inférieurs en $C_1$-$C_5$ ; Sp représente des groupes divalents ou trivalents en $C_1$ à $C_{18}$ à chaîne linéaire ou ramifiée, des groupes aryle ou hétéroaryle divalents ou trivalents, des groupes cycloalkyle ou hétérocycloalkyle en $C_3$-$C_{18}$ divalents ou trivalents, des groupes aryl- ou hétéroaryl-(alkyle en $C_1$-$C_{18}$) divalents ou trivalents, des groupes cycloalkyl-e ou hétérocycloalkyl-(alkyle en $C_1$-$C_{18}$) divalents ou trivalents, ou des groupes alkyle insaturés en $C_2$-$C_{18}$ divalents ou trivalents, où si Sp représente un groupe trivalent, il peut être en outre substitué avec des groupes amino, alkylamino, arylamino, hétéroarylamino, carboxyle, alkoxy inférieurs, hydroxy, thiol ou alkylthio inférieurs ; Q représente un atome d'hydrogène, d'halogène, un groupe amino, alkylamino, dialkylamino, arylamino, hétéroarylamino, pipéridino, pyrrolidino, pipérazino, carboxyle, nitrophényloxycarbonyle, carboxaldéhyde, alkoxy inférieur, hydroxy, thiol, (alkyl inférieur)-dicarboxyle, $-CONHNH_2$, $-NHCONHNH_2$, $-CSNHNH_2$, $-NHCSNHNH_2$ ou $-ONH_2$ ; à condition que lorsque Sp représente un groupe éthylidyne, Q ne puisse pas être un atome d'hydrogène, et que lorsque $CH_3$-SSS-W est LL-33288, $\alpha_1$-I, $\alpha_2$-Br, $\alpha_2$-I, $\beta_1$-Br, $\beta_1$-I, $\gamma_1$-Br, $\gamma_1$-I, $\delta_1$-I, BBM-1675, FR-900405, FR-900406, PD114759, PD115028, CL-1577A, CL-1577B, CL-1577D, CL-1577E ou CL-1724 et que Sp est divalent, Q ne puisse pas être un atome d'hydrogène, d'halogène, un groupe amino, alkylamino, dialkylamino, arylamino, hétéroarylamino, pipéridino, pyrrolidino, pipérazino ou carboxyle.

2. Disulfure substitué de formule Q-Sp-SS-W selon la revendication 1, préparé à partir d'un composé de formule $CH_3SSS$-W dans laquelle :

W représente 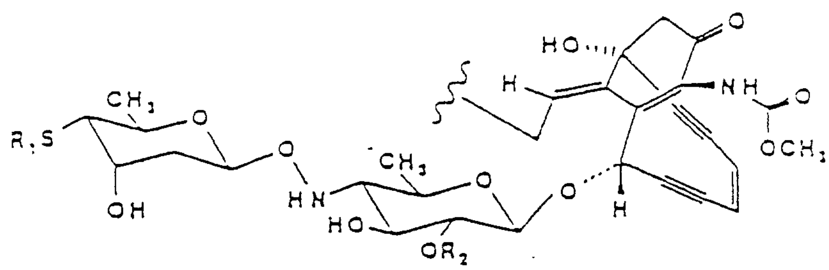

$R_1$ représente 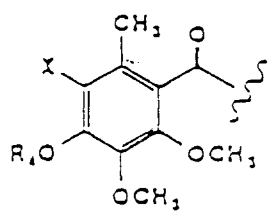 ;

$R_2$ représente 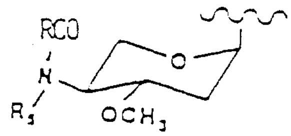 ;

$R_4$ représente 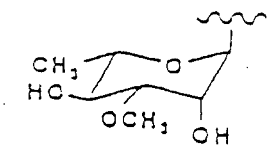 ou H ;

$R_5$ représente $CH_3$, $C_2H_5$ ou $(CH_3)_2CH$ ; X représente un atome d'iode ou de brome ; R représente un atome d'hydrogène, $CH_3$ ou un groupe aryle monosubstitué ;
et Sp ainsi que Q sont tels que définis.

**3.** Conjugué véhicule-médicament de formule :

$$\begin{array}{l} Tu\text{-}(Z\text{-}Sp\text{-}SS\text{-}W)_m \\ \;| \\ (Y)_{n-m} \end{array}$$

préparé à partir d'un composé de formule $CH_3SSS$-W, $CH_3SSS$-W étant un dérivé N-acylé d'un antibiotique antitumoral dénommé LL-E33288, $\alpha_1$-I, $\alpha_2$-Br, $\alpha_2$-I, $\beta_1$-Br, $\beta_1$-I, $\gamma_1$-Br, $\gamma_1$-I, $\delta_1$-I, BBM-1675, FR-900405, FR-900406, PD114759, PD115028, CL-1577A, CL-1577B, CL-1577D, CL-1577E ou CL-1724, W étant tel que décrit ci-dessus à la revendication 1, en faisant réagir $CH_3SSS$-W avec un composé de formule générale Q-Sp-SH, Sp représentant un groupe divalent ou trivalent en $C_1$ à $C_{18}$ à chaîne linéaire ou ramifiée, aryle ou hétéroaryle divalent ou trivalent, cycloalkyle ou hétérocycloalkyle en $C_3$-$C_{18}$ divalent ou trivalent, aryl- ou hétéroaryl-(alkyle en $C_1$-$C_{18}$) divalent ou trivalent, cycloalkyl- ou hétérocycloalkyl-(alkyle en $C_1$-$C_{18}$) divalent ou trivalent, ou alkyle en $C_2$-$C_{18}$ insaturé divalent ou trivalent, où si Sp représente un groupe trivalent, il peut être en outre substitué avec des groupes amino, alkylamino, arylamino, hétéroarylamino, carboxyle, alkoxy inférieurs, hydroxy, thiol ou alkylthio inférieurs, à condition que lorsque $CH_3$-SSS-W est l'antibiotique antitumoral de base LL-E33288 $\alpha_1$-Br, $\alpha_1$-I, $\alpha_2$-Br, $\alpha_2$-I, $\beta_1$-Br, $\beta_1$-I, $\gamma_1$-Br, $\gamma_1$-I, $\delta_1$-I, BBM-1675, FR-900405, FR-900406, PD114759, PD115028, CL-1577A, CL-1577B, CL-1577D, CL-1577E ou CL-1724 lui-même, Sp ne puisse alors pas être divalent ; et Q représente ou peut être ultérieurement converti en un atome d'halogène, un groupe amino, alkylamino, carboxyle, carboxaldéhyde, hydroxy, thiol, $\alpha$-haloacétyloxy, (alkyl inférieur)-dicarboxyle, -$CONHNH_2$, -$NHCONHNH_2$, -$NHCSNHNH_2$, -ONH2, -$CON_3$,

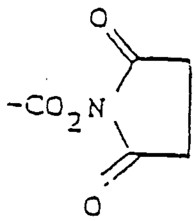 , 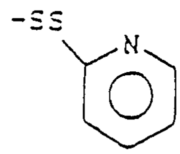 , 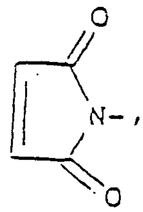 , 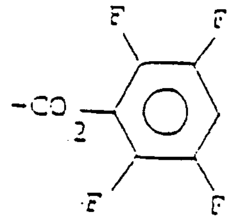 ,

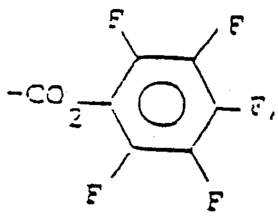 , 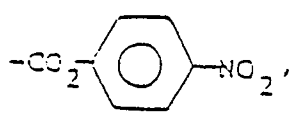 , 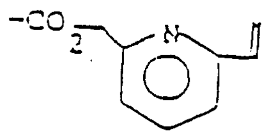 , 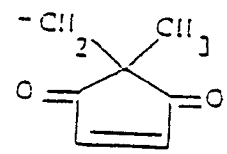

pour produire un intermédiaire de formule Q-Sp-SS-W, dans laquelle Q, Sp et W sont tels que définis ci-dessus,

en faisant réagir Q-Sp-SS-W avec une molécule de formule Tu-$(Y)_n$ dans laquelle le véhicule Tu est défini comme étant un anticorps mono- ou polyclonal, ses fragments, ses produits analogues obtenus par manipulation chimique ou génétique, ou des facteurs de croissance ou des stéroïdes ; Y représente un groupe amino, carboxy ou thiol de chaîne latérale d'une protéine, un groupe aldéhyde dérivé de résidus d'hydrate de carbone de glycoprotéine, ou un groupe amidoalkylthio ; et n représente un entier de 1 à 100, pour produire un composé de formule :

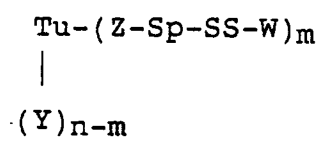

dans laquelle Tu, Y, Sp, W et n sont tels que définis ci-dessus, et Z est formé par réaction covalente des groupes Q et Y directement ou après réduction ultérieure, et Z représente -CONH-, -CONHN=CH-, -CONHNHCH$_2$-, -NHCONHN=CH-, -NHCONHNHCH$_2$-, -NHCSNHN=CH-, -NHCSNHNHCH$_2$-, -ON=CH-, -NH-, -NHCH$_2$-, -N=CH-, -CO$_2$-, -NHCH$_2$CH$_2$-, -SS-,

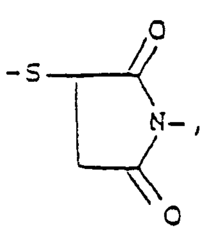 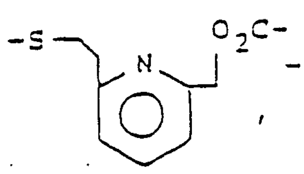 , 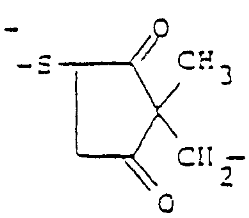 ou 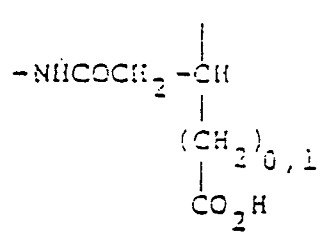

et m varie de 0,1 à 15.

**4.** Conjugué protéine-médicament selon la revendication 3, de formule :

$$\begin{array}{l} Tu-(Z-Sp-SS-W)_m \\ | \\ (Y)_{n-m} \end{array}$$

préparé à partir de la classe de dérivés N-acylés des antibiotiques antitumoraux dénommés LL-E33288 ($CH_3SSS$-W) :

en déplaçant le groupe dithiométhyle à l'aide d'un composé de formule Q-Sp-SH dans laquelle Sp représente des groupes divalents ou trivalents en $C_2$ à $C_{10}$ à chaîne linéaire ou ramifiée, et des groupes aryl- ou hétéroaryl-(alkyle en $C_2$-$C_5$) divalents ou trivalents, où si Sp représente un groupe trivalent, il peut être en outre substitué avec des groupes amino, hétéroarylamino, hydroxy ou thiol ; et Q est ou peut être ultérieurement converti en un groupe carboxyle, (alkyl inférieur)-dicarboxylanhydride, $-CO_2Su$, $-CONHNH_2$, ou

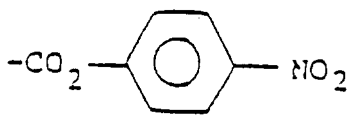

pour produire un intermédiaire de formule générale Q-Sp-SS-W dans laquelle Q, Sp et W sont tels que définis ci-dessus,
en faisant réagir Q-Sp-SS-W avec une molécule de formule $Tu-(Y)_n$ dans laquelle Tu représente un anticorps monoclonal ayant une réactivité préférentielle avec un antigène associé à une tumeur humaine, Y représente un groupe amino de chaîne latérale présent sur l'anticorps, ou un groupe aldéhyde généré par oxydation des groupes hydrate de carbone de l'anticorps, et n est un entier de 1 à 100, pour produire un composé de formule :

$$\begin{array}{l} Tu-(Z-Sp-SS-W)_m \\ | \\ (Y)_{n-m} \end{array}$$

dans laquelle Tu, Y, Sp, W et n sont tels que définis ci-dessus, et Z est formé par réaction covalente des groupes Q et Y directement ou après réduction ultérieure, et Z représente -CONH-, -CONHN=CH-, $-CONHNHCH_2$-, ou

$$\begin{array}{r} | \\ -NHCOCH_2-CH \\ | \\ (CH_2)_{0,1} \\ | \\ CH_2H \end{array}$$

et m varie de 0,1 à 15.

5. Procédé de production d'un disulfure substitué de formule Q-Sp-SS-W à partir d'un composé de formule $CH_3SSS$-W qui est un dérivé N-acylé d'un antibiotique antitumoral dénommé LL-E33288, $\alpha_1$-I, $\alpha_2$-Br, $\alpha_2$-I, $\beta_1$-Br, $\beta_1$-I, $\gamma_1$-Br, $\gamma_1$-I, $\delta_1$-I, BBM-1675, FR-900405, FR-900406, PD114759, PD115028, CL-1577A, CL-1577B, CL-1577D, CL-1577E ou CL-1724, dans lequel :

W représente

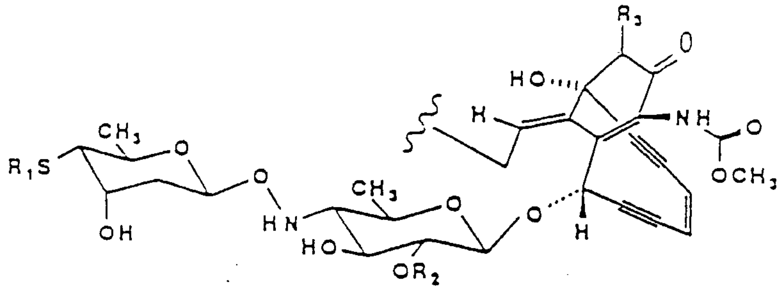

$R_1$ représente

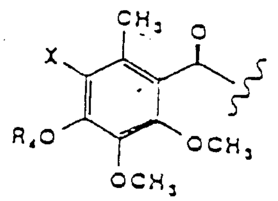

ou $CH_3$ ;

$R_2$ représente

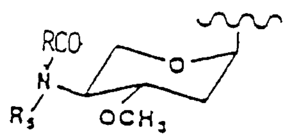

;

$R_3$ représente

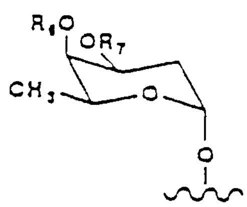

ou H ;

$R_4$ représente

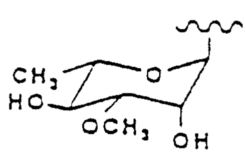

ou H ;

$R_6$ ou $R_7$ représente H ou 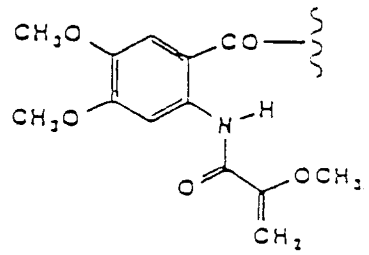 ;

$R_5$ représente $CH_3$, $C_2H_5$ ou $(CH_3)_2CH$ ;
X représente un atome d'iode ou de brome ;
R représente un atome d'hydrogène ou un groupe alkylène ou alkyle en $C_1$-$C_{10}$ ramifié ou non ramifié, un groupe aryle ou hétéroaryle, ou un groupe aryl-(alkyle en $C_1$-$C_5$) ou hétéroaryl-(alkyle en $C_1$-$C_5$), tous étant éventuellement substitués avec un ou plusieurs groupes hydroxy, amino, carboxy, halo, nitro, alkoxy inférieurs en $C_1$-$C_3$ ou thioalkoxy inférieurs en $C_1$-$C_5$ ; Sp représente des groupes divalents ou trivalents en $C_1$ à $C_{18}$ à chaîne linéaire ou ramifiée, des groupes aryle ou hétéroaryle divalents ou trivalents, des groupes cycloalkyle ou hétérocycloalkyle en $C_3$-$C_{18}$ divalents ou trivalents, des groupes aryl- ou hétéroaryl-(alkyle en $C_1$-$C_{18}$) divalents ou trivalents, des groupes cycloalkyl- ou hétérocycloalkyl-(alkyle en $C_1$-$C_{18}$) divalents ou trivalents, ou des groupes alkyle insaturés en $C_2$-$C_{18}$ divalents ou trivalents, où si Sp représente un groupe trivalent, il peut être en outre substitué avec des groupes amino, alkylamino, arylamino, hétéroarylamino, carboxyle, alkoxy inférieurs, hydroxy, thiol ou alkylthio inférieurs ; Q représente un atome d'hydrogène, d'halogène, un groupe amino, alkylamino, dialkylamino, arylamino, hétéroarylamino, pipéridino, pyrrolidino, pipérazino, carboxyle, nitrophényloxycarbonyle, carboxaldéhyde, alkoxy inférieur, hydroxy, thiol, (alkyl inférieur)-dicarboxyle, -$CONHNH_2$, -$NHCONHNH_2$, -$CSNHNH_2$, -$NHCSNHNH_2$ ou -$ONH_2$ ; à condition que lorsque Sp représente un groupe éthylidyne, Q ne puisse pas représenter un atome d'hydrogène ; et lorsque $CH_3SSS$-W est LL-E33288, $\alpha_1$-I, $\alpha_2$-Br, $\alpha_2$-I, $\beta_1$-Br, $\beta_1$-I, $\gamma_1$-Br, $\gamma_1$-I, $\delta_1$-I, BBM-1675, FR-900405, FR-900406, PD114759, PD115028, CL-1577A, CL-1577B, CL-1577D, CL-1577E ou CL-1724, et que Sp représente un groupe divalent, Q ne puisse pas représenter un atome d'hydrogène, d'halogène, un groupe amino, alkylamino, dialkylamino, arylamino, hétéroarylamino, pipéridino, pyrrolidino, pipérazino ou carboxyle ; selon lequel :

on fait réagir un antibiotique antitumoral de type trisulfure de méthyle choisi parmi ceux mentionnés ci-dessus, avec un mercaptan substitué de manière appropriée, dans de l'acétonitrile ou un mélange d'acétonitrile et de tétrahydrofuranne, ou d'acétonitrile et de diméthylformamide, à une température de -20° à environ +40 °C, pendant une heure à 6 jours, de préférence en présence d'un équivalent d'une base de type amine tertiaire ou d'un mélange d'un équivalent d'une base de type amine tertiaire et d'un équivalent d'un acide carboxylique organique.

**6.** Procédé de préparation des dérivés ciblés :

$$\begin{array}{l} Tu\text{-}(Z\text{-}Sp\text{-}SS\text{-}W)_m \\ \,| \\ (Y)_{n-m} \end{array}$$

de composés de formule $CH_3SSS$-W dans laquelle $CH_3SSS$-W est un dérivé N-acylé d'un antibiotique antitumoral de type LL-E33288, $\alpha_1$-I, $\alpha_2$-Br, $\alpha_2$-I, $\beta_1$-Br, $\beta_1$-I, $\gamma_1$-Br, $\gamma_1$-I, $\delta_1$-I, BBM-1675, FR-900405, FR-900406, PD114759, PD115028, CL-1577A, CL-1577B, CL-1577D, CL-1577E ou CL-1724, selon lequel :

on fait réagir $CH_3SSS$-W avec un composé de formule Q-Sp-SH dans laquelle Sp représente un groupe divalent ou trivalent en $C_1$-$C_{18}$ à chaîne linéaire ou ramifiée, un groupe aryle ou hétéroaryle divalent ou trivalent, un groupe cycloalkyle ou hétérocycloalkyle en $C_3$-$C_{18}$ divalent ou trivalent, un groupe aryl- ou hetéroaryl-(alk-

yle en $C_1$-$C_{18}$) divalent ou trivalent, un groupe cycloalkyl- ou hétérocycloalkyl-(alkyle en $C_1$-$C_{18}$) divalent ou trivalent ou un groupe alkyle en $C_2$-$C_{18}$ insaturé divalent ou trivalent, où si Sp représente un groupe trivalent, il peut être en outre substitué avec des groupes amino, alkylamino, arylamino, hétéroarylamino, carboxyle, alkoxy inférieurs, hydroxy, thiol ou alkylthio inférieurs, à condition que lorsque $CH_3$-SSS-W est l'antibiotique antitumoral de base LL-E33288 , $\alpha_1$-I, $\alpha_2$-Br, $\alpha_2$-I, $\beta_1$-Br, $\beta_1$-I, $\gamma_1$-Br, $\gamma_1$-I, $\delta_1$-I, BBM-1675, FR-900405, FR-900406, PD114759, PD115028, CL-1577A, CL-1577B, CL-1577D, CL-1577E ou CL-1724 lui-même, Sp ne puisse pas être divalent ; et Q représente un atome d'halogène, un groupe amino, alkylamino, carboxyle, carboxaldéhyde, hydroxy, (alkyl inférieur)-dicarboxyl anhydride, $-CONHNH_2$, $-NHCONHNH_2$, $-NHCSNHNH_2$, $-ONH_2$ ou

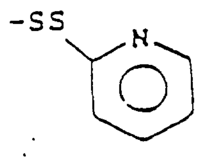

dans de l'acétonitrile en présence d'un équivalent de triéthylamine ou d'un équivalent de triéthylamine et d'un équivalent d'acide acétique à -10° jusqu'à -30 °C pendant 1 à 48 heures,

on isole l'intermédiaire de formule Q-Sp-SS-W dans laquelle Q, Sp et W sont tels que définis ci-dessus, puis on fait réagir le composé de formule Q-Sp-SS-W dans laquelle Sp et W sont tels que définis ci-dessus, et Q représente un atome d'halogène, un groupe amino, alkylamino, carboxyle, carboxaldéhyde, hydroxy, ou anhydride d'acide (alkyl inférieur)-dicarboxylique, avec une molécule de formule Tu-$(Y)_n$ dans laquelle Tu représente un anticorps mono ou polyclonal, ses fragments, ses analogues obtenus par manipulation chimique ou génétique, ou des facteurs de croissance ou des stéroïdes ; Y représente une fonction amino ou carboxy de chaîne latérale; n varie de 1 à 100, dans un tampon aqueux à un pH de 6,5 à 9 à une température de 4° à 40 °C, directement ou en présence d'un carbodiimide soluble dans l'eau, pour générer le composé :

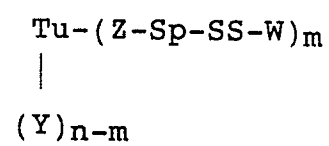

dans laquelle Tu, Sp, W, n et Y sont tels que définis ci-dessus, m varie de 1 à 15, et Z est formé par réaction covalente des groupes Q et Y, et il représente -CONH-, -NH-,

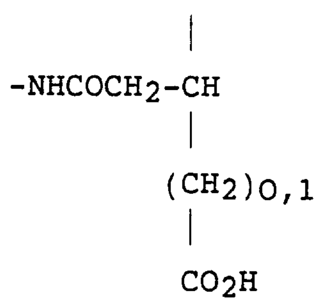

-N=CH- ou $-CO_2$- ou

on fait réagir le composé de formule Q-Sp-SS-W dans laquelle Sp et W sont tels que définis ci-dessus et Q représente un groupe acide carboxylique, avec du N-hydroxysuccinimide, du 2,3,5,6-tétrafluorophénol, du pentafluorophénol ou du 4-nitrophénol, en présence d'un agent d'activation de groupe carboxyle tel qu'un carbodiimide, afin de générer un composé de formule Q-Sp-SS-W dans laquelle Sp et W sont tels que définis ci-dessus, et Q représente :

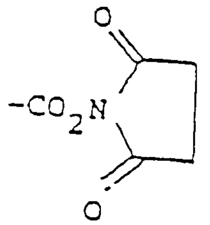 , 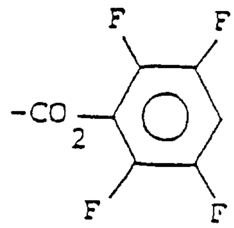 , 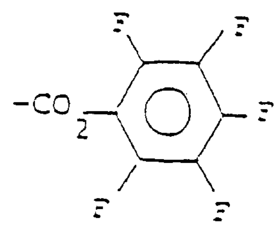 ,

ou 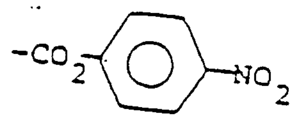

avec une molécule de formule Tu-$(Y)_n$ dans laquelle Tu et n sont tels que définis ci-dessus, et Y est un groupe amino de chaîne latérale, dans une solution tampon aqueuse à un pH de 6,5 à 9, à une température de 4° à 40 °C inclus, pour générer des composés de formule :

$$\begin{array}{l} Tu\text{-}(Z\text{-}Sp\text{-}SS\text{-}W)_m \\ \,| \\ (Y)_{n-m} \end{array}$$

dans laquelle Tu, Sp, Y et n sont tels que définis ci-dessus, m varie de 1 à 15, et Z est formé par réaction covalente entre les groupes Q et Y, et il est défini comme groupe -CONH-, ou

on fait réagir un composé de formule Q-Sp-SS-W dans laquelle Sp et W sont tels que définis ci-dessus, et Q représente -$CONHNH_2$, avec de l'acide nitreux dans de l'acétonitrile aqueux, pour générer un composé de formule Q-Sp-SS-W dans laquelle Sp et W sont tels que définis ci-dessus, et Q représente -$CON_3$, avec un composé de formule Tu-(Y)n dans laquelle Tu, Y et n sont tels que définis ci-dessus, pour produire un composé de formule :

$$\begin{array}{l} Tu\text{-}(Z\text{-}SP\text{-}SS\text{-}W)_m \\ \,| \\ (Y)_{n-m} \end{array}$$

dans laquelle Tu, Z, Sp, W, m, Y et n sont tels que définis ci-dessus ; ou

on fait réagir un composé de formule Q-Sp-SS-W dans laquelle Sp et W sont tels que définis ci-dessus, et Q représente un groupe hydroxy, avec un anhydride d'acide alpha-haloacétique, pour produire un composé dans lequel Q représente un groupe $\alpha$-haloacétyloxy, et on fait réagir le composé $\alpha$-haloacétyloxy-Sp-SS-W ou un composé de formule Q-Sp-SS-W, Sp et W étant tels que définis ci-dessus, et Q représente :

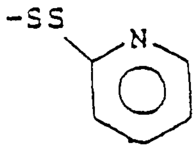 , 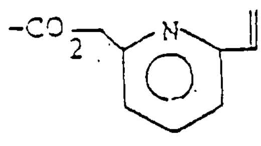 ou 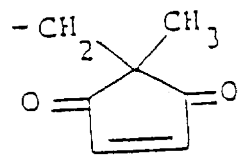

avec une molécule de formule Tu-$(Y)_n$ dans laquelle Tu est tel que défini ci-dessus, Y représente un groupe thiol de chaîne latérale d'une protéine, ou un groupe amidoalkylthio introduit sur un groupe amine de Tu en employant des réactifs tels que l'ester de l'acide 3-(2-dithiopyridyl)propionique dérivé de l'hydroxysuccinimide suivi d'une réduction avec un agent tel que du dithiothréitol, ou un groupe amidoalkylthio introduit sur un groupe amine de Tu en employant du 2-iminothiolanne, et n varie de 1 à 10, dans des conditions aqueuses tamponnées à un pH de 4,5 à 7, à une température de 4° à 40 °C inclus, pour produire un composé de formule :

$$\begin{array}{l}\text{Tu-(Z-SP-SS-W)}_m\\ \ \ |\\ (\text{Y})_{n-m}\end{array}$$

dans laquelle Tu, Sp, W et n sont tels que définis ci-dessus, et Z est formé par réaction covalente des groupes Q et Y, et Z représente :
-SS-,

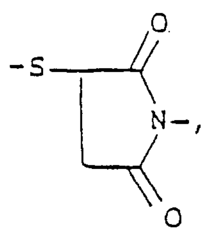 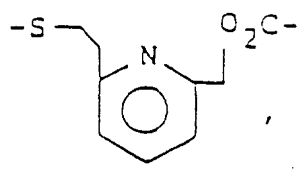 , ou 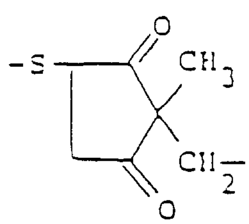

et n varie de 0,1 à 10 ; ou
on fait réagir un composé de formule Q-Sp-SS-W dans laquelle Sp et W sont tels que définis ci-dessus, et Q représente $-NH_2$, $-CONHNH_2$, $-NHCONHNH_2$, $-NHCSNHNH_2$ ou $-ONH_2$, avec une molécule de formule Tu-$(Y)_n$ dans laquelle Tu est tel que défini ci-dessus, et Y représente un groupe aldéhyde généré à partir de groupes hydrate de carbone présents sur Tu, par oxydation en présence d'un périodate alcalino-terreux, dans un tampon aqueux à un pH de 4,0 à 6,5 à une température de 4° à 40 °C inclus, et n varie de 1 à 20, afin de générer un composé de formule :

$$\begin{array}{l}\text{Tu-(Z-Sp-SS-W)}_m\\ \ \ |\\ (\text{Y})_{n-m}\end{array}$$

dans laquelle Tu, Sp, W, Y et n sont tels que définis ci-dessus, et Z est formé par réaction covalente de Q et Y, et il représente -ON=CH-, -N=CH-, -CONHN=CH-, -NHCONHN=CH- ou -NHCSNHN=CH-, et m varie de 0,1 à

15 ; ou on traite le composé immédiatement mentionné ci-dessus, de formule :

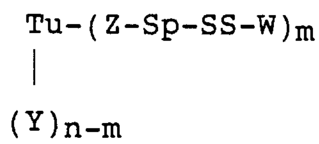

dans laquelle Tu, Z, Sp, W, Y, n et m sont tels qu'immédiatement définis ci-dessus, avec de l'acétylhydrazine ou de la tyrosine hydrazine dans un tampon aqueux à un pH de 4,0 à 6,5 et à une température de 4° à 40 °C inclus, pour générer un composé de formule :

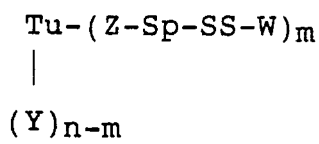

dans laquelle Tu, Z, Sp, W, n et m sont tels qu'immédiatement définis ci-dessus, et Y représente -$CH=NNHCOCH_3$ ou

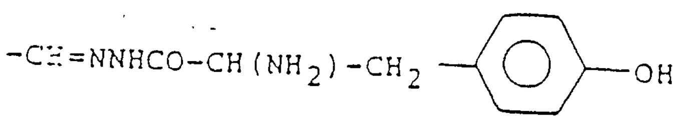

et
on fait réagir ce composé avec du cyanoborohydrure de sodium ou du borohydrure de sodium dans un tampon aqueux à un pH de 4,0 à 6,5, à une température de 4° à 40 °C inclus, pour générer un composé de formule :

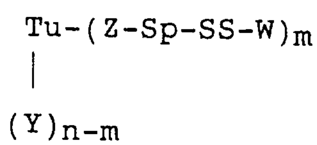

dans laquelle Tu, Sp, W, m et n sont tels que définis ci-dessus, Z représente -$NH-CH_2$-, -$CONHNHCH_2$-, -$NHCONHNHCH_2$- ou -$NHCSNHNHCH_2$-, et Y représente -$CH_2NHNHCOCH_3$ ou

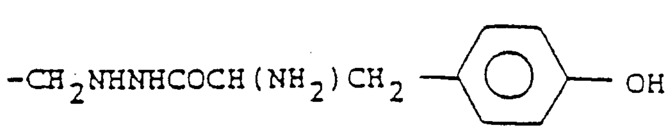

**7.** Produit contenant un analogue de disulfure substitué de formule Q-Sp-SS-W, préparé à partir d'un composé de formule $CH_3SSS$-W qui est un dérivé N-acylé de LL-E33288, $\alpha_1$-I, $\alpha_2$-Br, $\alpha_2$-I, $\beta_1$-Br, $\beta_1$-I, $\gamma_1$-Br, $\gamma_1$-I, $\delta_1$-I, BBM-1675, FR-900405, FR-900406, PD114759, PD115028, CL-1577A, CL-1577B, CL-1577D, CL-1577E ou CL-1724, destiné à être utilisé dans la thérapie d'infections bactériennes, dans lequel :

W représente 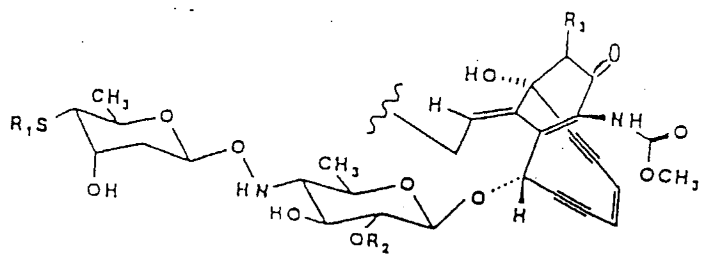

$R_1$ représente 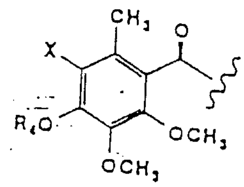 ou $CH_3$ ;

$R_2$ représente 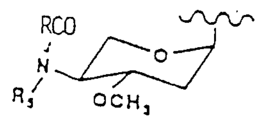 ;

$R_3$ représente 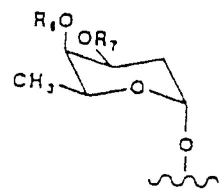 ou H ;

$R_4$ représente 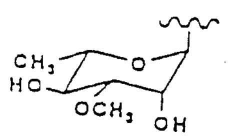 ou H ;

$R_6$ ou $R_7$ représente H ou 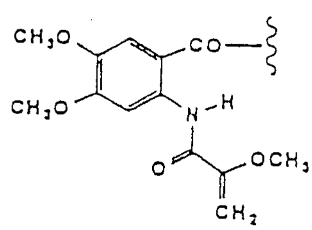

$R_5$ représente $CH_3$, $C_2H_5$ ou $(CH_3)_2CH$ ;
X représente un atome d'iode ou de brome ;

R représente un atome d'hydrogène ou un groupe alkyle en $C_1$-$C_{10}$ ou alkylène en $C_1$-$C_{10}$ ramifié ou non ramifié, un groupe aryle ou hétéroaryle, ou un groupe aryl-(alkyle en $C_1$-$C_5$) ou hétéroaryl-(alkyle en $C_1$-$C_5$), tous étant éventuellement substitués avec un ou plusieurs groupes hydroxy, amino, carboxyle, halo, nitro, alkoxy inférieurs en $C_1$-$C_3$ ou thioalkoxy inférieurs en $C_1$-$C_5$ ; Sp représente des groupes en $C_1$-$C_{18}$ divalents ou trivalents à chaîne linéaire ou ramifiée, des groupes aryle ou hétéroaryle divalents ou trivalents, des groupes cycloalkyle ou hétérocycloalkyle en $C_3$-$C_{18}$ divalents ou trivalents, des groupes aryl- ou hétéroaryl-(alkyle en $C_1$-$C_{18}$) divalents ou trivalents, des groupes cycloalkyl- ou hétérocycloalkyl-(alkyle en $C_1$-$C_{18}$) divalents ou trivalents ou des groupes alkyle en $C_2$-$C_{18}$ insaturés divalents ou trivalents, où si Sp représente un groupe trivalent, il peut être en outre substitué avec des groupes amino, alkylamino, arylamino, hétéroarylamino, carboxyle, alkoxy inférieurs, hydroxy, thiol ou alkylthio inférieurs ; Q représente un atome d'hydrogène, d'halogène, un groupe amino, alkylamino, dialkylamino, arylamino, hétéroarylamino, pipéridino, pyrrolidino, pipérazino, carboxyle, nitrophényloxycarbonyle, carboxaldéhyde, alkoxy inférieur, hydroxy, thiol, (alkyl inférieur)-dicarboxyle, $-CONHNH_2$, $-NHCONHNH_2$, $-CSNHNH_2$, $-NHCSNHNH_2$ ou $-ONH_2$ ; à condition que lorsque Sp représente un groupe éthylidyne, Q ne puisse pas représenter un atome d'hydrogène, et que lorsque $CH_3SSS$-W représente LL-E33288, $\alpha_1$-I, $\alpha_2$-Br, $\alpha_2$-I, $\beta_1$-Br, $\beta_1$-I, $\gamma_1$-Br, $\gamma_1$-I, $\delta_1$-I, BBM-1675, FR-900405, FR-900406, PD114759, PD115028, CL-1577A, CL-1577B, CL-1577D, CL-1577E ou CL-1724 et que Sp représente un groupe divalent, Q ne puisse pas représenter un atome d'hydrogène, d'halogène, un groupe amino, alkylamino, dialkylamino, arylamino, hétéroarylamino, pipéridino, pyrrolidino, pipérazino ou carboxyle.

8. Produit contenant un disulfure substitué de formule Q-Sp-SS-W préparé à partir d'un composé de formule $CH_3SSS$-W qui est un dérivé N-acylé de LL-E33288, $\alpha_1$-I, $\alpha_2$-Br, $\alpha_2$-I, $\beta_1$-Br, $\beta_1$-I, $\gamma_1$-Br, $\gamma_1$-I, $\delta_1$-I, BBM-1675, FR-900405, FR-900406, PD114759, PD115028, CL-1577A, CL-1577B, CL-1577D, CL-1577E ou CL-1724, destiné à être utilisé dans une thérapie de tumeur, dans lequel :

W représente 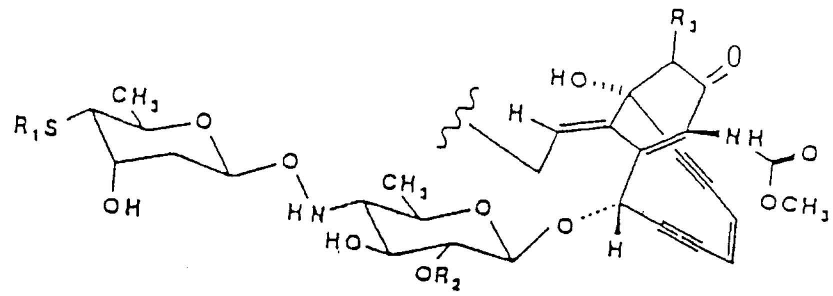

$R_1$ représente 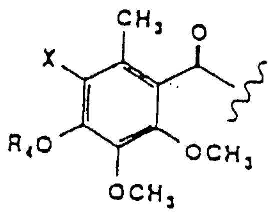 ou $CH_3$ ;

$R_2$ représente 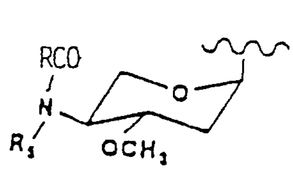 ;

$R_3$ représente 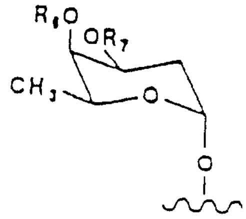 ou H ;

$R_4$ représente 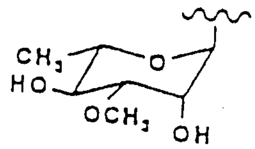 ou H ;

$R_6$ ou $R_7$ représente H ou 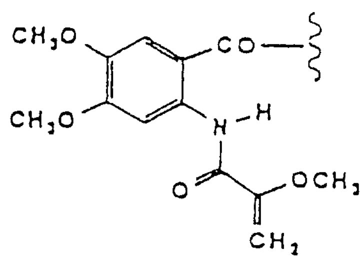 ;

$R_5$ représente $CH_3$, $C_2H_5$ ou $(CH_3)_2CH$ ;
X représente un atome d'iode ou de brome ;
R représente un atome d'hydrogène ou un groupe alkylène en $C_1$-$C_{10}$ ou alkyle en $C_1$-$C_{10}$ ramifié ou non ramifié, un groupe aryle ou hétéroaryle, ou un groupe aryl-(alkyle en $C_1$-$C_5$) ou hétéroaryl-(alkyle en $C_1$-$C_5$), tous étant éventuellement substitués avec un ou plusieurs groupes hydroxy, amino, carboxy, halo, nitro, alkoxy inférieurs en $C_1$-$C_3$ ou thioalkoxy inférieurs en $C_1$-$C_5$ ; Sp représente des groupes en $C_1$-$C_{18}$ divalents ou trivalents à chaîne linéaire ou ramifiée, des groupes aryle ou hétéroaryle divalents ou trivalents, des groupes cycloalkyle ou hétérocycloalkyle en $C_3$-$C_{18}$ divalents ou trivalents, des groupes aryl- ou hétéroaryl-(alkyle en $C_1$-$C_{18}$) divalents ou trivalents, des groupes cycloalkyl- ou hétérocycloalkyl-(alkyle en $C_1$-$C_{18}$) divalents ou trivalents, ou des groupes alkyle en $C_2$-$C_{18}$ insaturés divalents ou trivalents, où si Sp représente un groupe trivalent, il peut être en outre substitué avec des groupes amino, alkylamino, arylamino, hétéroarylamino, carboxyle, alkoxy inférieurs, hydroxy, thiol ou alkylthio inférieurs ; Q représente un atome d'hydrogène, d'halogène, un groupe amino, alkylamino, dialkylamino, arylamino, hétéroarylamino, pipéridino, pyrrolidino, pipérazino, carboxyle, nitrophényloxycarbonyle, carboxaldéhyde, alkoxy inférieur, hydroxy, thiol, (alkyl inférieur)-dicarboxyle, $-CONHNH_2$, $-NHCONHNH_2$, $-CSNHNH_2$, $-NHCSNHNH_2$ ou $-ONH_2$ ; à condition que lorsque Sp représente un groupe éthylidyne, Q ne puisse pas représenter un atome d'hydrogène ; et que lorsque $CH_3$-SSS-W représente LL-E33288, $\alpha_1$-I, $\alpha_2$-Br, $\alpha_2$-I, $\beta_1$-Br, $\beta_1$-I, $\gamma_1$-Br, $\gamma_1$-I, $\delta_1$-I, BBM-1675, FR-900405, FR-900406, PD114759, PD115028, CL-1577A, CL-1577B, CL-1577D, CL-1577E ou CL-1724, et que Sp est divalent, Q ne puisse pas représenter un atome d'hydrogène, d'halogène, un groupe amino, alkylamino, dialkylamino, arylamino, hétéroarylamino, pipéridino, pyrrolidino, pipérazino ou carboxyle.

**9.** Produit contenant un composé de formule :

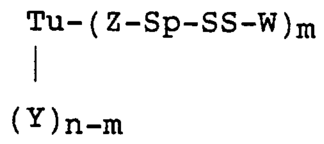

préparé à partir d'un composé de formule générale $CH_3SSS$-W dans laquelle $CH_3SSS$-W est un dérivé N-acylé d'un antibiotique antitumoral dénommé LL-E33288, $\alpha_1$-I, $\alpha_2$-Br, $\alpha_2$-I, $\beta_1$-Br, $\beta_1$-I, $\gamma_1$-Br, $\gamma_1$-I, $\delta_1$-I, BBM-1675, FR-900405, FR-900406, PD114759, PD115028, CL-1577A, CL-1577B, CL-1577D, CL-1577E ou CL-1724, selon un procédé comprenant les étapes consistant :

à faire réagir $CH_3SSS$-W avec un composé de formule Q-Sp-SH dans laquelle Sp représente un groupe en $C_1$-$C_{18}$ divalent ou trivalent à chaîne linéaire ou ramifiée, un groupe aryle ou hétéroaryle divalent ou trivalent, un groupe cycloalkyle ou hétérocycloalkyle en chaîne linéaire ou ramifiée en $C_3$-$C_{18}$, un groupe aryl- ou hétéroaryl-(alkyle en $C_1$-$C_{18}$) divalent ou trivalent, un groupe cycloalkyl- ou hétérocycloalkyl-(alkyle en $C_1$-$C_{18}$) divalent ou trivalent ou un groupe alkyle en $C_2$-$C_{18}$ insaturé divalent ou trivalent, où si Sp représente un groupe trivalent, il peut être en outre substitué avec des groupes amino, alkylamino, arylamino, hétéroarylamino, carboxyle, alkoxy inférieurs, hydroxy, thiol ou alkylthio inférieurs, à condition que lorsque $CH_3$-SSS-W est l'antibiotique antitumoral de base LL-E33288 $\alpha_1$-I, $\alpha_2$-Br, $\alpha_2$-I, $\beta_1$-Br, $\beta_1$-I, $\gamma_1$-Br, $\gamma_1$-I, $\delta_1$-I, BBM-1675, FR-900405, FR-900406, PD114759, PD115028, CL-1577A, CL-1577B, CL-1577D, CL-1577E ou CL-1724 lui-même, Sp ne puisse pas être divalent ; et Q représente ou peut être ultérieurement converti en un atome d'halogène, un groupe amino, alkylamino, carboxyle, carboxaldéhyde, hydroxy, thiol, $\alpha$-haloacétyloxy, (alkyl inférieur)dicarboxyle, $-CONHNH_2$, $-NHCONHNH_2$, $-NHCSNHNH_2$, $-ONH_2$, $-CON_3$,

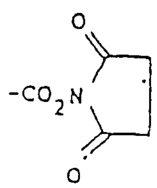 ; 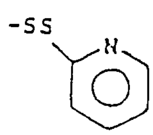 , 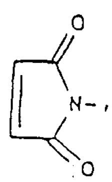 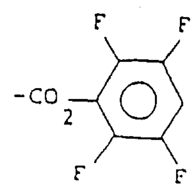 ,

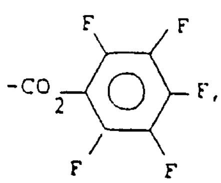 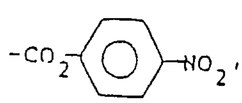 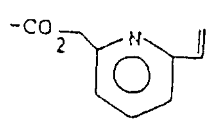 , 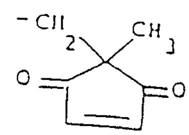

pour produire un intermédiaire de formule Q-Sp-SS-W dans laquelle Q, Sp et W sont tels que définis ci-dessus,

à faire réagir Q-Sp-SS-W avec une molécule de formule Tu—$(Y)_n$ dans laquelle Tu est défini comme anticorps mono- ou polyclonal, ses fragments, ses analogues obtenus par manipulation chimique ou génétique, des facteurs de croissance ou des stéroïdes ; Y représente un groupe amino, carboxy ou thiol de chaîne latérale d'une protéine, un groupe aldéhyde dérivé de groupes hydrate de carbone de glycoprotéine, ou un groupe amidoalkylthio ; et n est un entier de 1 à 100, pour produire un composé de formule :

Tu-(Z-Sp-SS-W)m
|
(Y)n-m

dans laquelle Tu, y, Sp, W et n sont tels que définis ci-dessus, et Z est formé par réaction covalente des groupes Q et Y directement ou après réduction ultérieure, et Z représente -CONH-, -CONHN=CH-, -CONHNHCH$_2$-, -NHCONHN=CH-, -NHCONHNHCH$_2$-, -NHCSNHN=CH-, -NHCSNHNHCH$_2$-, -ON=CH-, -NH-, -NHCH$_2$-, -N=CH-, -CO$_2$-, -NHCH$_2$CO$_2$-, -SS-,

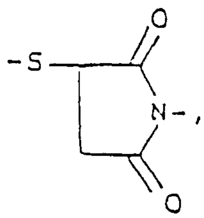
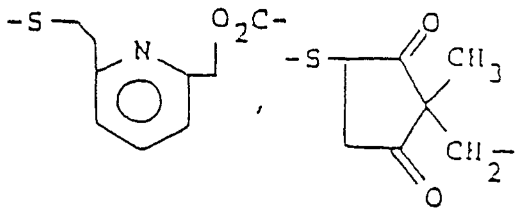
ou -NHCOCH$_2$-CH(-)-(CH$_2$)$_{0,1}$-CO$_2$H

et m varie de 0,1 à 15, pour une utilisation dans une thérapie de tumeur.

**10.** Produit d'un conjugué protéine-médicament de formule :

Tu-(Z-Sp-SS-W)m
|
(Y)n-m

préparé à partir de l'antibiotique antitumoral dénommé N-acétyl LL-E33288$\gamma_1^I$ (CH$_3$SSS-W), ayant :

a) un spectre ultraviolet tel qu'illustré sur la figure 1 ;
b) un spectre infrarouge tel qu'illustré sur la figure 2 ;
c) un spectre de résonance magnétique du proton tel qu'illustré sur la figure 3 ; et
d) un spectre de résonance magnétique nucléaire du carbone-13 tel qu'illustré sur la figure 4 ;

selon un procédé comprenant les étapes consistant :

à déplacer le groupe dithiométhyle avec un composé de formule Q-Sp-SH dans laquelle Sp représente des groupes en C$_2$-C$_5$ divalents ou trivalents à chaîne linéaire ou ramifiée ou des groupes aryl- ou hétéroaryl-(alkyle en C$_2$-C$_5$) divalents ou trivalents, où si Sp représente un groupe trivalent, il peut être en outre substitué avec des groupes amino, hétéroarylamino, hydroxy ou thiol ; et Q représente ou peut être ultérieurement converti en un groupe carboxyle, (alkyl inférieur)-dicarboxylanhydride, -CONHNH$_2$, ou

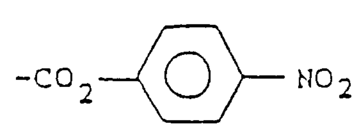

pour produire un intermédiaire de formule générale Q-Sp-SS-W dans laquelle Q, Sp et W sont tels que définis ci-dessus,

à faire réagir Q-Sp-SS-W avec une molécule de formule Tu-$(Y)_n$ dans laquelle Tu représente un anticorps monoclonal ayant une réactivité préférentielle avec un antigène associé à une tumeur humaine, Y représente un groupe amino de chaîne latérale présent sur l'anticorps, ou un groupe aldéhyde généré par oxydation des groupes hydrate de carbone de l'anticorps, et n est un entier de 1 à 100, pour produire un composé de formule :

```
Tu-(Z-Sp-SS-W)m
|
(Y)n-m
```

dans laquelle Tu, Y, Sp, W et n sont tels que définis ci-dessus, et Z est formé par réaction à covalence des groupes Q et Y, directement ou après réduction ultérieure, et Z représente -CONH-, -CONHNH=CH-, -$CONHNHCH_2$-, ou

```
               |
-NHCOCH2-CH
               |
             (CH2)0,1
               |
             CO2H
```

et m varie de 0,1 à 15, pour une utilisation dans une thérapie de tumeur afin de fournir, in vivo, le conjugué à un site antigénique.

**Revendications pour les Etats contractants suivants : ES, GR**

1. Un procédé pour la production d'un disulfure substitué de formule Q-Sp-SS-W, préparé à partir d'un composé de formule
   $CH_3SSS$-W qui est un dérivé N-acylé d'un antibiotique antitumoral dénommé LL-E33288, $\alpha_1$-I, $\alpha_2$-Br, $\alpha_2$-I, $\beta_1$-Br, $\beta_1$-I, $\gamma_1$-Br, $\gamma_1$-I, $\delta_1$-I, BBM-1675, FR-900405, FR-900406, PD114759, PD115028, CL-1577A CL-1577B, CL-1577D, CL-1577E ou CL-1724, dans lequel:

W représente 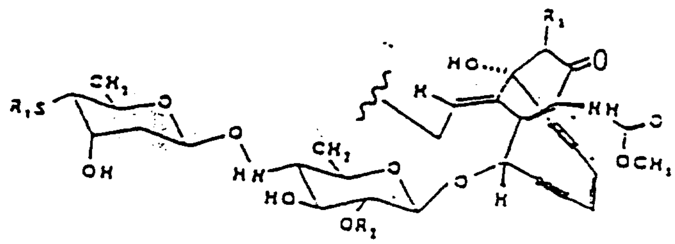

$R_1$ représente 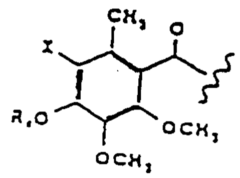 ou $CH_3$ ;

$R_2$ représente 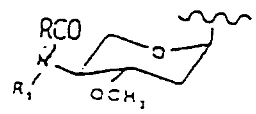 ;

$R_3$ représente 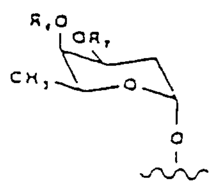 ou H ;

$R_4$ représente 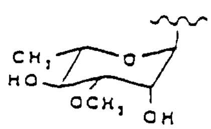 ou H ;

$R_6$ ou $R_7$ représente H ou 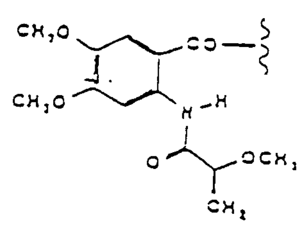

$R_5$ représente $CH_3$, $C_2H_5$ ou $(CH_3)_2CH$ ;
X représente un atome d'iode ou de brome ;
R représente un atome d'hydrogène, ou un groupe alkyle en $C_1$-$C_{10}$ ou alkylène en $C_1$-$C_{10}$ ramifié ou non ramifié, un groupe aryle ou hétéroaryle, ou un groupe aryl-(alkyle en $C_1$-$C_5$) ou hétéroaryl-(alkyle en $C_1$-$C_5$), tous étant éventuellement substitués avec un ou plusieurs groupes hydroxyle, amino, carboxy, halo, nitro, alkoxy inférieurs en $C_1$-$C_3$ ou thioalkoxy inférieurs en $C_1$-$C_5$ ; Sp représente des groupes divalents ou trivalents en $C_1$ à $C_{18}$ à chaîne linéaire ou ramifiée, des groupes aryle ou hétéroaryle divalents ou trivalents, des groupes cycloalkyle ou hétérocycloalkyle en $C_3$-$C_{18}$ divalents ou trivalents, des groupes aryl- ou hétéroaryl-(alkyle en $C_1$-$C_{18}$) divalents ou trivalents, des groupes cycloalkyl-e ou hétérocycloalkyl-(alkyle en $C_1$-$C_{18}$) divalents ou trivalents, ou des groupes alkyle insaturés en $C_2$-$C_{18}$ divalents ou trivalents, où si Sp représente un groupe trivalent, il peut être en outre substitué avec des groupes amino, alkylamino, arylamino, hétéroarylamino, carboxyle, alkoxy inférieurs, hydroxy, thiol ou alkylthio inférieurs ; Q représente un atome d'hydrogène, d'halogène, un groupe amino, alkylamino, dialkylamino, arylamino, hétéroarylamino, pipéridino, pyrrolidino, pipérazino, carboxyle, nitrophényloxycarbonyle, carboxaldéhyde, alkoxy inférieur, hydroxy, thiol, (alkyl inférieur)-dicarboxyle, $-CONHNH_2$, $-NHCONHNH_2$, $-CSNHNH_2$, $-NHCSNHNH_2$ ou $-ONH_2$ ; à condition que lorsque Sp représente un groupe éthylidyne, Q ne puisse pas être un atome d'hydrogène, et que lorsque $CH_3$-SSS-W est LL-33288, $\alpha_1$-I, $\alpha_2$-Br, $\alpha_2$-I, $\beta_1$-Br, $\beta_1$-I, $\gamma_1$-Br, $\gamma_1$-I, $\delta_1$-I, BBM-1675, FR-900405, FR-900406, PD114759, PD115028, CL-1577A, CL-1577B, CL-1577D, CL-1577E ou CL-1724 et que Sp est divalent, Q ne puisse pas être un atome d'hydrogène, d'halogène, un groupe amino, alkylamino, dialkylamino, arylamino, hétéroarylamino, pipéridino, pyrrolidino, pipérazino ou carboxyle; qui comprend la réaction d'un antibiotique antitumoral de type trisulfure de méthyle choisi parmi ceux mentionnés ci-dessus, avec un mercaptan substitué de manière appropriée, dans de l'acétonitrile ou un mélange d'acétonitrile et de tétrahydrofuranne, ou d'acétonitrile et de diméthylformamide, à une température de -20° à environ +40 °C, pendant une heure à 6 jours, de préférence en présence d'un équivalent d'une base de type amine tertiaire ou d'un mélange d'un équivalent d'une base de type amine tertiaire et d'un équivalent d'un acide carboxylique organique.

**2.** Procédé selon la revendication 1 dans lequel on prépare un disulfure substitué de formule Q-Sp-SS-W à partir d'un composé de formule $CH_3SSS$-W dans laquelle :

W représente 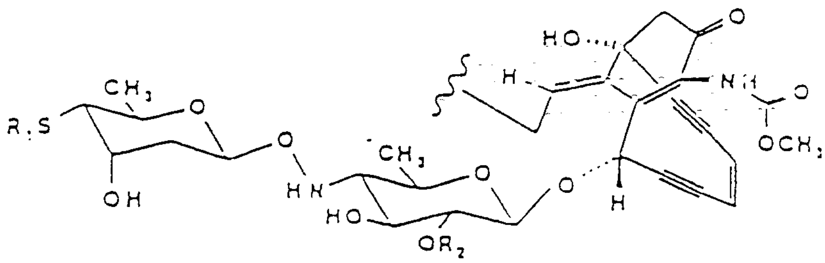

R1 représente 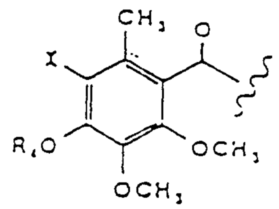 ;

R2 représente 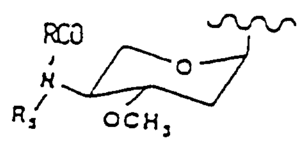 ;

R4 représente 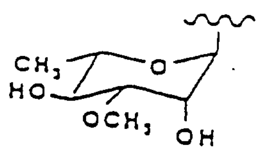 ou H ;

$R_5$ représente $CH_3$, $C_2H_5$ ou $(CH_3)_2CH$ ; X représente un atome d'iode ou de brome ; R représente un atome d'hydrogène, $CH_3$ ou un groupe aryle monosubstitué ;
et Sp ainsi que Q sont tels que définis.

**3.** Procédé de préparation des dérivés ciblés :

Tu-(Z-Sp-SS-W)$_m$
|
(Y)$_{n-m}$

de composés de formule $CH_3SSS$-W dans laquelle $CH_3SSS$-W est un dérivé N-acylé d'un antibiotique antitumoral de type LL-E33288, $\alpha_1$-I, $\alpha_2$-Br, $\alpha_2$-I, $\beta_1$-Br, $\beta_1$-I, $\gamma_1$-Br, $\gamma_1$-I, $\delta_1$-I, BBM-1675, FR-900405, FR-900406, PD114759, PD115028, CL-1577A, CL-1577B, CL-1577D, CL-1577E ou CL-1724, selon lequel :

on fait réagir $CH_3SSS$-W avec un composé de formule Q-Sp-SH dans laquelle Sp représente un groupe divalent ou trivalent en $C_1$-$C_{18}$ à chaîne linéaire ou ramifiée, un groupe aryle ou hétéroaryle divalent ou trivalent, un groupe cycloalkyle ou hétérocycloalkyle en $C_3$-$C_{18}$ divalent ou trivalent, un groupe aryl- ou hetéroaryl-(alkyle en $C_1$-$C_{18}$) divalent ou trivalent, un groupe cycloalkyl- ou hétérocycloalkyl-(alkyle en $C_1$-$C_{18}$) divalent ou trivalent ou un groupe alkyle en $C_2$-$C_{18}$ insaturé divalent ou trivalent, où si Sp représente un groupe trivalent, il peut être en outre substitué avec des groupes amino , alkylamino, arylamino, hétéroarylamino, carboxyle, alkoxy inférieurs, hydroxy, thiol ou alkylthio inférieurs, à condition que lorsque $CH_3$-SSS-W est l'antibiotique antitumoral de base LL-E33288 , $\alpha_1$-I, $\alpha_2$-Br, $\alpha_2$-I, $\beta_1$-Br, $\beta_1$-I, $\gamma_1$-Br, $\gamma_1$-I, $\delta_1$-I, BBM-1675, FR-900405, FR-900406, PD114759, PD115028, CL-1577A, CL-1577B, CL-1577D, CL-1577E ou CL-1724 lui-même, Sp ne puisse pas être divalent ; et Q représente un atome d'halogène, un groupe amino, alkylamino, carboxyle, carboxaldéhyde, hydroxy, (alkyl inférieur)-dicarboxyl anhydride, -$CONHNH_2$, -$NHCONHNH_2$, -$NHCSNHNH_2$, -$ONH_2$ ou

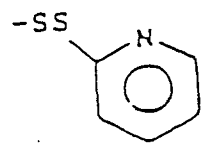

dans de l'acétonitrile en présence d'un équivalent de triéthylamine ou d'un équivalent de triéthylamine et d'un équivalent d'acide acétique à -10° jusqu'à -30 °C pendant 1 à 48 heures,
on isole l'intermédiaire de formule Q-Sp-SS-W dans laquelle Q, Sp et W sont tels que définis ci-dessus, puis on fait réagir le composé de formule Q-Sp-SS-W dans laquelle Sp et W sont tels que définis ci-dessus, et Q représente un atome d'halogène, un groupe amino, alkylamino, carboxyle, carboxaldéhyde, hydroxy, ou anhydride d'acide (alkyl inférieur)-dicarboxylique, avec une molécule de formule Tu-(Y)$_n$ dans laquelle Tu représente un anticorps mono ou polyclonal, ses fragments, ses analogues obtenus par manipulation chimique oui génétique, ou des facteurs de croissance ou des stéroïdes ; Y représente une fonction amino ou carboxy de chaîne latérale; n varie de 1 à 100, dans un tampon aqueux à un pH de 6,5 à 9 à une température de 4° à 40 °C, directement ou en présence d'un carbodiimide soluble dans l'eau, pour générer le composé :

Tu-(Z-Sp-SS-W)$_m$
|
(Y)$_{n-m}$

dans laquelle Tu, Sp, W, n et Y sont tels que définis ci-dessus, m varie de 1 à 15, et Z est formé par réaction covalente des groupes Q et Y, et il représente -CONH-, -NH-,

$$
\begin{array}{l}
\qquad\qquad\quad | \\
-NHCOCH_2-CH \\
\qquad\qquad\quad | \\
\qquad\qquad\quad (CH_2)_{0,1} \\
\qquad\qquad\quad | \\
\qquad\qquad\quad CO_2H
\end{array}
$$

-N=CH- ou $-CO_2-$ ou

on fait réagir le composé de formule Q-Sp-SS-W dans laquelle Sp et W sont tels que définis ci-dessus et Q représente un groupe acide carboxylique, avec du N-hydroxysuccinimide, du 2,3,5,6-tétrafluorophénol, du pentafluorophénol ou du 4-nitrophénol, en présence d'un agent d'activation de groupe carboxyle tel qu'un carbodiimide, afin de générer un composé de formule Q-Sp-SS-W dans laquelle Sp et W sont tels que définis ci-dessus, et Q représente :

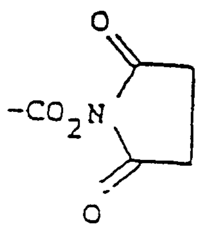 , 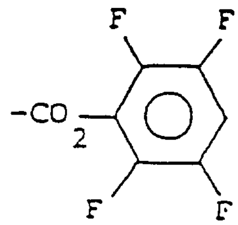 , 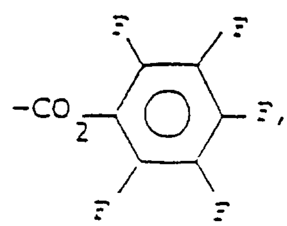 ,

ou 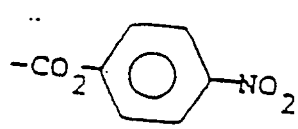

avec une molécule de formule $Tu-(Y)_n$ dans laquelle Tu et n sont tels que définis ci-dessus, et Y est un groupe amino de chaîne latérale, dans une solution tampon aqueuse à un pH de 6,5 à 9, à une température de 4° à 40 °C inclus, pour générer des composés de formule :

$$
\begin{array}{l}
Tu-(Z-Sp-SS-W)_m \\
\;| \\
(Y)_{n-m}
\end{array}
$$

dans laquelle Tu, Sp, Y et n sont tels que définis ci-dessus, m varie de 1 à 15, et Z est formé par réaction covalente entre les groupes Q et Y, et il est défini comme groupe -CONH-, ou

on fait réagir un composé de formule Q-Sp-SS-W dans laquelle Sp et W sont tels que définis ci-dessus, et Q représente $-CONHNH_2$, avec de l'acide nitreux dans de l'acétonitrile aqueux, pour générer un composé de formule Q-Sp-SS-W dans laquelle Sp et W sont tels que définis ci-dessus, et Q représente $-CON_3$, avec un composé de formule $Tu-(Y)_n$ dans laquelle Tu, Y et n sont tels que définis ci-dessus, pour produire un composé de formule :

$$\begin{array}{l} Tu-(Z-SP-SS-W)_m \\ | \\ (Y)_{n-m} \end{array}$$

dans laquelle Tu, Z, Sp, W, m, Y et n sont tels que définis ci-dessus ; ou

on fait réagir un composé de formule Q-Sp-SS-W dans laquelle Sp et W sont tels que définis ci-dessus, et Q représente un groupe hydroxy, avec un anhydride d'acide alpha-haloacétique, pour produire un composé dans lequel Q représente un groupe $\alpha$-haloacétyloxy, et on fait réagir le composé $\alpha$-haloacétyloxy-Sp-SS-W ou un composé de formule Q-Sp-SS-W, Sp et W étant tels que définis ci-dessus, et Q représente :

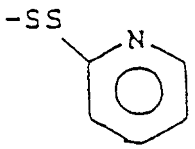 , 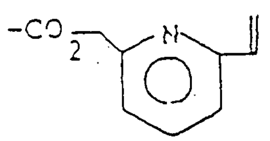 ou 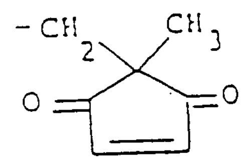

avec une molécule de formule $Tu-(Y)_n$ dans laquelle Tu est tel que défini ci-dessus, Y représente un groupe thiol de chaîne latérale d'une protéine, ou un groupe amidoalkylthio introduit sur un groupe amine de Tu en employant des réactifs tels que l'ester de l'acide 3-(2-dithiopyridyl)propionique dérivé de l'hydroxysuccinimide suivi d'une réduction avec un agent tel que du dithiothréitol, ou un groupe amidoalkylthio introduit sur un groupe amine de Tu en employant du 2-iminothiolanne, et n varie de 1 à 10, dans des conditions aqueuses tamponnées à un pH de 4,5 à 7, à une température de 4° à 40 °C inclus, pour produire un composé de formule :

$$\begin{array}{l} Tu-(Z-SP-SS-W)_m \\ | \\ (Y)_{n-m} \end{array}$$

dans laquelle Tu, Sp, W et n sont tels que définis ci-dessus, et Z est formé par réaction covalente des groupes Q et Y, et Z représente :
-SS-,

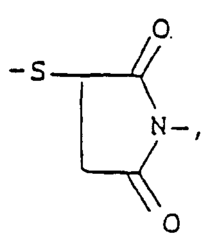 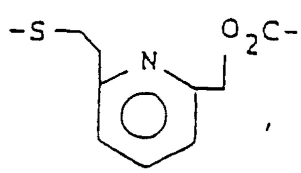 , ou 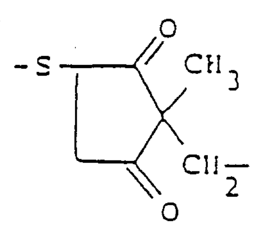

et n varie de 0,1 à 10 ; ou

on fait réagir un composé de formule Q-Sp-SS-W dans laquelle Sp et W sont tels que définis ci-dessus, et Q représente $-NH_2$, $-CONHNH_2$, $-NHCONHNH_2$, $-NHCSNHNH_2$ ou $-ONH_2$, avec une molécule de formule $Tu-(Y)_n$ dans laquelle Tu est tel que défini ci-dessus, et Y représente un groupe aldéhyde généré à partir de groupes hydrate de carbone présents sur Tu, par oxydation en présence d'un périodate alcalino-terreux, dans un

tampon aqueux à un pH de 4,0 à 6,5 à une température de 4° à 40 °C inclus, et n varie de 1 à 20, afin de générer un composé de formule :

$$\begin{array}{l} Tu-(Z-Sp-SS-W)_m \\ \quad | \\ (Y)_{n-m} \end{array}$$

dans laquelle Tu, Sp, W, Y et n sont tels que définis ci-dessus, et Z est formé par réaction covalente de Q et Y, et il représente -ON=CH-, -N=CH-, -CONHN=CH-, -NHCONHN=CH- ou -NHCSNHN=CH-, et m varie de 0,1 à 15 ; ou on traite le composé immédiatement mentionné ci-dessus, de formule :

$$\begin{array}{l} Tu-(Z-Sp-SS-W)_m \\ \quad | \\ (Y)_{n-m} \end{array}$$

dans laquelle Tu, Z, Sp, W, Y, n et m sont tels qu'immédiatement définis ci-dessus, avec de l'acétylhydrazine ou de la tyrosine hydrazine dans un tampon aqueux à un pH de 4,0 à 6,5 et à une température de 4° à 40 °C inclus, pour générer un composé de formule :

$$\begin{array}{l} Tu-(Z-Sp-SS-W)_m \\ \quad | \\ (Y)_{n-m} \end{array}$$

dans laquelle Tu, Z, Sp, W, n et m sont tels qu'immédiatement définis ci-dessus, et Y représente -$CH=NNHCOCH_3$ ou

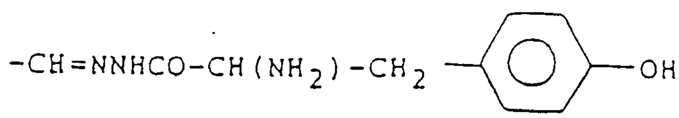

et

on fait réagir ce composé avec du cyanoborohydrure de sodium ou du borohydrure de sodium dans un tampon aqueux à un pH de 4,0 à 6,5, à une température de 4° à 40 °C inclus, pour générer un composé de formule :

$$\begin{array}{l} Tu-(Z-Sp-SS-W)_m \\ \quad | \\ (Y)_{n-m} \end{array}$$

dans laquelle Tu, Sp, W, m et n sont tels que définis ci-dessus, Z représente -$NH-CH_2$-, -$CONHNHCH_2$-, -$NHCONHNHCH_2$- ou -$NHCSNHNHCH_2$-, et Y représente -$CH_2NHNHCOCH_3$ ou

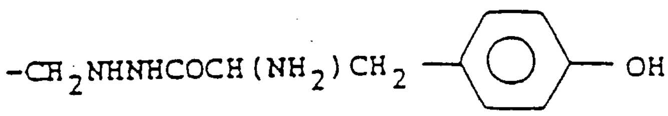

4. Procédé pour la préparation d'un conjugué véhicule-médicament de formule :

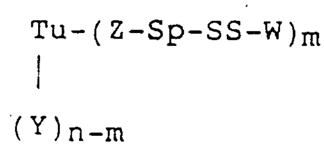

préparé a partir d'un composé de formule $CH_3SSS$-W, $CH_3SSS$-W étant un dérivé N-acylé d'un antibiotique antitumoral dénommé LL-E33288, $\alpha_1$-I, $\alpha_2$-Br, $\alpha_2$-I, $\beta_1$-Br, $\beta_1$-I, $\gamma_1$-Br, $\gamma_1$-I, $\delta_1$-I, BBM-1675, FR-900405, FR-900406, PD114759, PD115028, CL-1577A, CL-1577B, CL-1577D, CL-1577E ou CL-1724, W étant tel que décrit à la revendication 1, comprenant :

la réaction de $CH_3SSS$-W avec un composé de formule générale Q-Sp-SH, Sp représentant un groupe divalent ou trivalent en $C_1$ à $C_{18}$ à chaîne linéaire ou ramifiée, aryle ou hétéroaryle divalent ou trivalent, cycloalkyle ou hétérocycloalkyle en $C_3$-$C_{18}$ divalent ou trivalent, aryl- ou hétéroaryl-(alkyle en $C_1$-$C_{18}$) divalent ou trivalent, cycloalkyl- ou hétérocycloalkyl-(alkyle en $C_1$-$C_{18}$) divalent ou trivalent, ou alkyle en $C_2$-$C_{18}$ insaturé divalent ou trivalent, où si Sp représente un groupe trivalent, il peut être en outre substitué avec des groupes amino, alkylamino, arylamino, hétéroarylamino, carboxyle, alkoxy inférieurs, hydroxy, thiol ou alkylthio inférieurs, à condition que lorsque $CH_3$-SSS-W est l'antibiotique antitumoral de base LL-E33288 $\alpha_1$-Br, $\alpha_1$-I, $\alpha_2$-Br, $\alpha_2$-I, $\beta_1$-Br, $\beta_1$-I, $\gamma_1$-Br, $\gamma_1$-I, $\delta_1$-I, BBM-1675, FR-900405, FR-900406, PD114759, PD115028, CL-1577A, CL-1577B, CL-1577D, CL-1577E ou CL-1724 lui-même, Sp ne puisse alors pas être divalent ; et Q représente ou peut être ultérieurement converti en un atome d'halogène, un groupe amino, alkylamino, carboxyle, carboxaldéhyde, hydroxy, thiol, $\alpha$-haloacétyloxy, (alkyl inférieur)-dicarboxyle, $-CONHNH_2$, $-NHCONHNH_2$, $-NHCSNHNH_2$, $-ONH_2$, $-CON_3$,

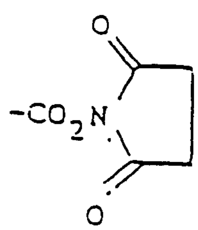 , 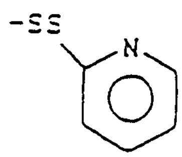 , 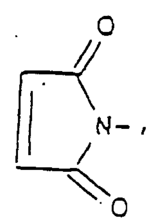 , 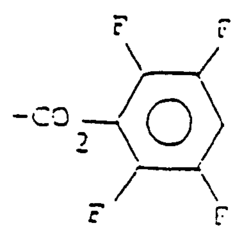 ,

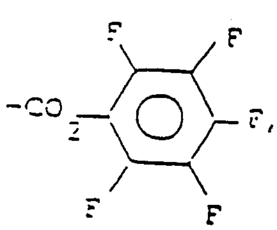 , 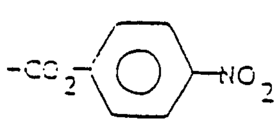 , 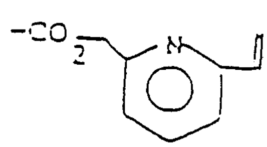 , 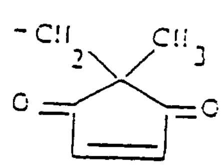

pour produire un intermédiaire de formule Q-Sp-SS-W, dans laquelle Q, Sp et W sont tels que définis ci-dessus,

la réaction de Q-Sp-SS-W avec une molécule de formule Tu-$(Y)_n$ dans laquelle le véhicule Tu est défini comme étant un anticorps mono- ou polyclonal, ses fragments, ses produits analogues obtenus par manipulation chimique ou génétique, ou des facteurs de croissance ou des stéroïdes ; Y représente un groupe amino, carboxy ou thiol de chaîne latérale d'une protéine, un groupe aldéhyde dérivé de résidus d'hydrate de carbone de glycoprotéine, ou un groupe amidoalkylthio ; et n représente un entier de 1 à 100, pour produire un composé de formule :

$$\begin{array}{l} Tu\text{-}(Z\text{-}Sp\text{-}SS\text{-}W)_m \\ | \\ (Y)_{n-m} \end{array}$$

dans laquelle Tu, Y, Sp, W et n sont tels que définis ci-dessus, et Z est formé par réaction covalente des groupes Q et Y directement ou après réduction ultérieure, et Z représente -CONH-, -CONHN=CH-, -CONHNHCH$_2$-, -NHCONHN=CH-, -NHCONHNHCH$_2$-, -NHCSNHN=CH-, -NHCSNHNHCH$_2$-, -ON=CH-, -NH-, -NHCH$_2$-, -N=CH-, -CO$_2$-, -NHCH$_2$CH$_2$-, -SS-,

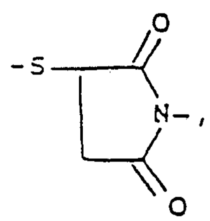 , 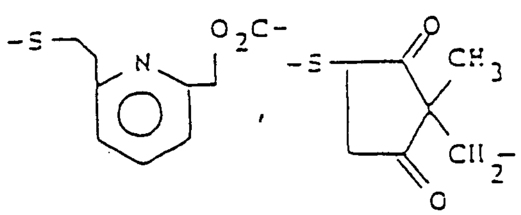 ou 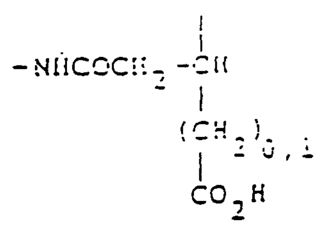

et m varie de 0.1 à 15.

5. Procédé pour la préparation d'un conjugué protéiné-médicament selon la revendication 4 de formule :

$$\begin{array}{l} Tu\text{-}(Z\text{-}Sp\text{-}SS\text{-}W)_m \\ | \\ (Y)_{n-m} \end{array}$$

préparé à partir de la classe de dérivés N-acylés des antibiotiques antitumoraux dénommés LL-E33288 ($CH_3SSS$-W) comprenant les étapes consistant à :

déplacer le groupe dithiométhyle à l'aide d'un composé de formule Q-Sp-SH dans laquelle Sp représente des groupes divalents ou trivalents en $C_2$ à $C_{10}$ à chaîne linéaire ou ramifiée, et des groupes aryl- ou hétéroaryl-(alkyle en $C_2$-$C_5$) divalents ou trivalents, où si Sp représente un groupe trivalent, il peut être en outre substitué avec des groupes amino, hétéroarylamino, hydroxy ou thiol ; et Q est ou peut être ultérieurement converti en un groupe carboxyle, (alkyl inférieur)-dicarboxylanhydride, -CO$_2$Su, -CONHNH$_2$, ou

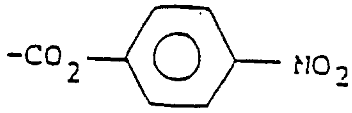

pour produire un intermédiaire de formule générale Q-Sp-SS-W dans laquelle Q, Sp et W sont tels que définis ci-dessus,

faire réagir Q-Sp-SS-W avec une molécule de formule Tu-$(Y)_n$ dans laquelle Tu représente un anticorps monoclonal ayant une réactivité préférentielle avec un antigène associé à une tumeur humaine, Y représente un groupe amino de chaîne latérale présent sur l'anticorps, ou un groupe aldéhyde généré par oxydation des groupes hydrate de carbone de l'anticorps, et n est un entier de 1 à 100, pour produire un composé de formule :

$$\begin{array}{l} Tu-(Z-Sp-SS-W)_m \\ | \\ (Y)_{n-m} \end{array}$$

dans laquelle Tu, Y, Sp, W et n sont tels que définis ci-dessus, et Z est formé par réaction covalente des groupes Q et Y directement ou après réduction ultérieure, et Z représente -CONH-, -CONHN=CH-, $-CONHNHCH_2-$, ou

$$\begin{array}{l} \qquad\qquad\quad | \\ -NHCOCH_2-CH \\ \qquad\qquad\quad | \\ \qquad\qquad (CH_2)_{0,1} \\ \qquad\qquad\quad | \\ \qquad\qquad CH_2H \end{array}$$

et m varie de 0,1 à 15.

6. Procédé pour la préparation d'un conjugué protéine-médicament de formule:

$$\begin{array}{l} Tu-(Z-Sp-SS-W)_m \\ | \\ (Y)_{n-m} \end{array}$$

préparé à partir de l'antibiotique antitumoral dénommé N-acétyl LL-E33288$\gamma_1$ ($CH_3SSS$-W), ayant:

a) un spectre ultraviolet tel qu'illustré sur la figure 1;
b) un spectre infrarouge tel qu'illustré sur la figure 2;
c) un spectre de résonance magnétique du proton tel qu'illustré sur la figure 3; et
d) un spectre de résonance magnétique nucléaire du carbone-13 tel qu'illustré sur la figure 4;

selon un procédé comprenant les étapes consistant:

à déplacer le goupe dithiométhyle avec un composé de formule Q-Sp-SH dans laquelle Sp représente des groupes en $C_2$-$C_5$ divalents ou trivalents à chaîne linéaire ou ramifiée ou des groupes aryl- ou hétéroaryl-(alkyle en $C_2$-$C_5$) divalents ou trivalents, où si Sp représente un groupe trivalent, il peut être en outre substitué avec des groupes amino, hétéroarylamino, hydroxy ou thiol; et Q représente ou peut être ultérieurement converti en un groupe carboxyle, (alkyl inférieur)-dicarboxylanhydride, $-CONHNH_2$, ou

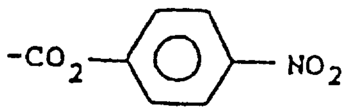

pour produire un intermédiaire de formule générale Q-Sp-SS-W dans laquelle Q, Sp et W sont tels que définis ci-dessus,

à faire réagir Q-,Sp-SS-W avec une molécule de formule Tu-$(Y)_n$ dans laquelle Tu représente un anticorps monoclonal ayant une réactivité préférentielle avec un antigène associé à une tumeur humaine, Y représente un groupe amino de chaîne latérale présent sur l'anticorps, ou un groupe aldéhyde généré par oxydation des groupes hydrate de carbone de l'anticorps, et n est un entier de 1 à 100, pour produire un composé de formule:

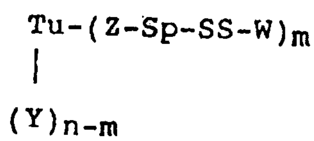

dans laquelle Tu, Y, Sp, W et n sont tels que définis ci-dessus, et Z est formé par réaction covalente des groupes Q et Y, directement ou après réduction ultérieure, et Z représente -CONH-, -CONHN=CH-, -$CONHNHCH_2$-, ou

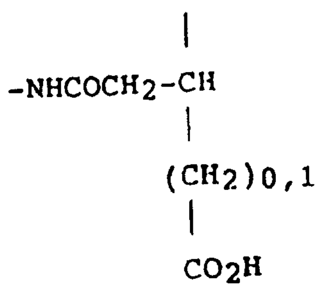

et m varie de 0,1 à 15.

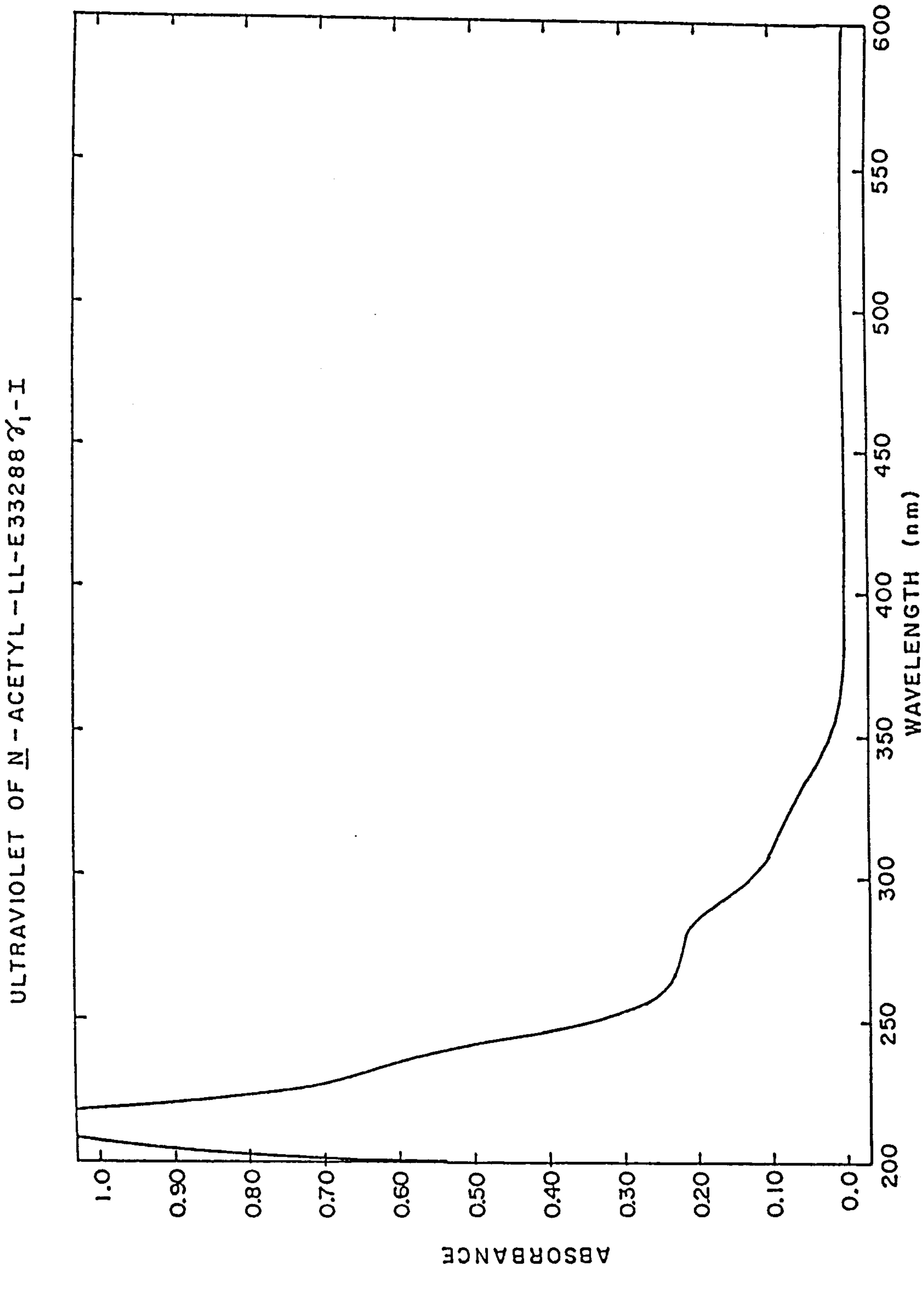
ULTRAVIOLET OF N-ACETYL-LL-E33288 γ1-I
ABSORBANCE
1.0
0.90
0.80
0.70
0.60
0.50
0.40
0.30
0.20
0.10
0.0
200
250
300
350
400
450
500
550
600
WAVELENGTH (nm)

FIGURE I

INFRARED OF N-ACETYL-LL-E33288 $\gamma_1$-I

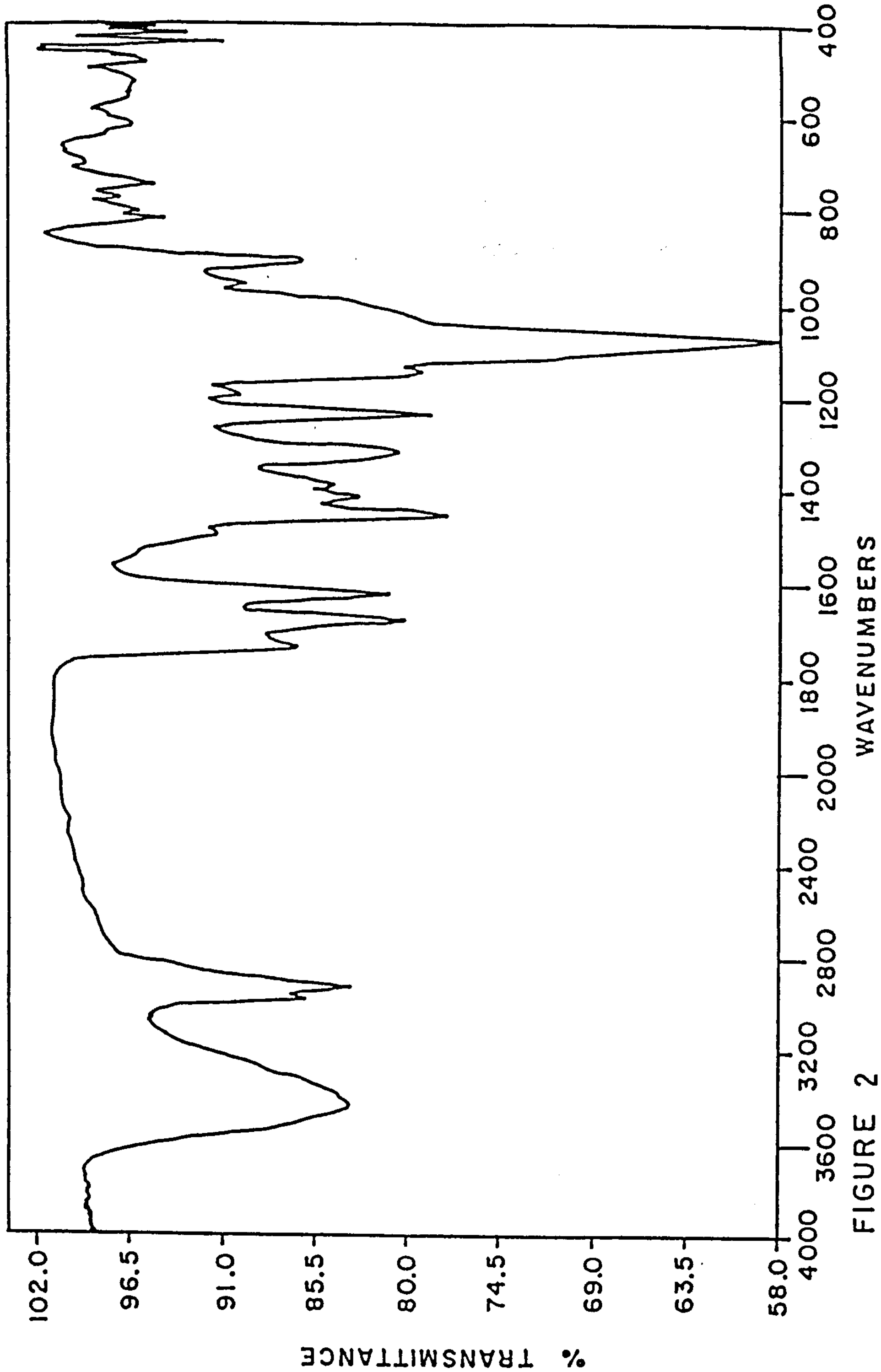


FIGURE 2

PROTON MAGNETIC RESONANCE OF N-ACETYL-LL-E33288$\gamma_1$-I

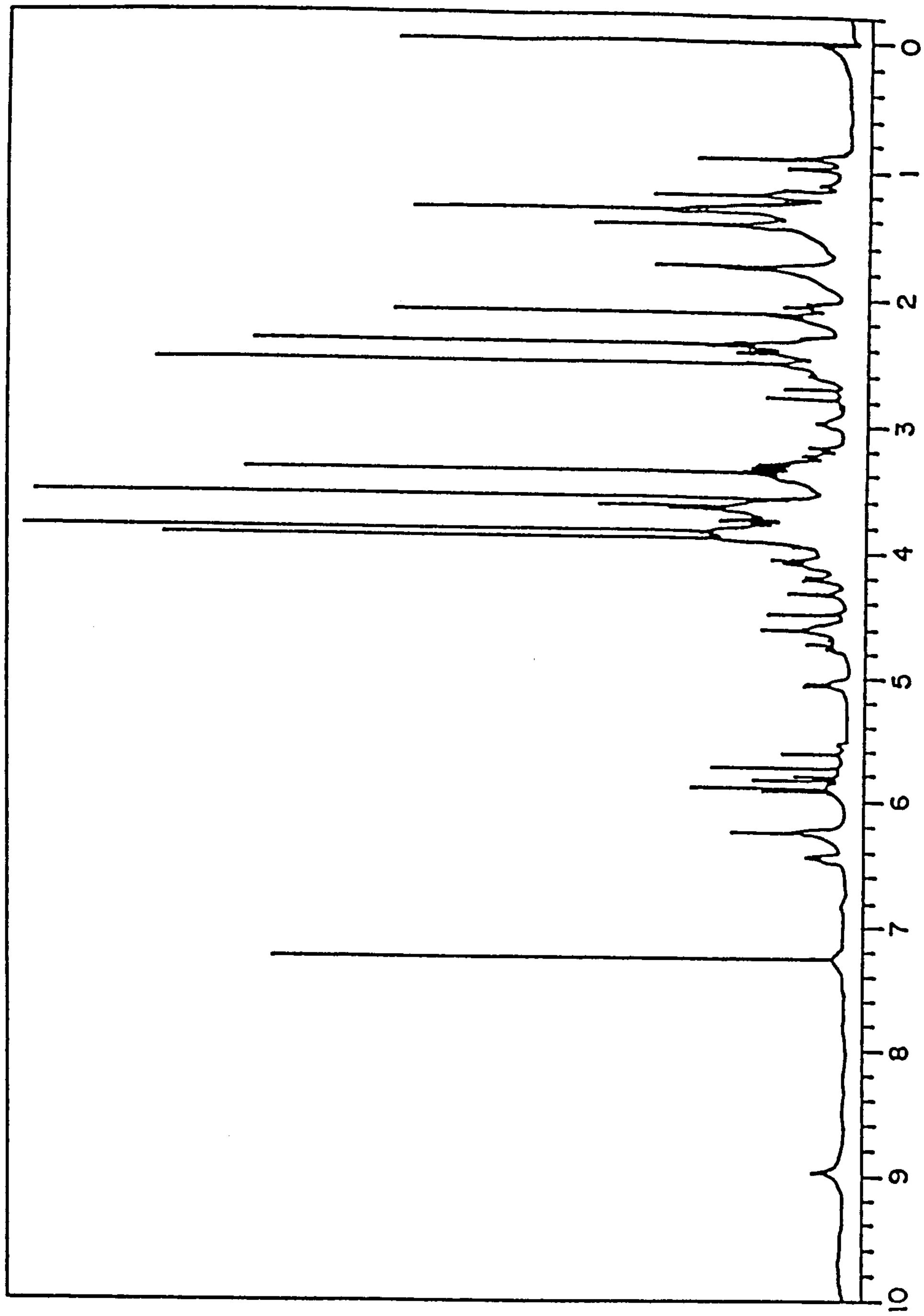


PPM

FIGURE 3

CARBON 13 OF N – ACETYL– LL-E33288$\gamma_1$-I

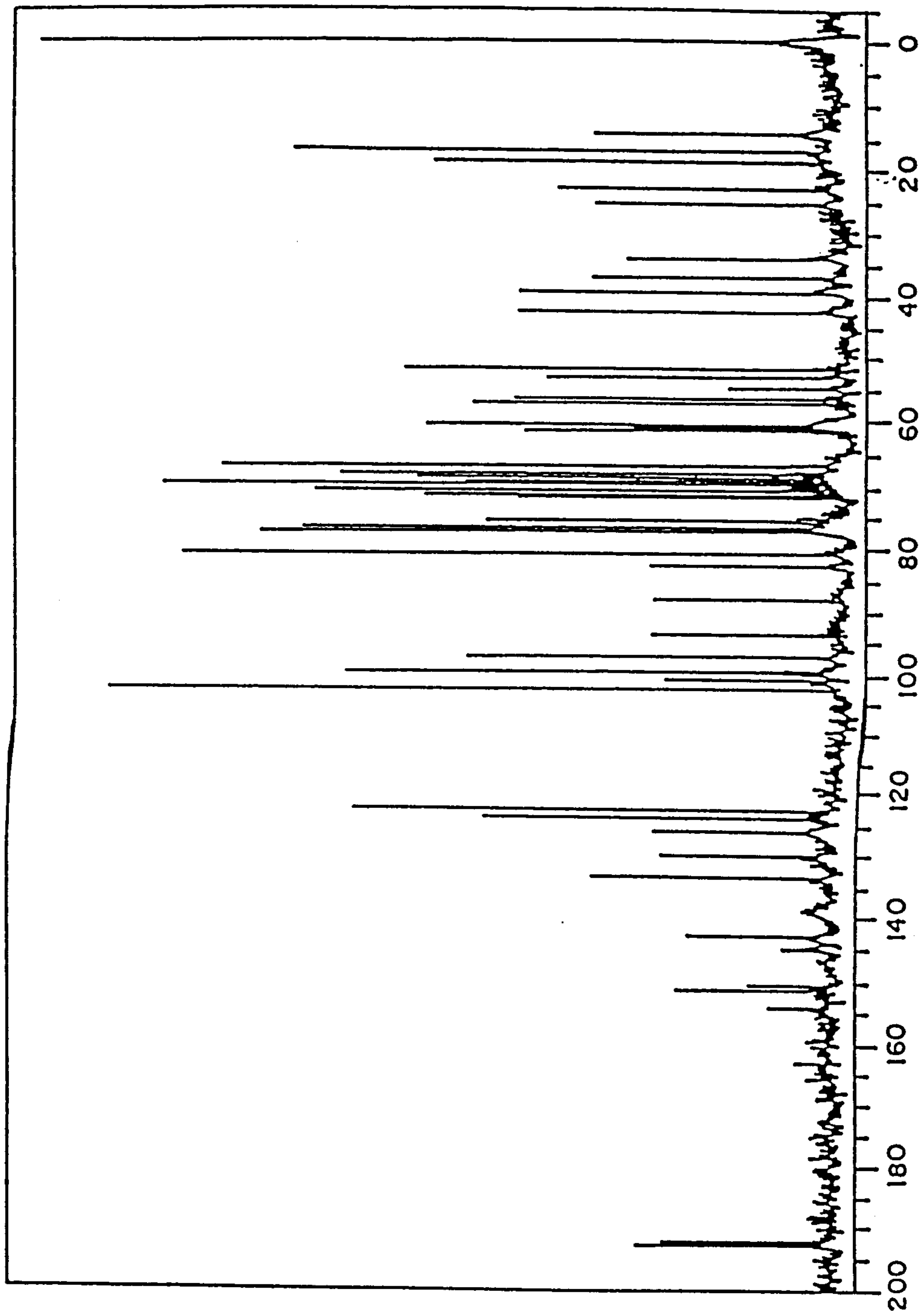


FIGURE 4